# EUROPEAN PATENT APPLICATION

(11) **EP 4 806 908 A2**
(43) Date of publication of application: **16.09.2026**
(21) Application number: 26195203.0
(22) Date of filing: 11.06.2021
(51) Int. Cl.: C07K 14/58

(54) **C-TYPE NATRIURETIC PEPTIDES AND METHODS THEREOF IN TREATING CANCER**

(30) Priority: 12.06.2020 US 202063038606 P; 15.06.2020 US 202063039225 P
(62) Document number(s) of the earlier application(s) in accordance with Art. 76 EPC: 21823022.5 / 4 164 672
(71) Applicant: PharmaIN Corporation, Bothell, WA 98011-8202 (US)
(72) Inventor: TACHIBANA, Hirofumi, Bothell, Washington, 98011 (US); KUMAZOE, Motofumi, Bothell, Washington, 98011 (US); TANAKA, Yasutake, Bothell, Washington, 98011 (US); NOJIRI, Takashi, Bothell, Washington, 98011 (US); CASTILLO, Gerardo, Bothell, Washington, 98011 (US); NISHIMOTO-ASHFIELD, Akiko, Bothell, Washington, 98011 (US); BOLOTIN, Elijah, Bothell, Washington, 98011 (US); YAO, Yao, Bothell, Washington, 98011 (US)
(74) Representative: J A Kemp LLP

(57) **Abstract**

The present disclosure relates to methods of treating a subject having abnormal vasculature by administering to the subject a therapeutically effective bolus dose of a composition including a long acting C-type natriuretic peptide (CNP), CNP derivative, long acting CNP derivative, long acting CNP receptor (NPRB) agonist, or any combination thereof. The present disclosure further relates to treating a subject in need of an increase in cytotoxic T cell and/or NK cell activity by administering to the subject a therapeutically effective bolus dose of a composition including a long acting C-type natriuretic peptide (CNP), CNP derivative, long acting CNP derivative, long acting CNP receptor (NPRB) agonist, or any combination thereof.

## Description

### CROSS-REFERENCE TO RELATED APPLICATIONS

This application claims the benefit of U.S. Patent Application No. 63/038,606, filed June 12, 2020, and U.S. Patent Application No. 63/039,225, filed June 15, 2020, the disclosure of each of which is incorporated herein by reference in its entirety.

### STATEMENT REGARDING SEQUENCE LISTING

The sequence listing associated with this application is provided in text format in lieu of a paper copy and is hereby incorporated by reference into the specification. The name of the text file containing the sequence listing is 74048_Sequence.txt. The text file is 16 KB and was created on June 11, 2021.

### BACKGROUND

### Existing Cancer Therapies

Cancer is a leading cause of death worldwide, accounting for 7 to 8 million deaths (approximately 13% of all deaths) yearly since 2004. Deaths from cancer worldwide are projected to continue to rise, with an estimated 12 million deaths by 2030, and with lung, stomach, liver, colon, and breast cancers deaths being the most prevalent. In 2019, roughly 1.8 million people were diagnosed with cancer in the United States, with lung, colon, pancreas, breast or prostate, and liver cancers accounting for over half a million cases.

Existing cancer treatments are inadequate in reducing this death rate and thus innovative treatments are needed. Existing cancer treatments, such as radiation or chemotherapy, are associated with severe side effects that increase patient suffering. A recent approach to treat cancer is to activate the patient's own immune system to attack a tumor or a cancer by activating cytotoxic T cells within the cancer or tumor tissue, and/or by reducing or eliminating Treg immune cells to facilitate proliferation of cytotoxic T cells in the cancer or tumor tissue, thereby damaging or eliminating the cancer or tumor tissue. For example, an immune checkpoint protein inhibitor can be used to bind directly to an immune checkpoint protein or its corresponding ligand, resulting in an activation of the immune system to attack tumor or cancer cells by activating cytotoxic T cells within the cancer or tumor tissue, and/or by reducing or eliminating Treg immune cells to facilitate proliferation of cytotoxic T cells in the cancer tissue or tumor, thereby damaging or eliminating the cancer or tumor tissue. Another approach for cancer treatment is to activate natural killer cells (NK cells) and/or cytotoxic T-cells, together with suppression of Treg cells and/or myeloid derived suppressor cells (MDSCs).

A further approach for treating cancer is chimeric antigen receptor T-call (CAR T-cell) therapy. In CAR T-cell therapy, a patient's T cells (a type of immune system cell) are changed in the laboratory so that they can be programmed to attack cancer cells. For example, T cells are taken from a patient's blood, then the gene for a special receptor that binds to a certain protein on the patient's cancer cells is added in the laboratory. The special receptor is called a chimeric antigen receptor (CAR). Large numbers of the CAR T-cells are grown in the laboratory and given to the patient by infusion. CAR T-cell therapy is being studied in the treatment of certain types of cancer. CAR T-cell therapy is also called chimeric antigen receptor T-cell therapy. CAR specificity comes from the extracellular domain, which is derived from the antigen-binding site of a monoclonal antibody that recognizes the tumor, while the intracellular domain recapitulates the normal series of events by which T cells are activated and incorporates stimulatory and costimulatory domains, such as CD28 or 4-1BB (CD137), to augment CAR T-cell survival and proliferation. Because CAR T-cells carry their own co-stimulatory signaling, in theory they are less likely to be down-regulated by tumor cells compared to unmodified T cells. CAR T-cell side effects include cytokine release syndrome similar to flu-like symptoms, but can sometimes be serious and life threatening. An additional side effect includes neurologic events such as encephalopathy (brain disease, injury, malfunction), confusion, aphasia (difficulty understand or speaking), drowsiness, agitation, seizures, loss of balance, and altered consciousness.

It is believed that any drug that can activate the cytotoxic/killer cells of the immune system (*e.g.,* cytotoxic T cells and NK cells) to attack tumor or cancer is effective in numerous cancers. However, the use of cytotoxic T cell or natural killer cell immunostimulants such as immune checkpoint inhibitors that bind directly to immune checkpoint proteins or their ligands to facilitate tumor destruction can also result in severe autoimmune disease and/or damage to healthy organs. Despite the risk, several cytotoxic cell immunostimulants have been developed and approved by the Food and Drug Administration for the treatment of various cancers or malignancies because of their high benefit (*e.g.,* survival) to risk (*e.g.,* immune-related side effects) ratio.

For example, immune checkpoint protein PD-1 inhibitors such as nivolumab and/or pembrolizumab (anti-PD 1 antibodies) were approved by the FDA for the treatment of squamous cell head & neck cancer, malignant melanoma, Merkel cell carcinoma, hepatocellular carcinoma, advanced renal cell carcinoma, cancers due to microsatellite instability-high (MSI-H) or defect in mismatch repair (dMMR), cervical cancer, small cell lung carcinoma, non-small cell lung carcinoma, gastric and GEJ carcinoma (adenocarcinomas of the esophagogastric junction), PMBCL (primary mediastinal B-cell lymphoma, a rare B-cell non-Hodgkin lymphoma), classical Hodgkin lymphoma, and locally advanced or metastatic urothelial cancers. Some representative examples of cytotoxic cell immunostimulants that have utility against multiple cancers include 1) pembrolizumab (Keytruda^{®}), prescribing information revised: 02/2019; 2) nivolumab (Opdivo^{®}), prescribing information revised: 02/2019; 3) ipilimumab (Yervoy^{®}), prescribing information revised: 07/2018; 4) atezolizumab (Tecentriq^{®}), prescribing information revised: 03/2019; 5) avelumab (Bavencio^{®}), prescribing information revised: 10/2018; 6) durvalumab (Imfinzi^{®}), prescribing information revised: 02/2018; and 7) cemiplimab (Libtayo^{®}), prescribing information revised: 09/2018). However, the effectiveness of these immunostimulants remains limited (*see, e.g.,* Fashoyin-Aji et al., The Oncologist 2019; 24:103-109, incorporated herein by reference in its entirety), and these immunostimulants carry the risk of severe autoimmune side effects. Nevertheless, because of the utility of cytotoxic cell immunostimulants for the treatment of many types of cancers, the FDA has approved several cytotoxic cell immunostimulants for multiple cancers including cancers of head and neck, skin (*e.g.,* Merkel, squamous, melanoma), liver (*e.g.,* hepatocellular carcinoma), kidney, cervix, lung (*e.g.,* small cell and non-small cell), breast, stomach, colon, esophagus, lymph node (*e.g.,* Hodgkin & non-Hodgkin PMBCL), pancreas, stomach, ovaries and other organs of the body (*e.g.,* caused by microsatellite instability-high (MSI-H) or defect in mismatch repair (dMMR)), urethra, bladder, ureters, renal pelvis and surrounding organs (urothelial cancer).

### CNP and NPRB receptor

CNP was first isolated in 1990 from porcine brain by Sudoh *et al.* and is a peptide that consists of 22 amino acid residues. *See, e.g.,* Sudoh et al., Biochem. Biophys. Res. Commun. 1989; 159:1427-1434. CNP has a ring structure and is similar structurally to related natriuretic peptides, atrial natriuretic peptide (ANP), and B-type natriuretic peptide (BNP), but lacks a carboxy-terminal extension. *See, e.g.,* Hunt et al., J. Clin. Endocrinol. Metab. 1994; 78:1428-1435. CNP is a highly conserved natriuretic peptide among various species. *See, e.g.,* Imura et al., Front. Neuroendocrinol. 1992; 13:217-249. For example, in humans, CNP gene (NPPC) is located on chromosome 2, whereas the mouse CNP gene is located on chromosome 1. CNP gene is composed of two exons and one intron. *See, e.g.,* Ogawa et al., The Journal of Clinical Investigation. 1994; 93:1911-192110; and Ogawa et al., Genomics. 1994; 15(24):383-387. It is produced as a preprohormone or a 126 amino acid residue parent-CNP peptide that is converted to 103 amino acid residue pro-CNP after removal of 23 amino acid residues at the carboxyl end, and is further processed to a 53 amino acid residue-containing CNP-53 and a 22 amino acid residue-containing CNP by the enzyme furin. *See, e.g.,* Lumsden et al., Curr. Pharm. Des. 2010; 16:4080-4088; Wu et al., J. Biol. Chem. 2003; 278:25847-25852; and Chopra et al., Indian J. Endocrinol. Metab. 2013; 17:83-90. The higher molecular weight CNP-53 (CNP 51-103) predominates in tissues, whereas CNP-22 (CNP 82-103) is found mainly in plasma and cerebrospinal fluid but both contain 17-amino acid residue ring structure common to all natriuretic peptide. In comparison to ANP and BNP, the plasma half-life of CNP is relatively short and is about 2 to 3 min in humans. *See, e.g.,* Potter LR. FEBS J. 2011; 278:1808-1817. Normal plasma CNP concentrations (both forms) are in low femtomole per milliliter range. *See, e.g.,* Das B.B. and Solinger R., Cardiovasc Hematol Agents Med Chem. 2009, 7, 29-42. CNP is mainly produced and secreted from the endothelium of vasculature and male genital glands and acts as a relaxing peptide. *See, e.g.,* Suga et al., Endocrinology. 1998; 139:1920-1926.

CNP peptides have two known membrane receptors, namely natriuretic peptide receptor B (NPRB) and natriuretic peptide receptor C (NPRC). The NPRB receives messages from CNP and activates downstream signaling pathways, whereas NPRC is mainly a clearance receptor that is primarily involved in clearance or degradation of CNP. *See, e.g.,* Itoh H and Nakao K, Nihon Rinsho. 1997; 55:1923-1936; Koller et al., Science. 1991; 252:120-123; Suga et al., Endocrinology. 1992; 130:229-239; and Potter LR and Hunter T. J. Biol. Chem. 2001; 276:6057-6060. NPRB is also known by other names such as guanylate cyclase B (GC-B) or B-type natriuretic peptide receptor 2 (NPR2).

The remaining natriuretic peptide receptor, NPRA, is activated by atrial natriuretic peptide (ANP) and B-type natriuretic peptide (BNP), but is not activated by CNP. While ANP and BNP activate both NPRA and NPRB, CNP selectively activates NPRB, and all three natriuretic peptides bind to NPRC (which lacks guanylyl cyclase activity) and undergo clearance and degradation. *See, e.g.,* Koller et al., Science. 1991; 252:120-123; Suga et al., Endocrinology. 1992; 130:229-239; and Potter LR and Hunter T. J. Biol. Chem. 2001; 276:6057-6060. The differences in the physiological consequences of activation of one receptor versus both NPRA and NPRB receptors remain unclear. In addition, testing of the *in vivo* effects of CNP is confounded by the difficulty of simple bolus administration of CNP because of its short half-life (2-13 minutes), and the fact that bolus administration is associated with an acute drop in blood pressure. *See, e.g*., Kimura et al., J Surg Res. 2015, 194(2); 631-637. Indeed, it is unknown prior to the present disclosure whether NPRB agonist or CNP can be given as a bolus to treat cancer, nor whether NPRB agonist or CNP can be given as a bolus dose to treat cancer in combination with immune checkpoint inhibitor(s) or CAR T-cell therapy.

Cell culture studies have shown that CNP did not inhibit growth of small-cell lung cancer cells (*see, e.g.,* Vesely et al., Eur J Clin Invest 2005, 35(1), 60-69) and breast adenocarcinoma cells *(see, e.g.,* Vesely et al., Eur J Clin Invest 2005, 35(6), 388-398); and at 1 µM had no anti-cancer effects in general (*see, e.g.,* Vesely, Curr Pharm Des., 2010, p 1159-1166; US Patent No. 7,846,900) compared to 1 µM for other natriuretic peptides such as long acting natriuretic peptide (LANP), vessel dilator, kaliuretic peptide, and ANP. A less significant effect was observed when the concentration was increased. To have an effect in cell culture, a 100-fold concentration in the culture medium was required (*i.e.,* 100 µM or 220 µg/ml in culture medium). However, a concentration of 200 µg/ml in the blood is impractical to use *in vivo* because it would require impractical doses of more than 200 mg/Kg, when accounting for loss of bioavailability and degradation common to most *in vivo*-administered peptides.

Furthermore, it is known that CNP should not be used as a bolus dose to destroy or stop cancer cells, because CNP is known to elevate intracellular cyclic-GMP, and elevation of intracellular cyclic-GMP protects cells from apoptotic death and is thus contrary to the objectives in cancer treatment. Intracellular cyclic-GMP does not escape from the cell efficiently and consequently most studies measure cyclic GMP in the intracellular compartment. Other studies (*see, e.g.,* US Patent No. 9,759,725) describe inhibiting CNP production to treat cancer, and thus is contrary to using guanylate cyclase C agonists such as CNP to destroy cancer cells.

EP 3189835B1 described suppressing the metastasis of a malignant tumor by continuous infusion of native CNP, a natriuretic peptide receptor GC-B agonist, to increase intracellular cyclic-GMP. However, EP 3189835B1 does not describe increasing plasma cyclic-GMP by administration of a bolus of long acting CNP. As a skilled person of the art would appreciate, continuous infusion is considered to be impractical, less convenient, and does not increase plasma cyclic-GMP without also causing a corresponding drop in blood pressure when used as a method of treatment. Furthermore, bolus administration of CNP is understood to be undesirable because the half-life of CNP is very short (2 minutes) and a bolus dose will therefore be degraded very quickly before it can have any effect. Additionally, a short spike of CNP can cause an acute hypotension known to be dangerous to patients and is a liability. Indeed, it is not known to treat cancer with a bolus administration of CNP without causing a drop in blood pressure. For example, for the CNP treatment of rhabdomyosarcoma tumor bearing mice, CNP must be administered by infusion at a slow rate of 2.5 ug/kg/min for 4 weeks (*see, e.g.,* Zenitani et al., Cancer Med., 2016 5(5) p 795-805) rather than a bolus dose; with limited efficacy. Additionally, such treatment has significant limitations and requires an infusion pump that must be carefully calibrated to decrease the likelihood of severe blood pressure drop.

A previous study in healthy human volunteers demonstrated that CNP bolus injection caused a transient but significant decrease in both systolic and diastolic blood pressure with a significant increase in heart rate, with only a limited and transient increase in plasma cyclic-GMP of less than 90 minutes. Igaki et al., Hypertens Res 1998; 21: 7-13. In general, all CNPs produce hemodynamic effects or similar blood pressure-reducing activity in mice, nonhuman primates, rats, dogs, and humans. *See, e.g.,* Wendt et al., J Pharmacol Exp Ther 353:132-149, April 2015. A CNP variant (BMN-111; sequence PGQEHPNARK YKGANKKGLS KGCFGLKLDR IGSMSGLGC(SEQ ID NO. 1)) with increased neutral-endopeptidase (NEP) resistance is currently in development. Studies of BMN-111 in animals and man have demonstrated that as the dose increases to the desired therapeutic level, arterial blood pressure (BP) drops and heart rate (HR) increases. In addition to investigating various variants of CNP, different CNP conjugates were obtained by conjugating the CNP moiety to either PEG or proteinaceous compounds. These PEGylated and chimeric CNP exhibited a similar hemodynamic response as observed for the non-PEGylated CNP variants. All variants previously studied showed similar BP-reducing activity. *See, e.g.,* Wendt, J., Pharmacol Exp Ther 353:132-149, April 2015. Therefore, without wishing to be bound by theory, it is believed that increasing the bolus dose of a drug having CNP activity to increase drug exposure may be associated with unacceptable cardiovascular side-effects, such as hypotension.

There is a need for efficacious, safe, and convenient (*e.g.,* bolus administration) cancer treatments which do not cause cardiovascular side-effects, such as hypotension, while maintaining or enhancing blood cyclic-GMP level. The present disclosure seeks to fulfill these needs and provides further related advantages.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In one aspect, the present disclosure features a method of treating a subject having an abnormal vasculature, in any tissue or organ, including administering to the subject a therapeutically effective bolus dose of a composition comprising a long acting CNP, a very long acting CNP, a long acting CNP derivative, a very long acting CNP derivative, a long acting NPRB agonist, a very long acting NPRB agonist, or any combination thereof, wherein administering the therapeutically effective bolus dose of the composition provides a vasculature normalization or an increase in pericyte coating index by at least 10% (*e.g.,* by at least 15%, or by at least 20%), wherein the composition does not decrease blood pressure by more than 20% (*e.g.,* by more than 15%, by more than 10%, or by more than 5%) of a baseline blood pressure measurement, where the baseline blood pressure measurement is an average blood pressure prior to administration of the composition, and wherein the composition increases plasma cyclic-GMP level at from 1 hour to 12 hours (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) after administration of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject). The subject can further have a low number of cytotoxic T cells, a low number of activated NK cells, or both a low number of cytotoxic T cells and a low number of activated NK cells.

In another aspect, the present disclosure features a method of increasing cytotoxic T-cells and/or activated NK cells, including administering to a subject in need thereof a therapeutically effective bolus dose of a composition including a long acting CNP, a very long acting CNP, a long acting CNP derivative, a very long acting CNP derivative, a long acting NPRB agonist, a very long acting NPRB agonist, or any combination thereof,
wherein administering the therapeutically effective bolus dose of the composition provides an increase in the number of cytotoxic T cells and/or NK cells of at least 15% (*e.g.,* of at least 20%, or of at least 30%) above the level prior to administration of the composition or above the level in a healthy subject, wherein administering the therapeutically effective bolus dose of the composition does not decrease blood pressure by more than 20% (e.g., by more than 15%, by more than 10%, or by more than 5%) of a baseline blood pressure measurement, where the baseline blood pressure measurement is an average blood pressure prior to administration of the composition, and wherein the composition increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).

In yet another aspect, the present disclosure features a method of treating a subject, the subject having an abnormal vasculature, in any tissue or organ, and the subject has a condition (i) to (viii): (i) a low number of cytotoxic T cells, (ii) a low number of activated NK cells, (iii) a high number of Treg cells, (iv) a high level of expression of TGFβ, (v) a high level or expression of Foxp3, (vi) a high number of myeloid-derived suppressor cells or MDSCs, (vii) a high level or expression of Bv8; or (viii) any combination thereof; or the subject is in need of (ix) to (xvi): (ix) an increase in a number of cytotoxic T-cells; (x) an increase in activated NK cells; (xi) a decrease in a number of Treg cells; (xii) a decrease in TGF-β expression; (xiii) a decrease in Foxp3 expression; (xiv) a decrease in a number of myeloid-derived suppressor cells (MDSCs); (xv) a decrease in Bv8 expression, or (xvi) any combination thereof; including administering to the subject a therapeutically effective bolus dose of a composition comprising a long acting CNP, a very long acting CNP, a long acting CNP derivative, a very long acting CNP derivative, a long acting NPRB agonist, a very long acting NPRB agonist, or any combination thereof,
wherein administering the therapeutically effective bolus dose of the composition provides a vasculature normalization or an increase in pericyte coating index by at least 10% (*e.g.,* by at least 15%, or by at least 20%), a reduction in tumor size when present, an increase in the number of cytotoxic T-cells, an increase in the number of activated NK cells, a reduction in the number of Treg cells, a decrease in the level or expression of TGFβ, a decrease in the level or expression of Foxp3, a decrease in the number of myeloid-derived suppressor cells (MDSCs), a decrease in the level or expression of Bv8, an improvement in survival/lifespan, or a combination thereof, wherein the composition does not decrease blood pressure by more than 20% (*e.g.,* by more than 15%, by more than 10%, or by more than 5%) of a baseline blood pressure measurement, where the baseline blood pressure measurement is an average blood pressure prior to administration of the composition; and wherein the composition increases plasma cyclic-GMP level at from 1 hour to 12 hours (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (*e.g.,* above 2x, above 3x, above 4x, or above 5x) of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject). In some embodiments, the subject can further have a low number of cytotoxic T cells, a low number of activated NK cells, or both a low number of cytotoxic T cells and a low number of activated NK cells.

In another aspect, the present disclosure features a method of treating a subject having one or more of a condition selected from a tumor, an abnormal vasculature in a tumor tissue, a low number of cytotoxic T cells, a low number of activated NK cells, a high number of Treg cells, a high level of expression of TGFβ, a high level or expression of Foxp3, a high number of myeloid-derived suppressor cells or MDSCs, and a high level or expression of Bv8; or the subject is in need of an increase in a number of cytotoxic T-cells; an increase in activated NK cells; a decrease in a number of Treg cells; a decrease in TGF-β expression; a decrease in Foxp3 expression; a decrease in a number of myeloid-derived suppressor cells (MDSCs); a decrease in Bv8 expression, or any combination thereof; including administering to the subject a therapeutically effective bolus dose of a composition including a long acting CNP, a very long acting CNP, a long acting CNP derivative, a very long acting CNP derivative, a long acting NPRB agonist, a very long acting NPRB agonist, or any combination thereof, wherein administering the therapeutically effective bolus dose of the composition provides a vasculature normalization or an increase in pericyte coating index by at least 10% (*e.g.,* by at least 15%, or by at least 20%) (*e.g.,* within the tumor tissue), a reduction in tumor size, an increase in the number of cytotoxic T-cells, an increase in the number of activated NK cells, a reduction in the number of Treg cells, a decrease in the level or expression of TGFβ, a decrease in the level or expression of Foxp3, a decrease in the number of myeloid-derived suppressor cells (MDSCs), a decrease in the level or expression of Bv8, an improvement in survival/lifespan, or a combination thereof, and wherein the composition does not decrease blood pressure by more than 20% (*e.g.,* by more than 15%, by more than 10%, or by more than 5%) of a baseline blood pressure measurement, where the baseline blood pressure measurement is an average blood pressure prior to administration of the composition; and wherein the composition increases plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).

In another aspect, the present disclosure features a method of treating cancer, or treating abnormal vasculature, including administering to the subject in need thereof a therapeutically effective bolus dose of a composition including a long acting CNP derivative or a very long acting CNP derivative including U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof, where each individual capital letter, with the exception of U, is an amino acid residue as represented by one-letter amino acid nomenclature, and where U is a moiety of Formula (I) or (II), where Formula (1) is

(aliphatic)ₐ-(X)-; (I)

wherein a is 0 or 1 (preferably a is 1); aliphatic is an optionally substituted C₄₋₂₄ chain (*e.g.,* optionally substituted C₁₀₋₂₄ chain, optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; X is a 1-10 amino acid residue or peptide sequence, wherein each amino acid residue is independently selected from lysine (K), arginine (R), glycine (G), alanine (A), glutamic acid (E), and aspartic acid (D); or X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1, and
Formula (II) is

   (polymer)ₐ-(Y)-; (II)
wherein a is 0 or 1 (preferably a is 1); polymer is cellulose, poly(ethylene glycol) (PEG), methoxy poly(ethylene glycol) (MPEG), poly(lactic-co-glycolic acid), poly(N-vinyl pyrrolidone), or a derivative thereof; Y is: a 1-10 amino acid residue or peptide sequence, wherein each amino acid residue is independently selected from lysine (K), arginine (R), glycine (G), alanine (A), glutamic acid (E), and aspartic acid (D); anon-amino acid linker comprising an ester, an amide, a thioether, an ether, a thioether, a carbamate moiety, or a combination thereof; an amino acid residue-containing linker, wherein the amino acid residue is covalently attached to (polymer)ₐ; or a peptide linker different from the 1-10 amino acid residue or peptide sequence,
wherein the composition does not decrease blood pressure by more than 15% of a baseline blood pressure measurement, where the baseline blood pressure measurement is an average blood pressure prior to administration of the composition; and wherein the composition increases plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).

In some embodiments, Y is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1. In some embodiments, U can be covalently bound to an N-terminal G or C residue and/or to an epsilon amino group of K residue.

In one aspect, the present disclosure provides a composition including a long acting CNP derivative comprising a peptide of formula U-CFGLKLDRIGSxSGLGC [SEQ ID NO. 30], wherein x is a natural or unnatural amino acid residue, provided that x is not a methionine residue; and U has is a moiety of Formula (I):

(aliphatic)ₐ-(X)-; (I)

wherein a is 0 or 1 (preferably a is 1);
aliphatic is an optionally substituted C₄₋₂₄ chain (*e.g.,* optionally substituted C₁₀₋₂₄ chain, optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and
X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In another aspect, the present disclosure features a method of treating a subject using a method described above with, or without, additional treatment with one or more immunostimulant(s) including immune checkpoint inhibitor(s) and/or CAR T-cell therapy.

### DESCRIPTION OF THE DRAWINGS

The foregoing aspects and many of the attendant advantages of this disclosure will become more readily appreciated as the same become better understood by reference to the following detailed description, when taken in conjunction with the accompanying drawings, wherein:

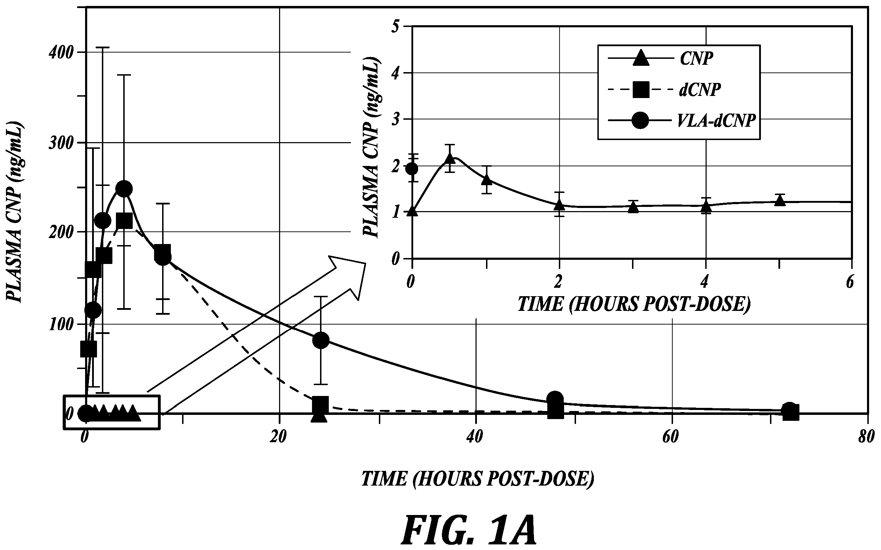

FIGURE 1A is a graph of plasma CNP [mean (SD); n= 5] in CD-1 mice after subcutaneous administration 2.0mg/Kg of native CNP, CNP derivative (dCNP), and very long acting CNP derivative (VLA-dCNP). The inset is an enlarge scale of the left bottom corner to show the low plasma level of CNP (diamond) when native CNP was administered. Error bars represent standard deviation of n=5 plasma samples. Baseline CNP level prior to administration was 1.74 (0.6) ng/mL [mean (SD); n=15]. FIGURE 1A shows the sustained plasma presence of dCNP and VLA-dCNP after bolus administrations in mice.

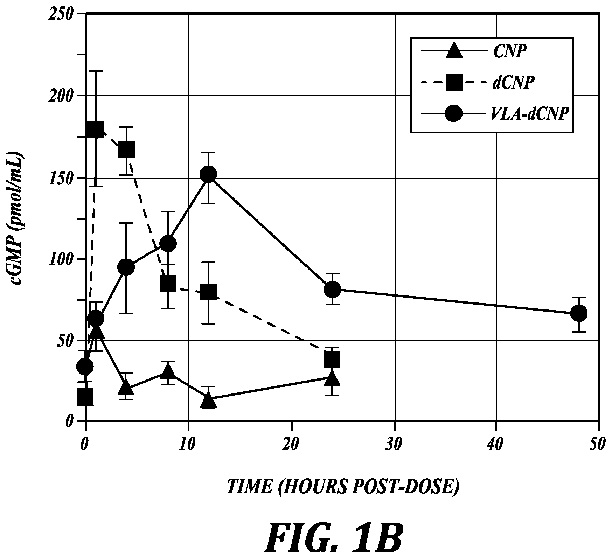

FIGURE 1B is a graph of plasma cGMP in male C57BL/6J mice measured using a cGMP kit from CisBio [Codolet, France] after subcutaneous administration 1.0mg/Kg of native CNP, CNP derivative (dCNP), and very long acting CNP derivative (VLA-dCNP). Baseline plasma cGMP level was 20 (3.7) pmol/mL or 7 (1.3) ng/mL [mean (SEM); n=8]. At 2 hours and beyond, subcutaneous administration of native CNP did not have significant elevation of plasma cGMP compared to the baseline, while similar administration of long acting CNPs (dCNP and VLA-dCNP) showed significant elevation of cGMP for at least 24 hours. FIGURE 1B shows the sustain presence of cyclic-GMP after bolus administrations of dCNP and VLA-dCNP compared to native CNP in mice.

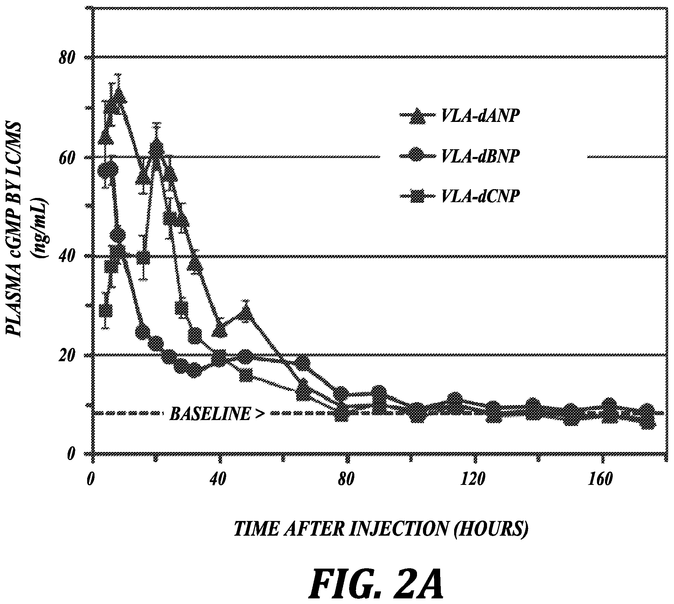

FIGURE 2A is a graph showing that a very long acting CNP derivative (VLA-dCNP) of the present disclosure increases plasma cGMP for 3 days without an associated drop in blood pressure. The graph shows a corresponding increase in plasma cGMP [mean (SEM); n=12] in dogs [mean (SEM); n=12] as monitored after bolus administration of 25ug/Kg of very long acting CNP derivative (VLA-dCNP), very long acting BNP derivative (VLA-dBNP), or very long acting BNP derivative (VLA-dANP). The baseline plasma cGMP level was 8 (2) ng/mL [mean (SD); n=12], a level which is similar to healthy human (*see, e.g.,* Igaki et al., Hypertens Res 1998; 21: 7-13, incorporated herein by reference in its entirety. All very long acting formulation of natriuretic peptide increases cGMP above the baseline of 8ng/ml. The cGMP AUC are VLA-dANP 3,483 ng*h/mL, VLA-dBNP 2,585 ng*h/mL, VLA-dCNP 2,627 ng*h/mL. The very long acting CNP derivative (VLA-dCNP) increased plasma cyclic-GMP for 3 days without an associated drop in blood pressure. FIGURE 2A shows the sustained presence of cyclic-GMP after bolus administration of VLA-dCNP compared to two other very long acting natriuretic peptides from the same family.

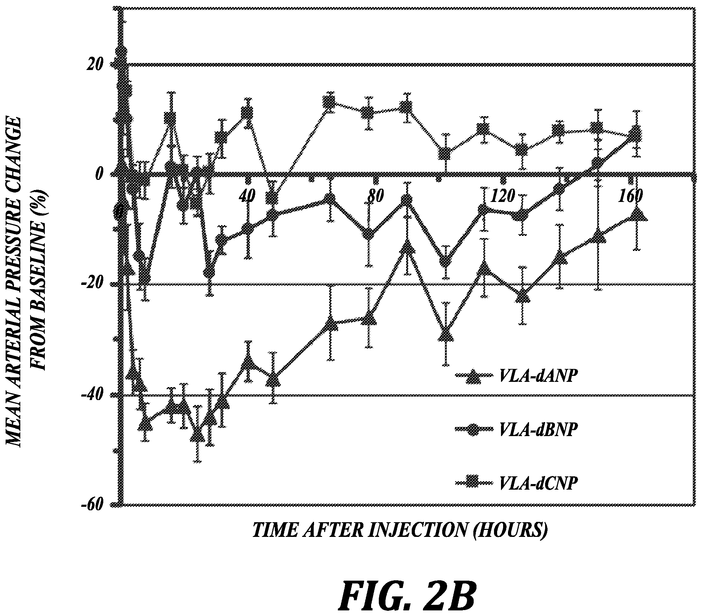

FIGURE 2B is graph showing mean arterial pressure in dogs [mean (SEM); n=12] as monitored after bolus administration of 25ug/Kg of very long acting CNP derivative (VLA-dCNP), very long acting BNP derivative (VLA-dBNP), or very long acting BNP derivative (VLA-dANP). VLA-dCNP does not cause significant blood pressure drop from baseline (0 hr) after administration at a very high dose. In comparison, other very long acting natriuretic peptides such as VLA-dBNP and VLA-dANP derivatives caused more than 15% drop in blood pressure. This is especially true for VLA-dANP where the drop in blood pressure can be as much as 50% for a similar increase in cGMP. In stark contrast, the very long acting CNP derivative (VLA-dCNP) increased plasma cyclic-GMP for 3 days without an associated drop in blood pressure. FIGURE 2B shows that absence of a drop in blood pressure after bolus administrations in dogs of a high dose of VLA-dCNP while the two other very long acting natriuretic peptides from the same family showed similar elevation of plasma cyclic-GMP but with dramatic drop in blood pressure (FIGURE 2A). This indicates that plasma cyclic-GMP is not the cause of the drop in blood pressure.

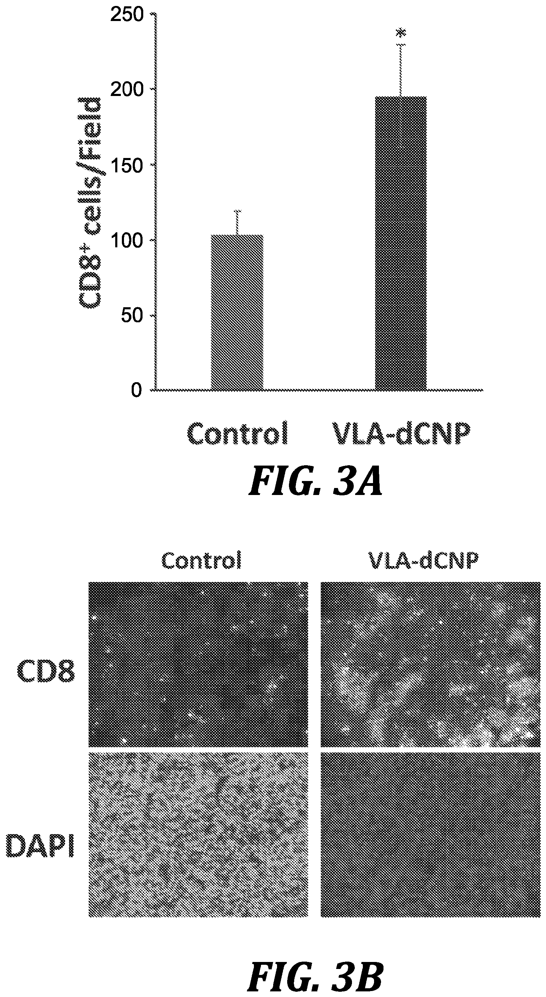

FIGURES 3A and 3B are a graph and associated micrographs showing that VLA-dCNP increased cluster of differentiation 8 positive (CD8+) T cells in breast tumor, indicating that VLA-dCNP facilitated entry, activation of tumor killing cells, and/or suppression of immune checkpoint inhibition against tumor.
FIGURE 3A is a graph of the number of CD8+ T cells per field in the micrographs of FIGURE 3B.
FIGURE 3B is a series of micrographs. The numbers of CD8-positive cells per fields were counted in FIGURE 3A; error bars are SEM. Statistical analysis was performed by Student t- tests by using GraphPad Prism 6.0 (n =4). *P < 0.05.
FIGURES 4A-4C are a series of graphs showing that VLA-dCNP increased activated T cell in breast tumor, indicating that VLA-dCNP facilitated entry, activation of tumor killing cells, and/or suppression of immune checkpoint inhibition against the tumor.

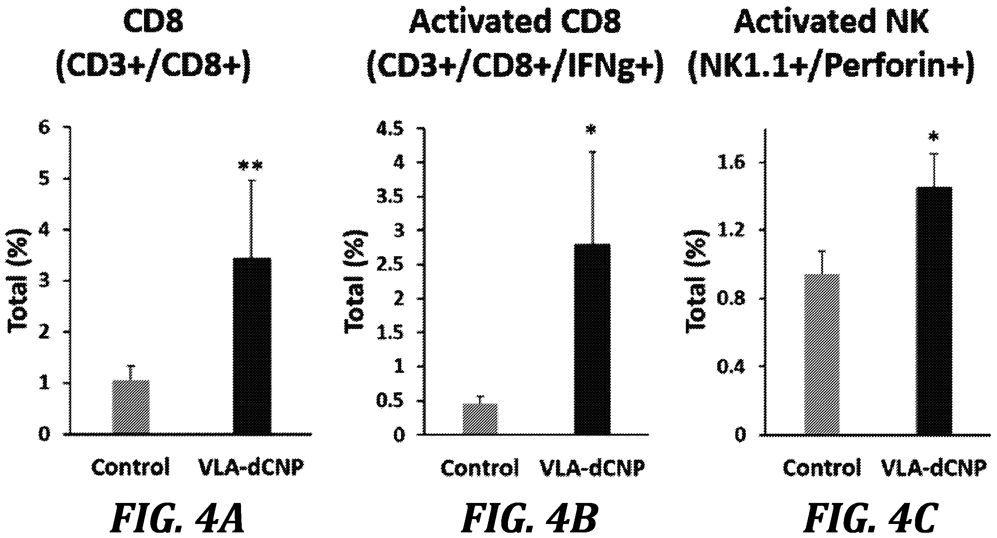

FIGURE 4A is a bar graph showing the amount of CD8 cells in a control mouse group and a group treated with VLA-dCNP.
FIGURE 4B is a bar graph showing the amount of activated CD8 cells in a control mouse group and a group treated with VLA-dCNP.
FIGURE 4C is a bar graph showing the amount of activated NK cells in a control mouse group and a group treated with VLA-dCNP.

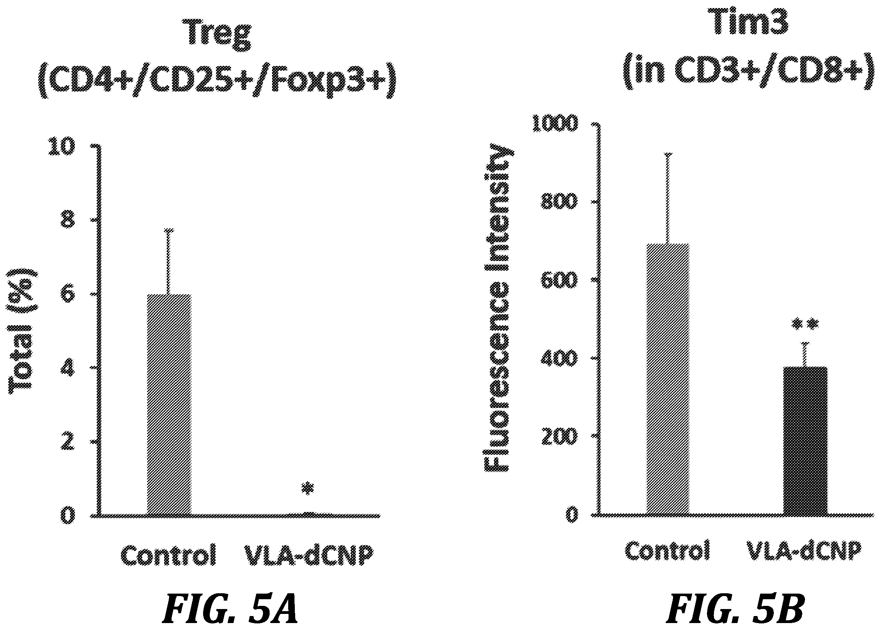

FIGURE 5A is a bar graph showing that VLA-dCNP eradicated Regulatory T cell in breast cancer/tumor, allowing the immune system to suppress tumor growth.
FIGURE 5B is a bar graph showing that VLA-dCNP decreased Tim3 in breast cancer cell/tumor.

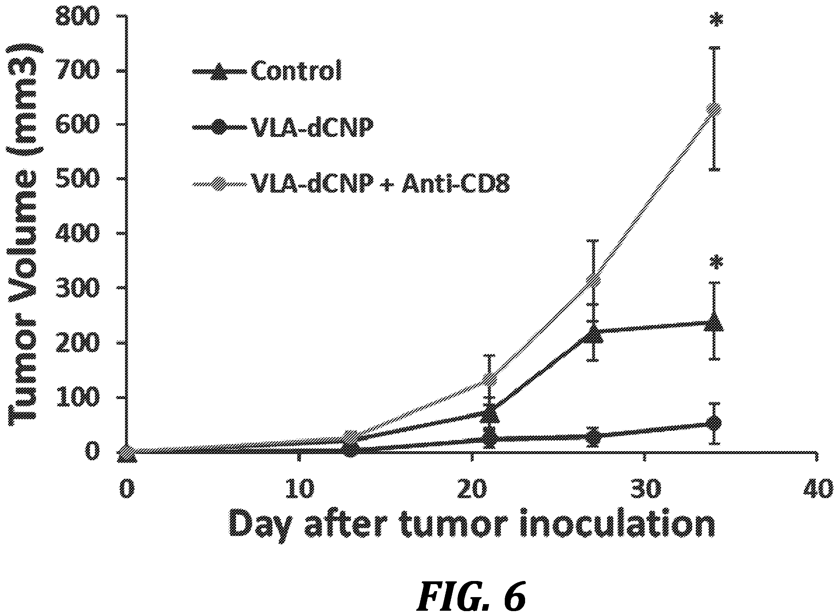

FIGURE 6 is a graph showing the effect of VLA-dCNP on bone tumor volume growth with or without cluster of differentiation 8 (CD8) depletion.

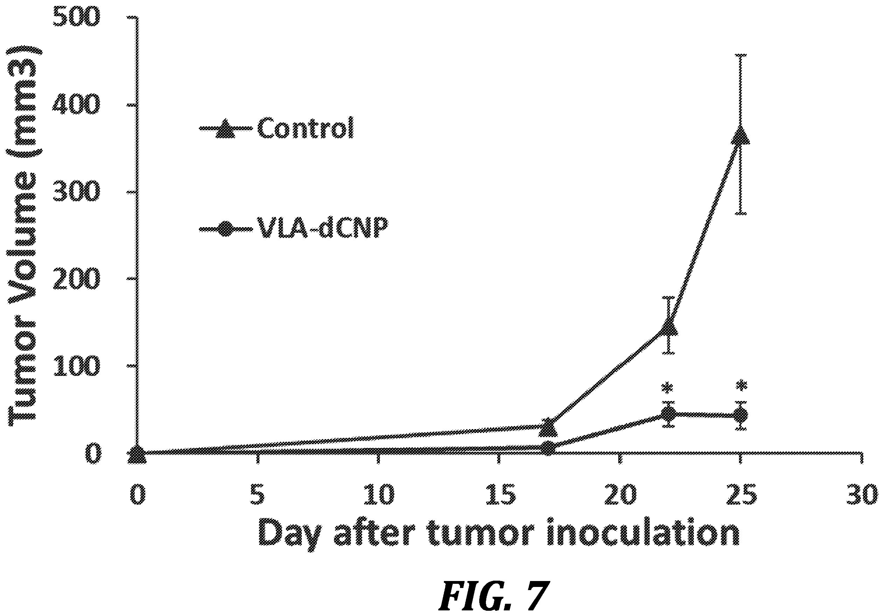

FIGURE 7 is a graph showing that VLA-dCNP suppressed the size of growth of bone cancer in subcutaneous implantation model in mouse.

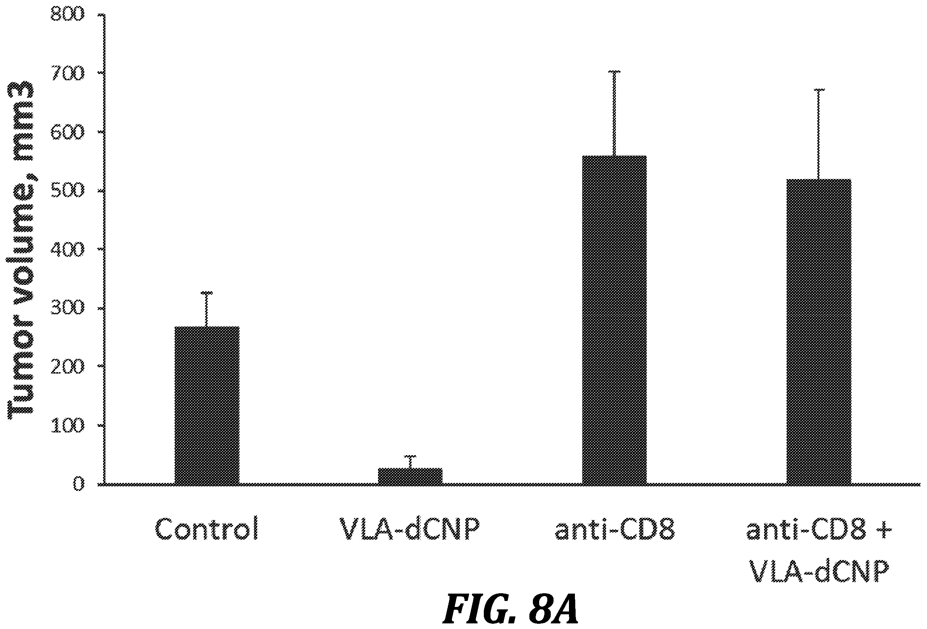

FIGURE 8A is a bar graph showing the effect of VLA-dCNP on bone tumor volume growth with or without cluster of differentiation 8 (CD8) depletion in an orthotopic implantation (femur) model in mice.

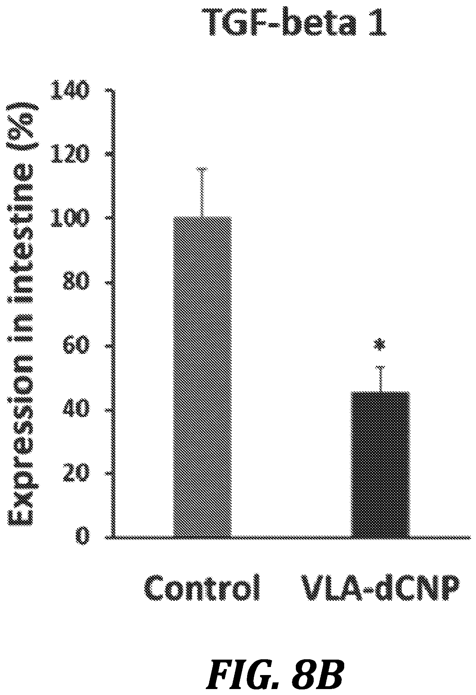

FIGURES 8B-8D are a series of bar graphs showing the effect of VLA-dCNP on activation of immunity in bone cancer subcutaneous implantation mouse model.
FIGURE 8B is a bar graph showing the expression of TGF-beta 1 in a control mouse group compared to a group treated with VLA-dCNP.

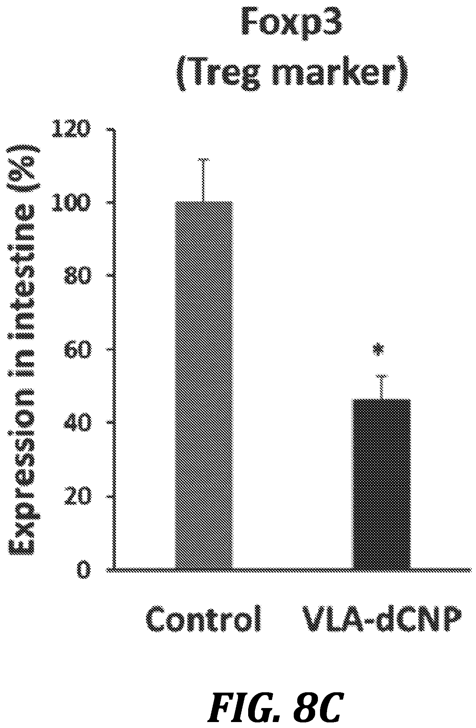

FIGURE 8C is a bar graph showing the expression of Foxp3 in a control mouse group compared to a group treated with VLA-dCNP.

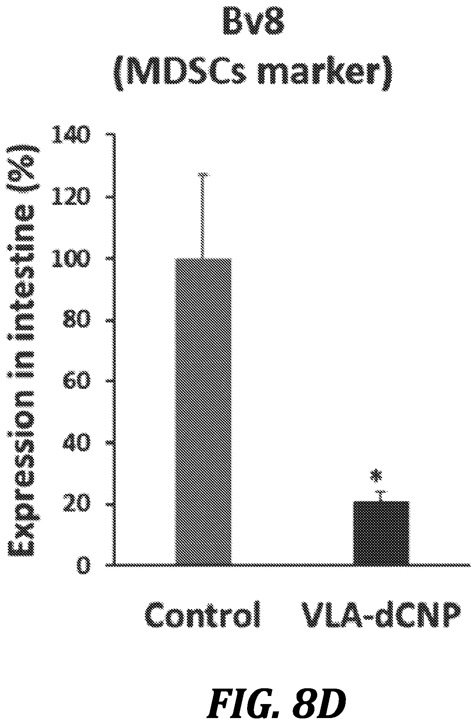

FIGURE 8D is a bar graph showing the expression of Bv8 in a control mouse group compared to a group treated with VLA-dCNP.

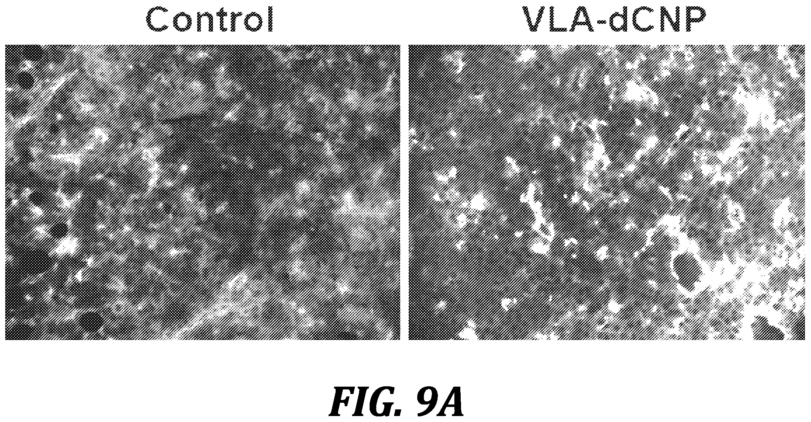

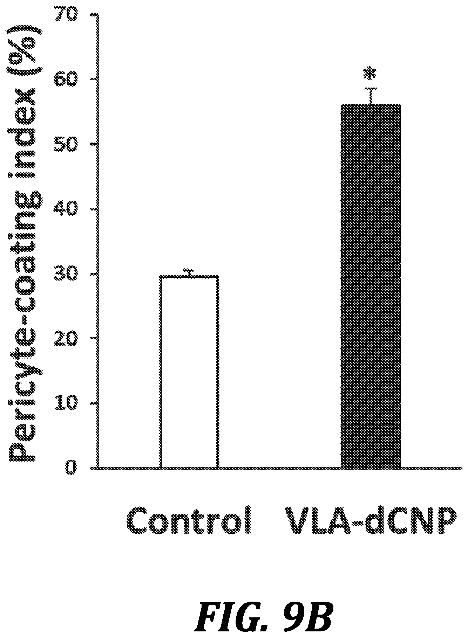

FIGURES 9A and 9B are a series of micrographs and a bar graph showing that VLA-dCNP normalized tumor vascular structure.
FIGURE 9A shows the fluorescence microscope images of red CD31 and green alpha-SMA for a sample treated with a control formulation and a sample treated with BLA-dCNP.
FIGURE 9B is a bar graph showing the % index of pericyte-coating.

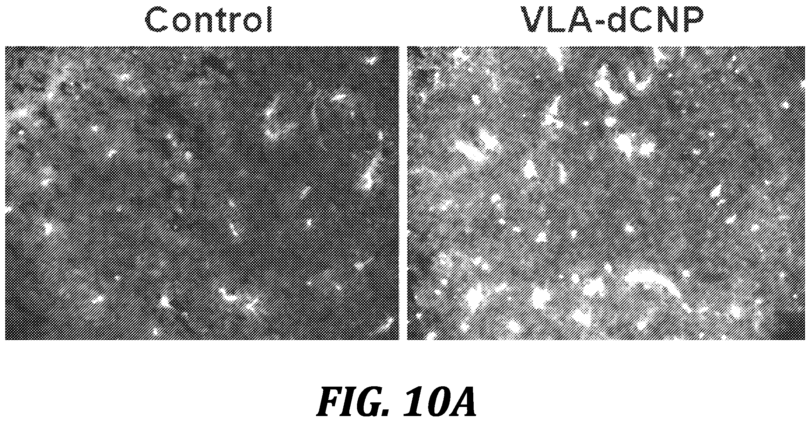

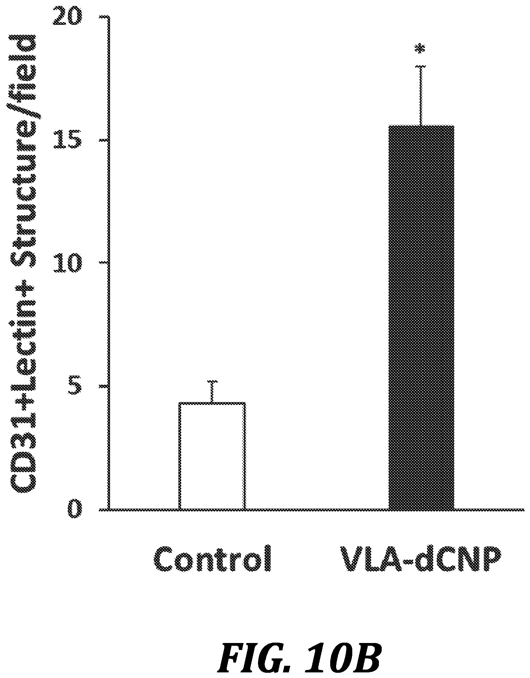

FIGURES 10A and 10B are a series of micrographs and an associated bar graph showing that VLA-dCNP normalized tumor vascular structure.
FIGURE 10A shows the fluorescence microscope images of red CD31 and green lectin for a sample treated with a control formulation and a sample treated with VLA-dCNP.
FIGURE 10B is a bar graph showing the count of CD31 and lectin structure per field; error bars are SEM. Statistical analysis was performed by Student t-tests by using GraphPad Prism *P < 0.05 vs. Control group (n =3).

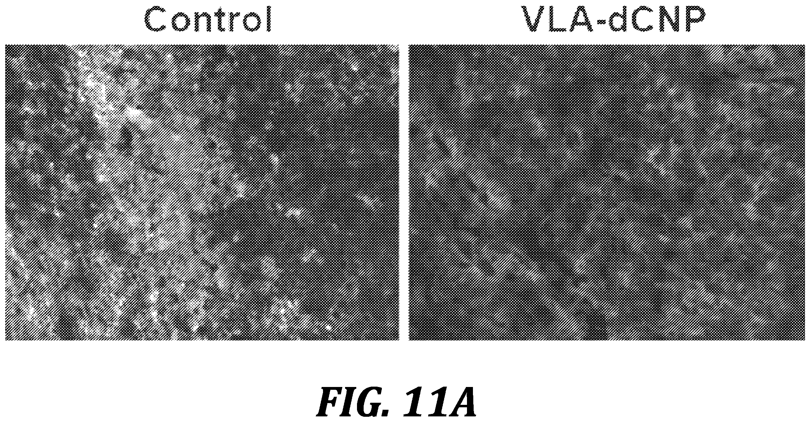

FIGURES 11A-11B are a series of micrographs and associated bar graph showing that VLA-dCNP reduced hypoxia condition in tumor tissue.
FIGURE 11A shows the fluorescence microscope images of red pimonidazole.

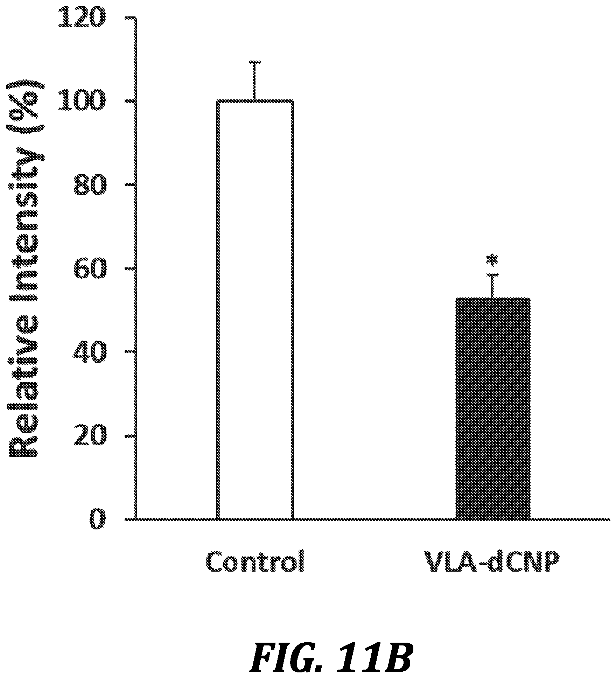

FIGURE 11B is a bar graph showing the percentage of relative intensity of red pimonidazole; error bars are SEM. Statistical analysis was performed by Student t-tests by using GraphPad Prism *P < 0.05 vs. Control group (n =4).

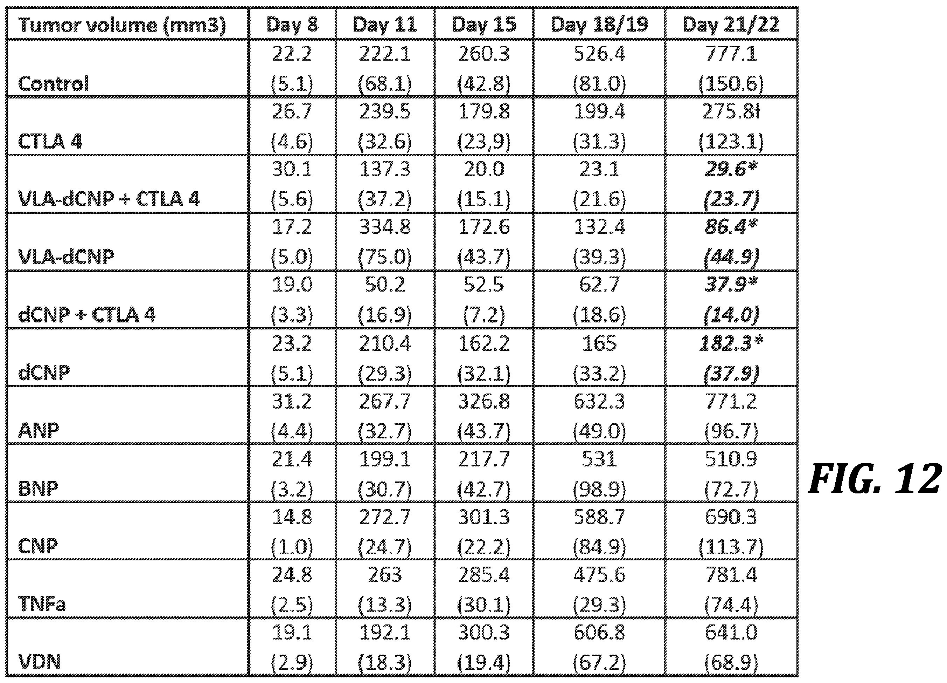

FIGURE 12 is a table showing the tumor size on various days with treatment with the indicated agents; VLA-dCNP and anti-mouse cytotoxic T-lymphocyte-associated protein 4 (CTLA-4) antibody combination therapy suppressed the growth of colon cancer in a subcutaneous implantation model in mouse.

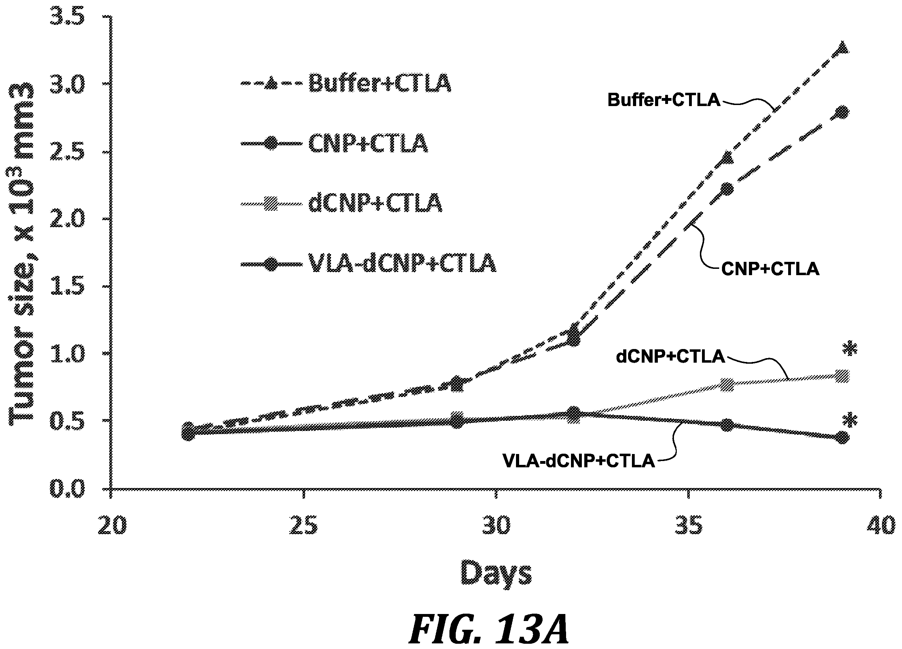

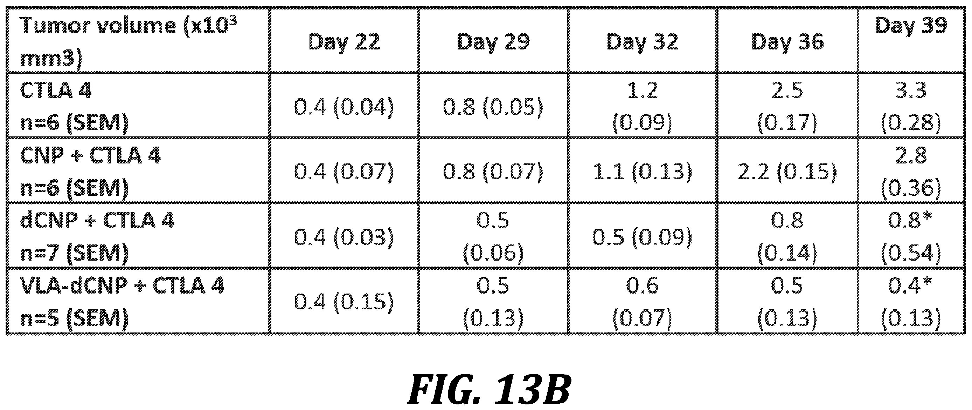

FIGURES 13A and 13B are a graph and associated table showing that VLA-dCNP and anti-(cytotoxic T-lymphocyte-associated protein 4) CTLA-4 antibody combination therapy suppressed the growth of colon cancer in a subcutaneous implantation model in mouse.
FIGURE 13A is a graph showing tumor size as a function of days with treatment with the indicated agents.
FIGURE 13B is a table showing tumor size on selected days with treatment with the indicated agents.

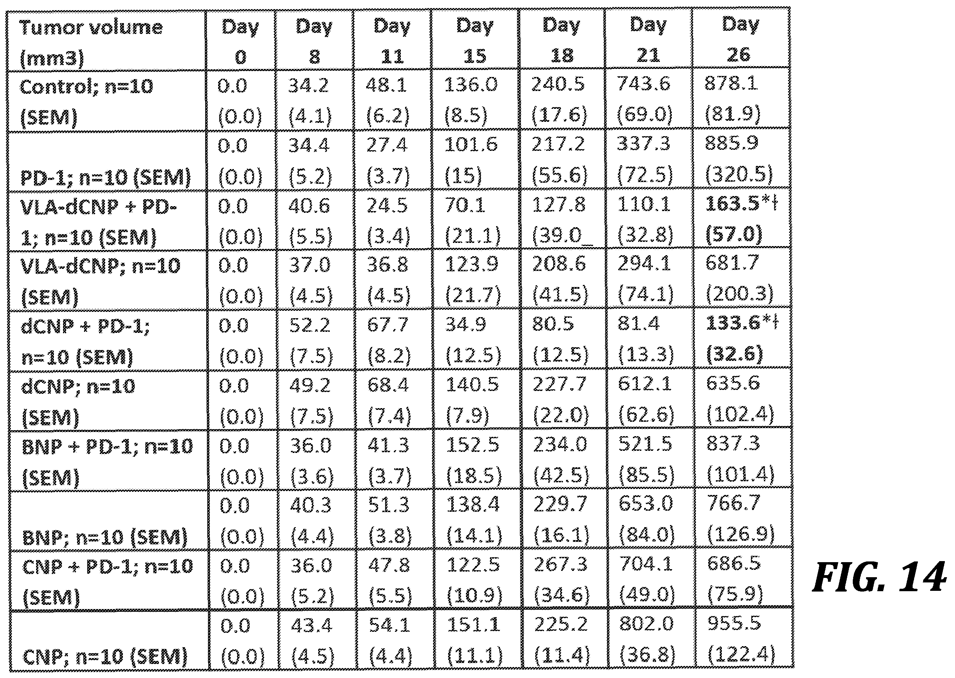

FIGURE 14 is a table showing tumor size on selected days with treatment with the indicated agents; VLA-dCNP and anti-mouse Programmed cell death protein 1 (PD-1) antibody combination therapy suppressed the growth of colon cancer in subcutaneous implantation model in mouse.

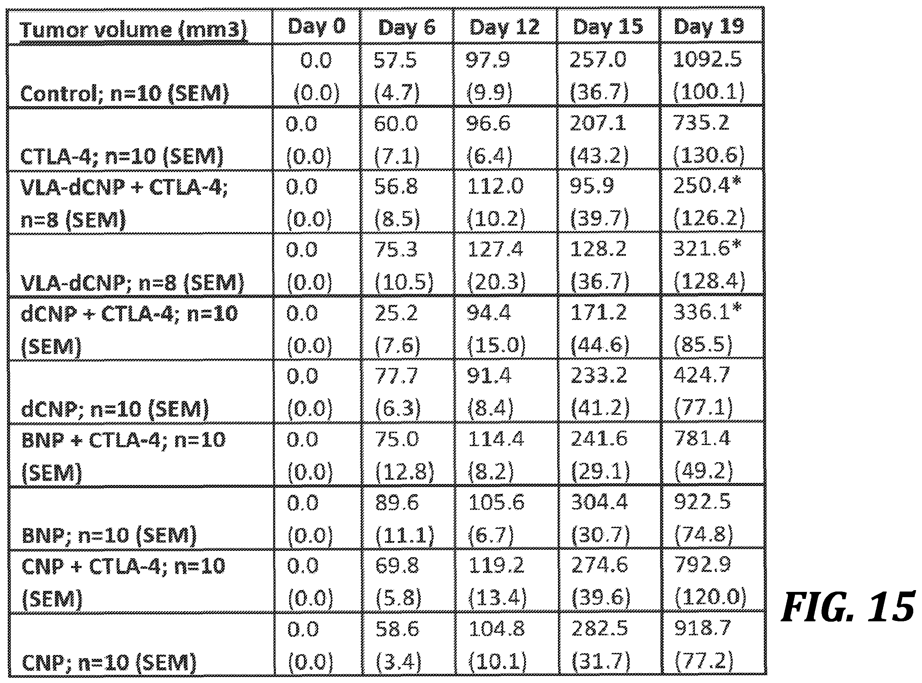

FIGURE 15 is a table showing tumor size on selected days with treatment with the indicated agents; VLA-dCNP and anti-cytotoxic T-lymphocyte-associated protein 4 (CTLA-4) antibody combination therapy suppressed the growth of skin cancer in orthotopic transplantation model in mice.

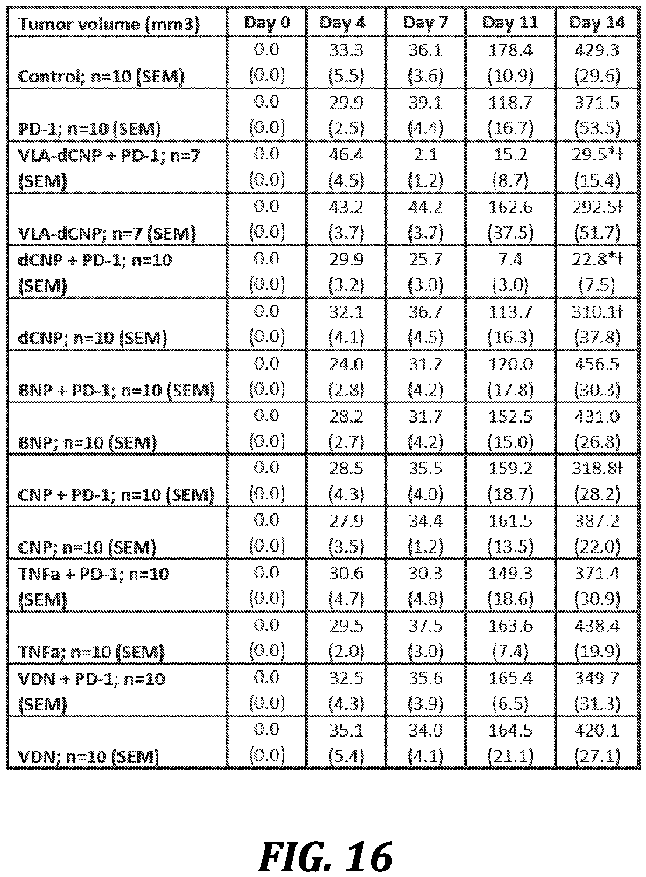

FIGURE 16 is a table showing tumor size on selected days with treatment with the indicated agents; VLA-dCNP or dCNP and anti-mouse Programmed cell death protein 1 (PD-1) antibody combination therapy suppressed mouse breast cancer in orthotopic transplantation model in mice.

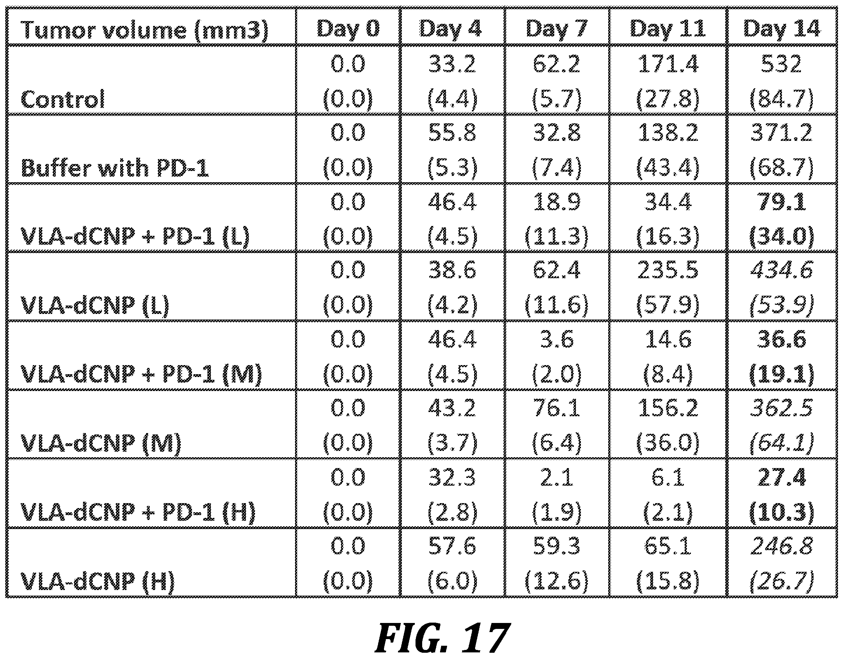

FIGURE 17 is a table showing tumor size on selected days with treatment with the indicated agents; VLA-dCNP and anti-mouse PD-1 (Programmed cell death protein 1) antibody combination therapy suppressed the growth (volume) of breast cancer in orthotopic transplantation model in mice showing dose response manner.

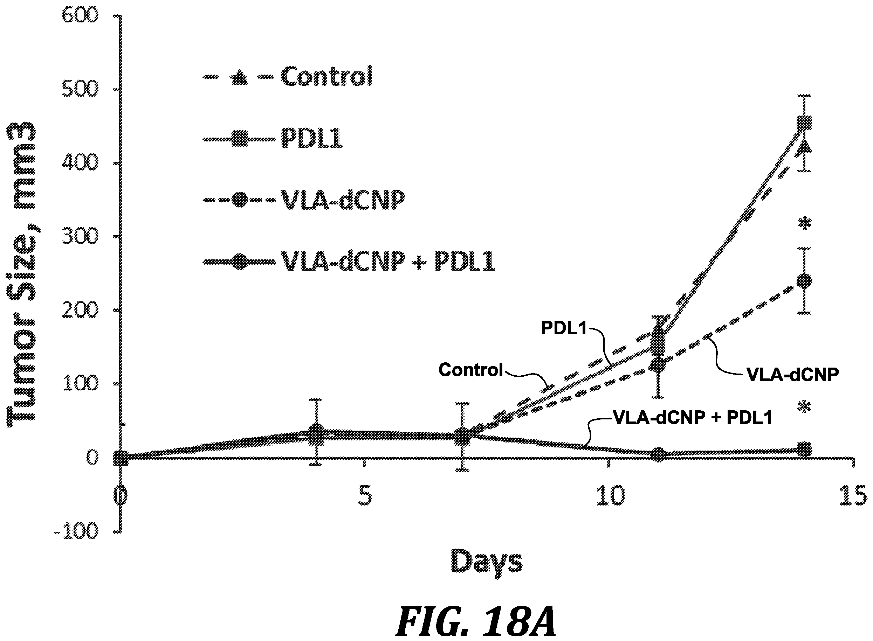

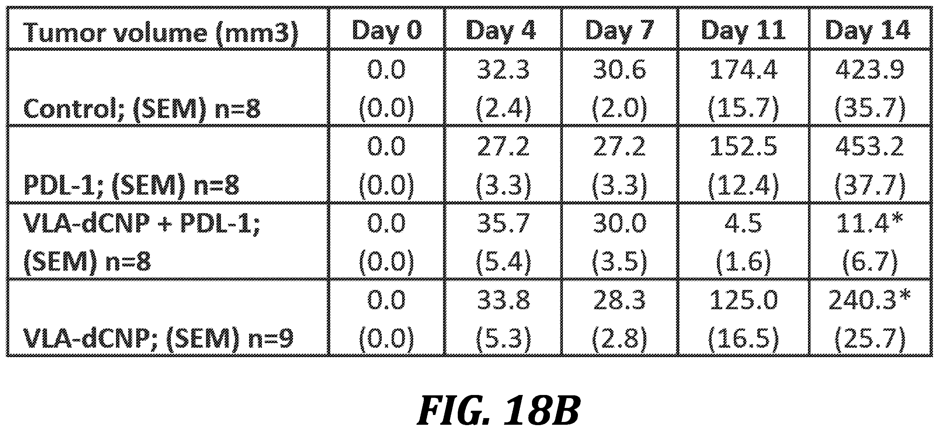

FIGURES 18A and 18B are a graph and associated table showing that VLA-dCNP and anti-Programmed death-ligand (PD-L1) antibody combination therapy suppressed the growth (volume) of breast cancer in orthotopic transplantation model in mice.
FIGURE 18A is a graph showing tumor size as a function of days with treatment with the indicated agents.
FIGURE 18B is a table showing tumor size on selected days with treatment with the indicated agents.

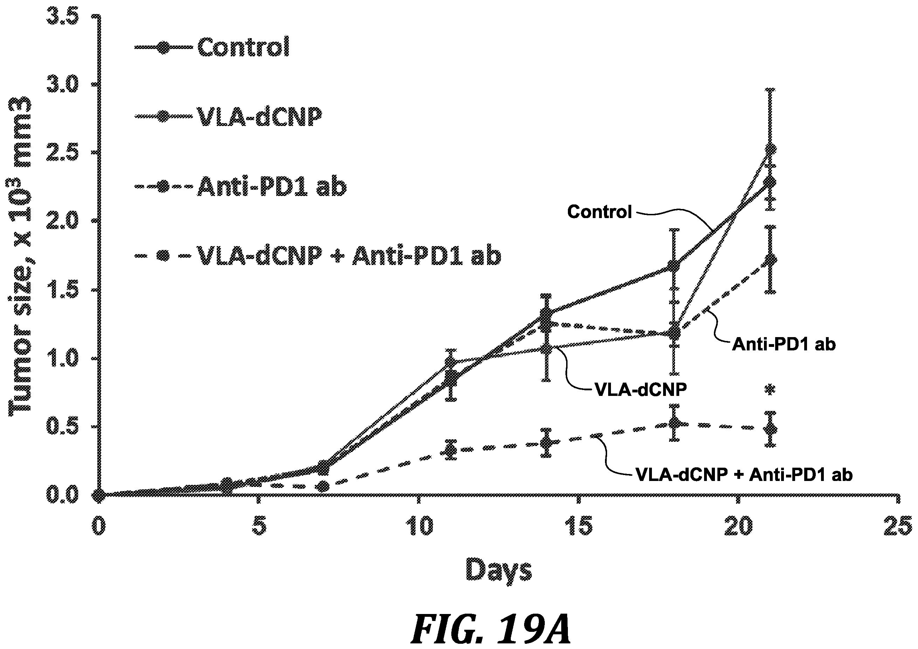

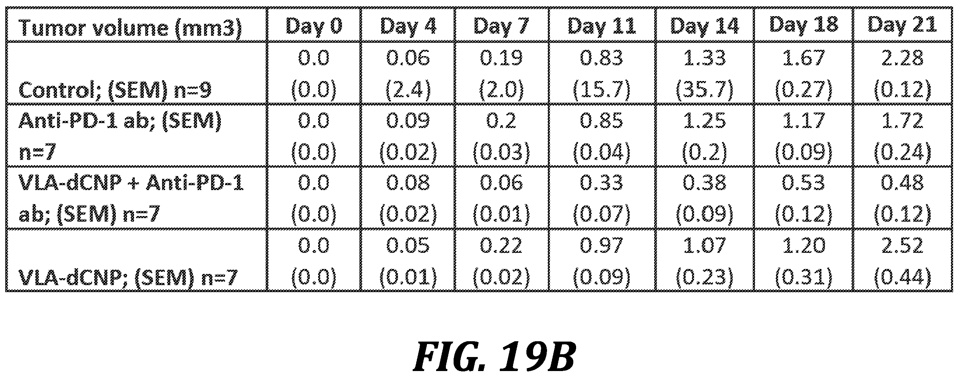

FIGURES 19A and 19B are a graph and associated table showing that VLA-dCNP and anti-PD-1 antibody combination therapy suppressed the growth (volume) of breast cancer in orthotopic transplantation model in mice.
FIGURE 19A is a graph showing tumor size as a function of days with treatment with the indicated agents.
FIGURE 19B is a table showing tumor size on selected days with treatment with the indicated agents.

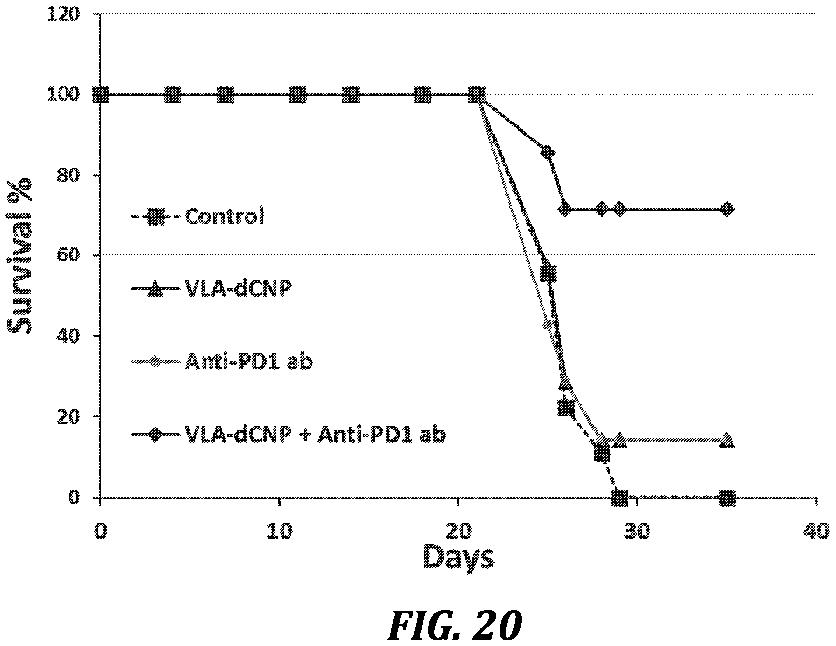

FIGURE 20 is a graph showing that VLA-dCNP and anti-PD-1 antibody combination therapy suppressed the growth of breast cancer and improve the survival in mice.

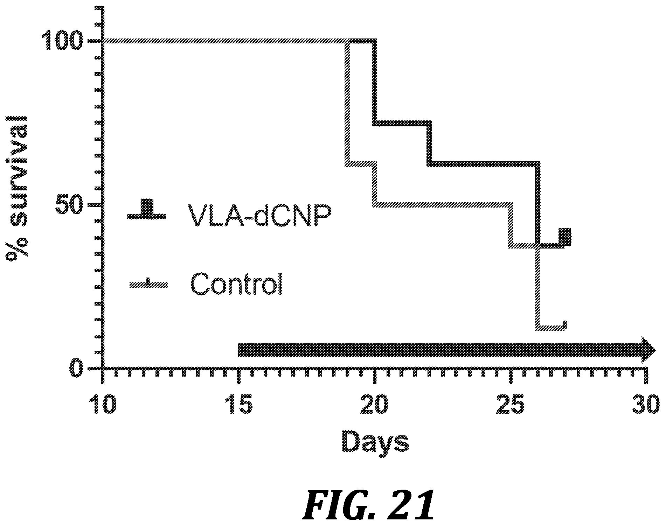

FIGURE 21 is a Kaplan-Meier curve showing that VLA-dCNP treatment improves the survival in mice with osteosarcoma in the tibial bone.

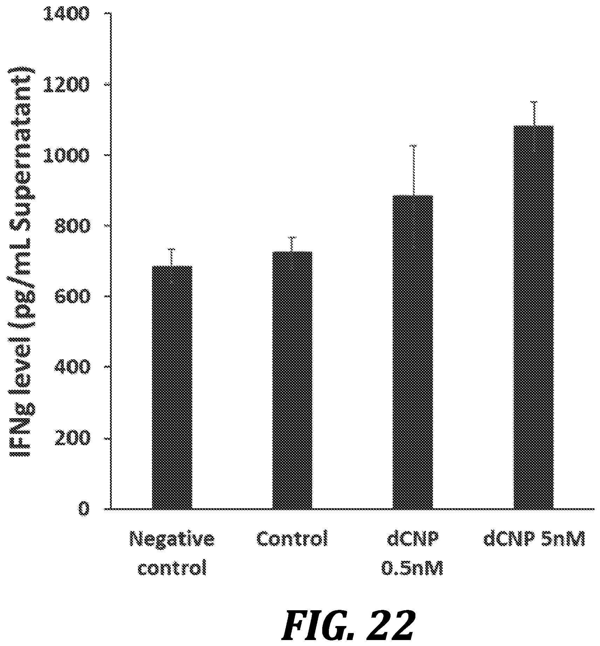

FIGURE 22 is a graph showing dCNP increases interferon gamma (IFNg) production in splenocytes exposed to cultured LM8 mouse osteosarcoma cancer cell line in a dose dependent manner.

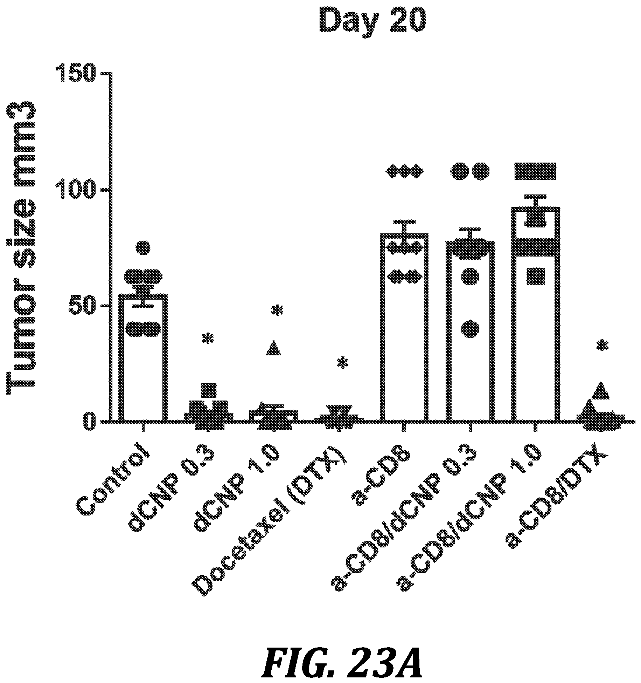

FIGURE 23A is a bar graph showing tumor size at Day 20 after a 14-day treatment with various test compositions starting at day 6. Error bars are SEM and individual dots represent individual animals in the group.

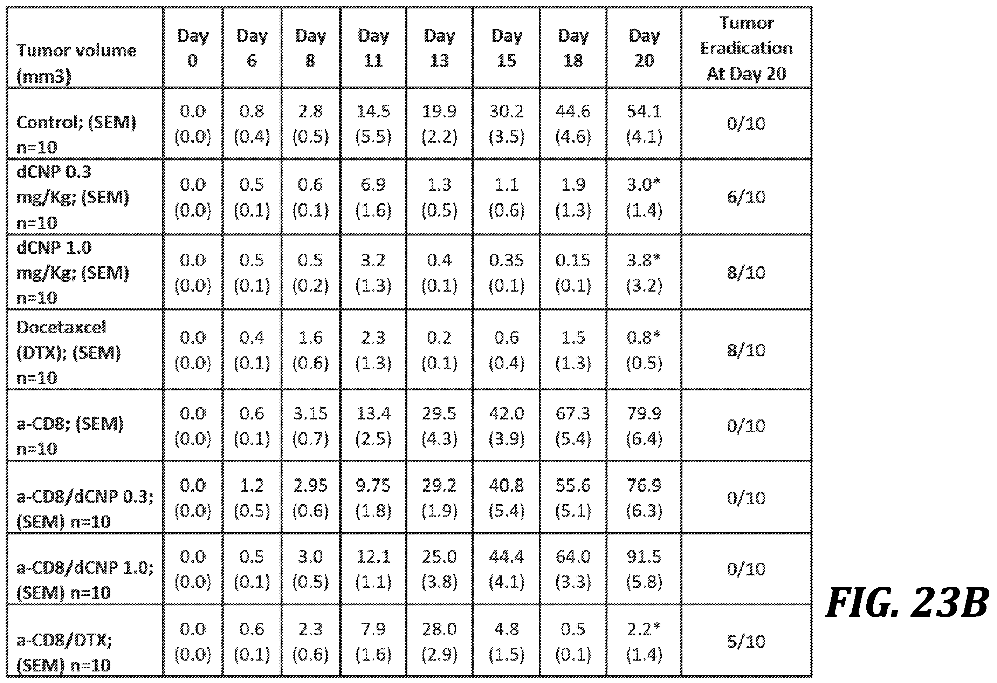

FIGURE 23B is a table showing tumor growth over the treatment period along with tumor eradication at Day 20.

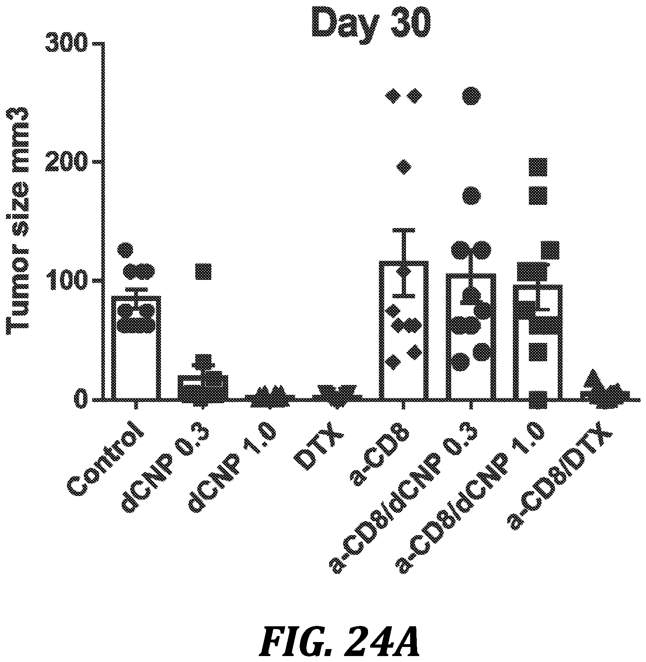

FIGURE 24A is a bar graph showing prostate tumor eliminating action of dCNP even when the when administration was started at much later stage (tumor size was ~70mm³; at day 19). Also shown is that dCNP is as effective as the cytotoxic chemotherapeutic agent in eliminating tumor. Error bars are SEM and individual dots represent individual animals in the group.

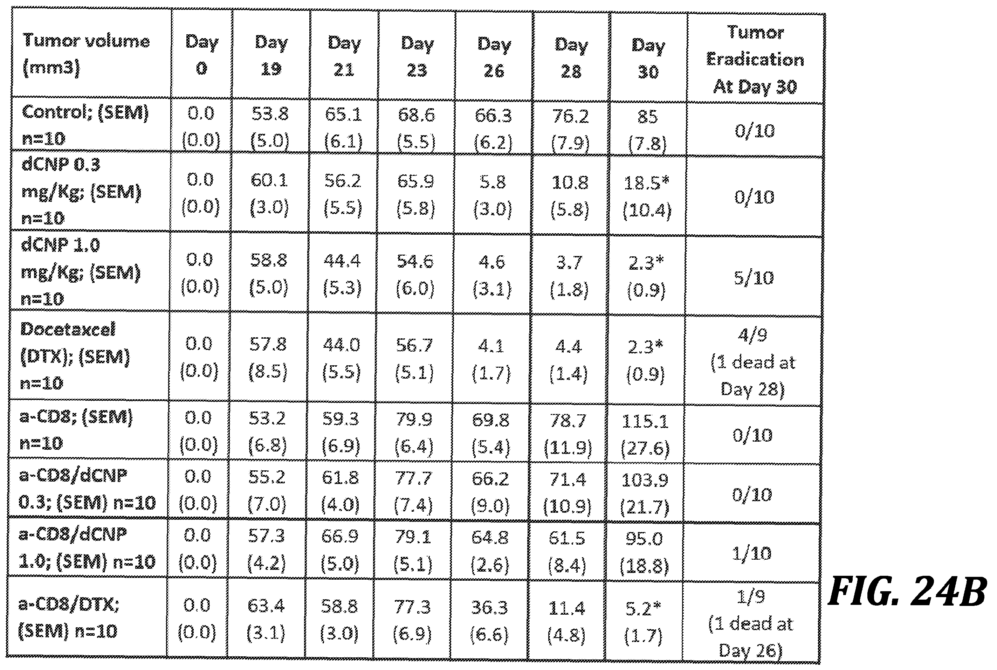

FIGURE 24B is a table showing prostate cancer growth over the treatment period along with tumor eradication at Day 30.

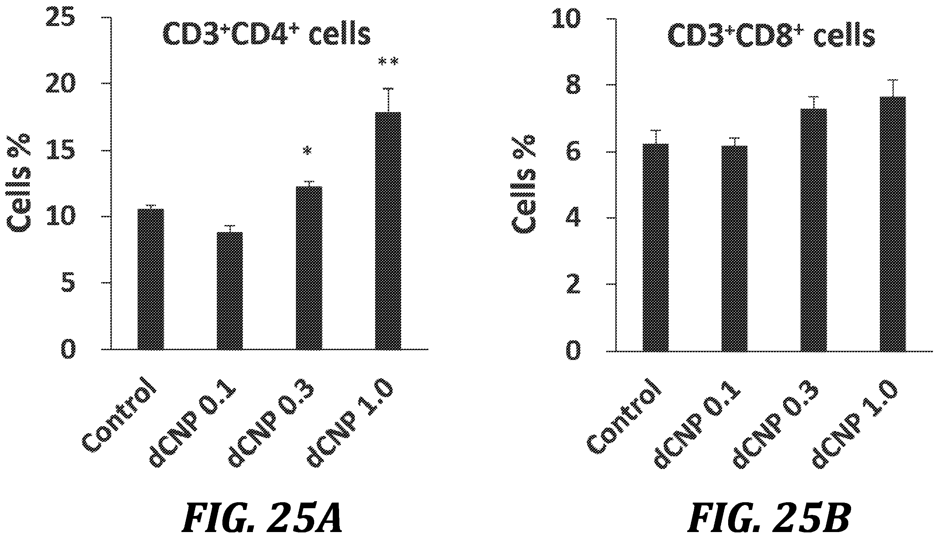

FIGURE 25A is a bar graph showing that that even in normal healthy mice, dCNP can activate the immune system as seen by an increase in T-cells (CD4) in blood.
FIGURE 25B is a bar graph showing that that even in normal healthy mice, dCNP can activate the immune system as seen by an increase in cytotoxic (CD8) T-cells in blood.

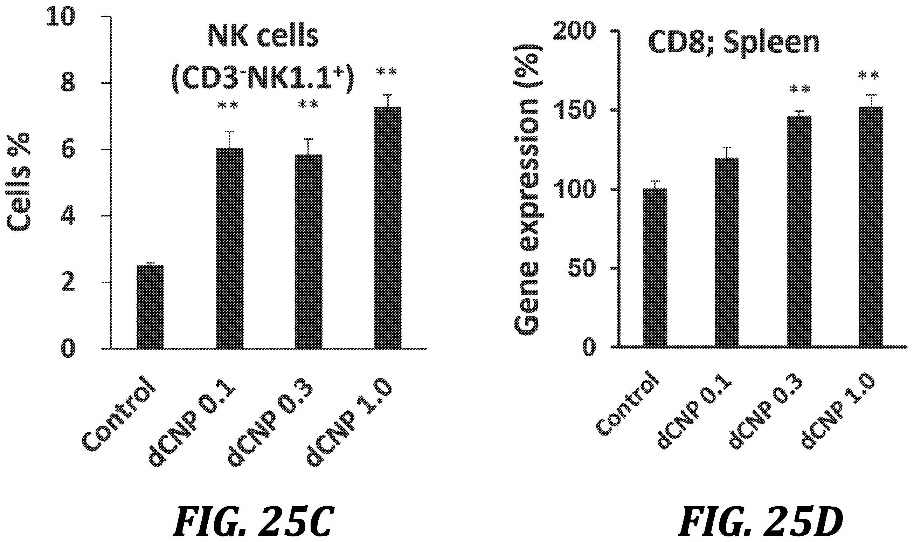

FIGURE 25C is a bar graph showing that that even in normal healthy mice, dCNP can activate the immune system as seen by an increase in natural killer (NK) cells in blood.
FIGURE 25D is a graph showing that that even in normal healthy mice, dCNP can activate the immune system as seen by an increase in CD8 gene expression in the spleen.

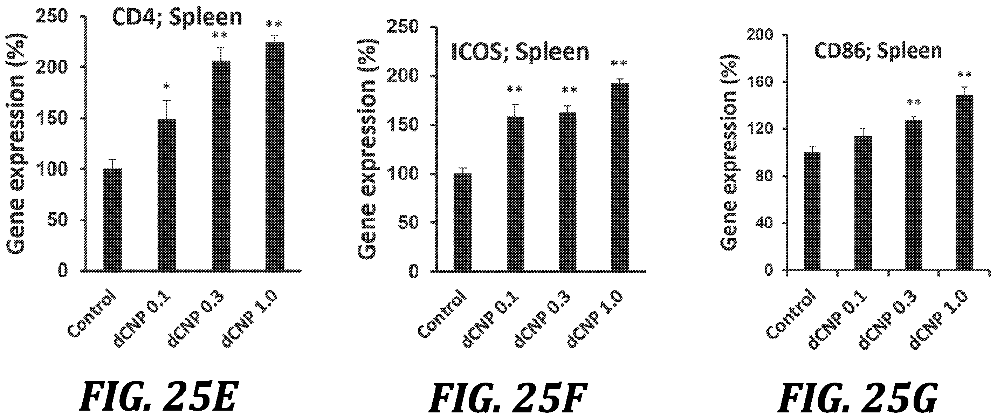

FIGURE 25E is a graph showing that that even in normal healthy mice, dCNP can activate the immune system as seen by an increase in CD4 gene expression in the spleen.
FIGURE 25F is a graph showing that that even in normal healthy mice, dCNP can activate the immune system as seen by an increase in ICOS gene expression in the spleen. ICOS is inducible T-cell co-stimulator and is an immune checkpoint protein and its expression indicates immune activation.
FIGURE 25G is a graph showing that that even in normal healthy mice, dCNP can activate the immune system as seen by an increase in CD86 gene expression in the spleen. CD86, along with CD80 provides costimulatory signals necessary for T cell activation and survival and its expression confirms immune activation.

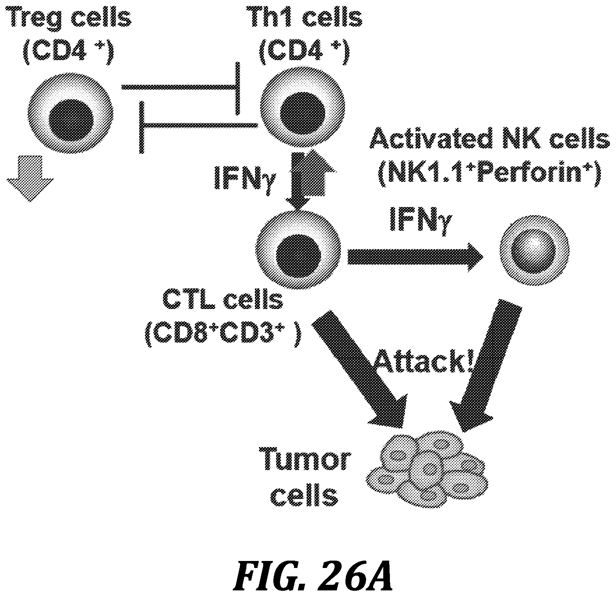

FIGURE 26A shows diagram of various immune cells, their mechanistic interaction for immune activation, and production of interferon gamma (IFNg) cytokine that triggers CD8 and NK cells anti-tumor activity or attack to eliminate of cancer cells. The grey arrows indicate increase (pointing up) or decrease (pointing down) in number of cells and dark arrows points to cells that it develops into in the presence of IFNg. Regulatory T cells (Tregs) are a specialized subpopulation of T cells that act to suppress T helper 1 (Th1) cells immune response (represented by horizontal T), thereby maintaining homeostasis and self-tolerance. Treg inhibits T cell proliferation and cytokine production to suppress the immune system by inhibiting development of Th1 into CD8+ cytotoxic T cell (CTL) and under normal condition prevents autoimmunity. Additionally, Th1 cells provide helper functions to other cells of the immune system-especially the antigen-presenting cells (APCs) such as macrophages, dendritic cells, and B cells-and are important for their activation and maturation.

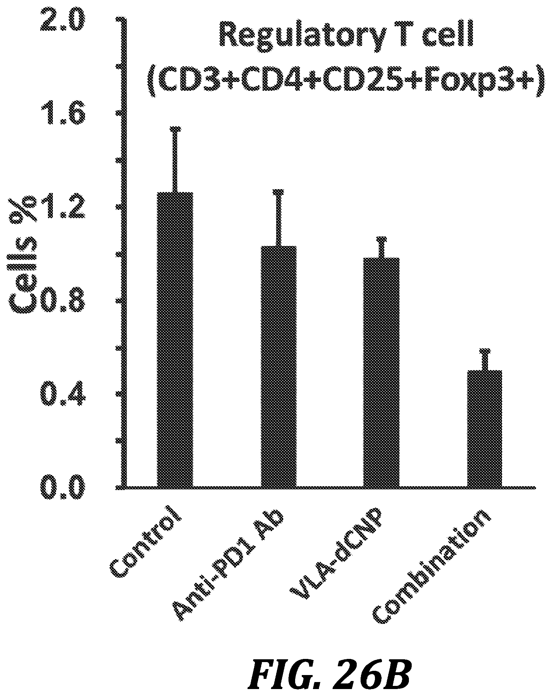

FIGURE 26B is a bar graph showing suppression of Tregs cells in an E0771 mouse breast cancer model.

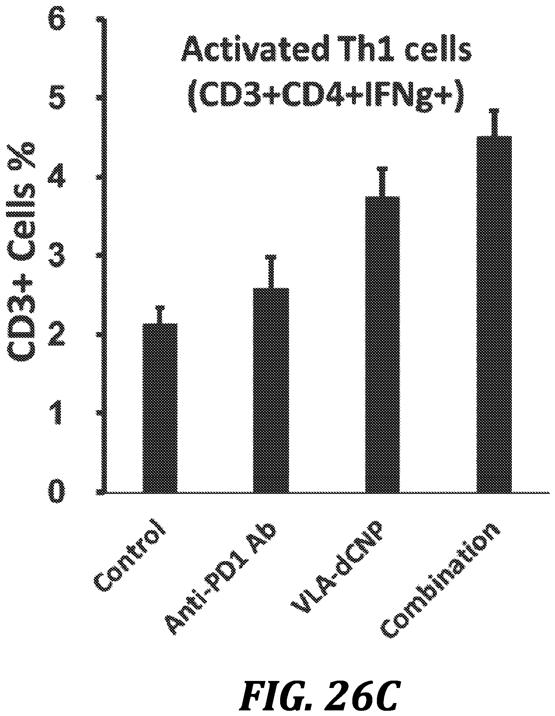

FIGURE 26C is a bar graph showing an increase of activated Th1 cells where VLA-dCNP alone is significantly more effective than Anti-PD1 alone.

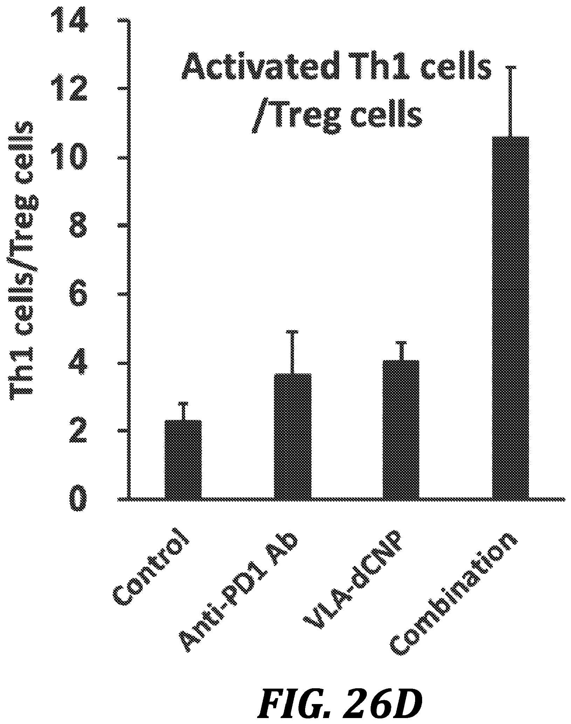

FIGURE 26D is a bar graph showing the ratio of Th1/Treg cells where VLA-dCNP alone is significantly more effective than Anti-PD1 alone in an E0771 mouse breast cancer model. Surprisingly when both dCNP and Anti-PD1 antibody are combined a synergistic effect was observed, where the effect is much greater than the sum of the effects of administration of dCNP alone and Anti-PD1 alone.

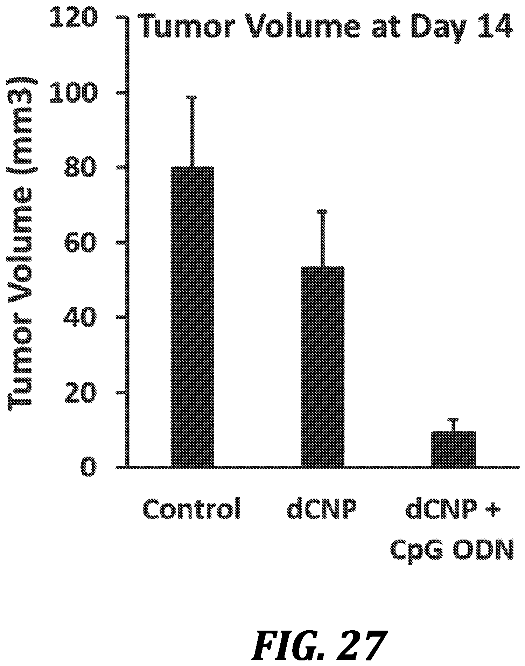

FIGURE 27 is a bar graph showing the significant tumor suppression when dCNP is combined with adjuvant CpG ODN-TLR9 Agonists in the mice breast cancer model.

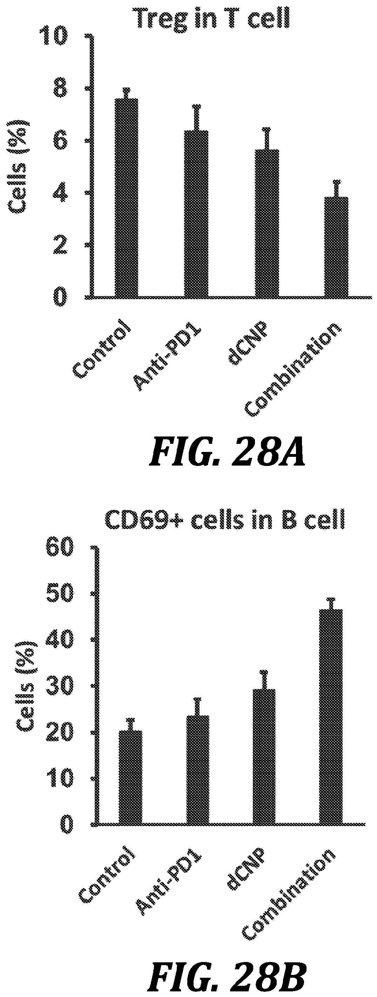

FIGURES 28A-28D are bar graphs showing that dCNP, anti-PD1 antibody, and combination of both are effective immune activators that can decrease tumor volume and Treg cells and increase CD69+ cells in mice breast cancer model.
FIGURE 28A is a bar graph showing that dCNP, anti-PD1 antibody, and combination of both are effective immune activators that can decrease Treg.
FIGURE 28B is a bar graph showing that dCNP, anti-PD1 antibody, and combination of both are effective immune activators that can increase CD69+ population in B cell.

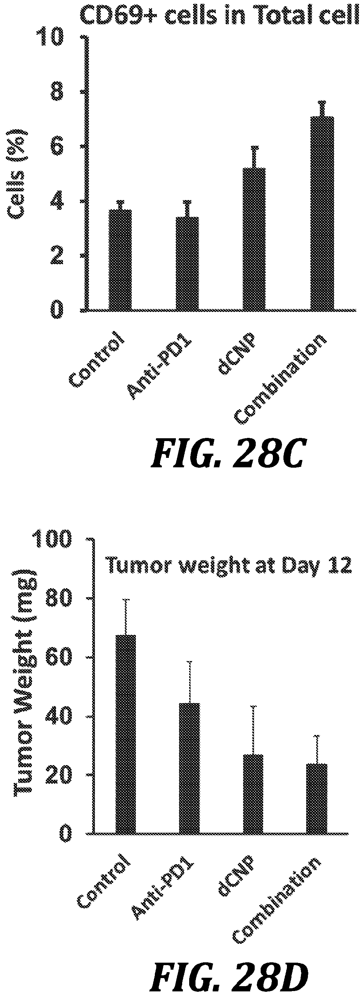

FIGURE 28C is a bar graph showing that dCNP, anti-PD1 antibody, and combination of both are effective immune activators that can increase CD69+ population in total cell.
FIGURE 28D is a bar graph showing that dCNP, anti-PD1 antibody, and combination of both are effective immune activators that can decrease tumor weight Suppression of Treg (Top Left) while increasing CD69+ (Top Right and Bottom Left) cell population is consistent with immune activation causing tumor volume suppression (Bottom Right).

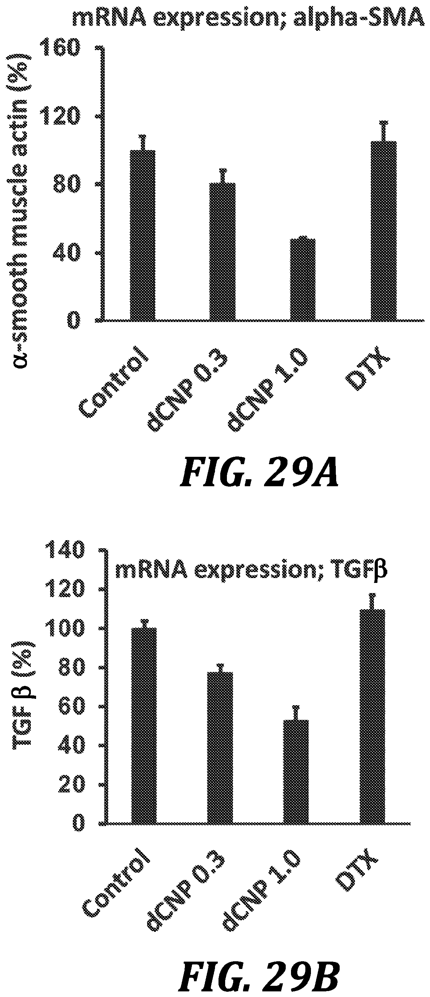

FIGURE 29A is a bar graph showing dCNP, but not Docetaxel (DTX) or buffer control, inhibit alpha-smooth muscle actin gene expression within the prostate tumor of a mice cancer model. The α-SMA is the marker of tissue fibrogenesis. TGFβ is a well-known mediator of fibrogenesis and upregulated and activated in fibrotic disease (*see, e.g.,* Growth Factors, 2011 29(5), 196-202).
FIGURE 29B is a bar graph showing dCNP, but not Docetaxel (DTX) or buffer control, inhibit TGFβ gene expression within the prostate tumor of a mice cancer model.

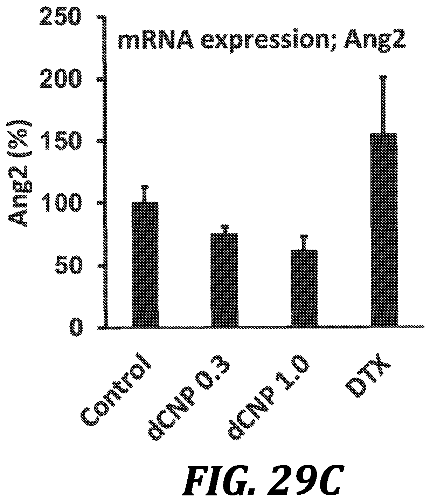

FIGURE 29C is a bar graph showing dCNP, but not Docetaxel or buffer control, inhibit Ang 2 gene expression within the prostate tumor of a mice cancer model. Ang2 inhibits the tumor vascular stabilization through antagonizing Ang1/Tie2 axis. Inhibition of Ang 2 expression stabilizes vasculature, enhances access to tumor, and improves drug delivery (*see, e.g.,* Cancer Cell, 2016 Vol 30, 953-967). dCNP stabilizes (Bottom graph) the tumor vasculature and creates a better anti-tumor tumor microenvironment for the immune system.

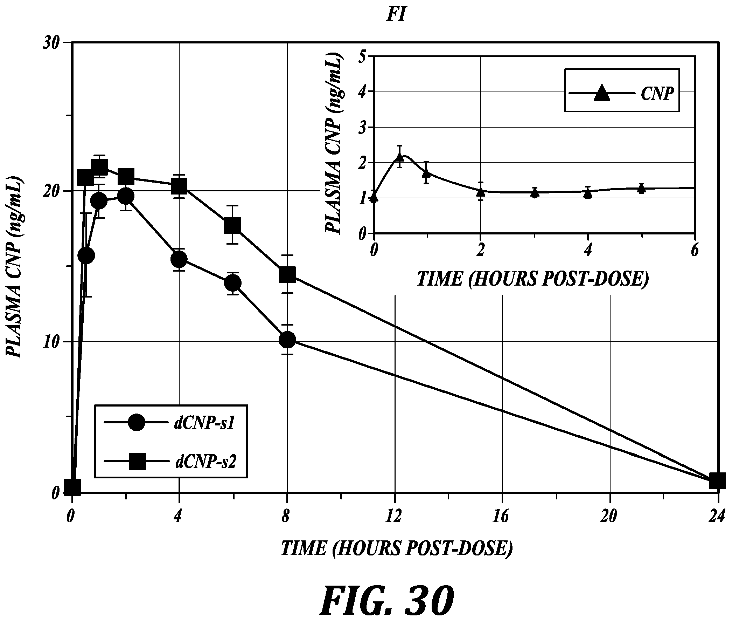

FIGURE 30 is a graph of plasma CNP [mean (SEM); n= 5] in CD-1 mice after subcutaneous administration of 2.0mg/Kg of CNP derivative s1 (dCNP-s1), and CNP derivative s2 (dCNP-s2). The inset shows the low plasma level of CNP (diamond) when native CNP was administered. Error bars represent standard error of the mean of n=5 plasma samples. Baseline CNP level prior to administration was 0.391 (0.02) ng/mL [mean (SEM); n=10]. Long acting dCNP-s1 and dCNP-s2 provides 10-fold higher blood level of CNP in a sustain manner (at least 8 hours) than native CNP when given at similar dose weight/Kg dose.

### DETAILED DESCRIPTION

The present disclosure relates to methods of using a NPRB-binding immune-activator for treating a subject having abnormal vasculature, in any tissue or organ, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. In some embodiments, the subject further has a low number of cytotoxic T cells, a low number of activated NK cells, or both a low number of cytotoxic T cells and a low number of activated NK cells. In some embodiments, the present disclosure relates to a method of increasing cytotoxic T-cells and/or activated NK cells, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. In some embodiments, the subject has a condition (i) to (viii): (i) a low number of cytotoxic T cells, (ii) a low number of activated NK cells, (iii) a high number of Treg cells, (iv) a high level of expression of TGFβ, (v) a high level or expression of Foxp3, (vi) a high number of myeloid-derived suppressor cells or MDSCs, (vii) a high level or expression of Bv8; or (viii) any combination thereof; or the subject is in need of (ix) to (xvi): (ix) an increase in a number of cytotoxic T-cells; (x) an increase in activated NK cells; (xi) a decrease in a number of Treg cells; (xii) a decrease in TGF-β expression; (xiii) a decrease in Foxp3 expression; (xiv) a decrease in a number of myeloid-derived suppressor cells (MDSCs); (xv) a decrease in Bv8 expression, or (xvi) any combination thereof. In some embodiments, the subject has cancer. The NPRB-binding immune-activator can normalize vascularization or provide an increase in pericyte coating index by at least 10% (*e.g.,* by at least 15%, or by at least 20%). In some embodiments, the NPRB-binding immune-activator can normalize vascularization (*e.g.,* within cancer tissue), decrease the tumor size, reduce hypoxia within the tumor tissue, increase the number of cytotoxic T cells within the cancer tissue, increase the number of activated NK cells within cancer tissue, decrease Treg cells, decrease myeloid-derived suppressor cells, decrease expression of TGFβ, decrease expression of Foxp3, decrease Bv8 expression, inhibit immune checkpoint activity within cancer tissue, and/or increase survival, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof.

In some embodiments, when administered to the subject in need thereof, the therapeutically effective bolus dose of the composition provides an increase in the number of cytotoxic T cells and/or NK cells of at least 15% (*e.g.,* of at least 20%, or of at least 30%) above the level prior to administration of the composition or above the level in a healthy subject. When administered to the subject, the composition does not decrease blood pressure by more than 20% (*e.g.,* by more than 15%, by more than 10%, or by more than 5%) of a baseline blood pressure measurement, where the baseline blood pressure measurement is an average blood pressure prior to administration of the composition, and the composition increases plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject). The compositions can include additional therapeutic agent(s) that also inhibit(s) immune checkpoint protein activity. In some embodiments, the compositions of the present disclosure can be administered prior to, concurrently with, and/or subsequent to radiation treatment, chemotherapy treatment, surgical treatment, CAR-T cell treatment, and/or antibody treatment.

In one aspect, the present disclosure features a method of treatment for cancer that includes increasing cyclic-GMP level in a plasma, and reducing or eliminating cancers. There is nothing in the art that indicates that bolus administration of a compound can activate natriuretic peptide receptor B (NPRB or NPR2) in a sustained manner and can activate the immune system to attack tumor or cancer, increase the number of cytotoxic T-cells and/or activated NK cells within the tumor tissue, reduce the number of immune suppressor cells (Treg cells), decrease immune suppressor cytokines (Transforming growth factor beta or TGFβ), decrease Foxp3 (Treg marker) expression, decrease Bv8 (marker for myeloid-derived suppressor cells or MDSCs marker) expression, and/or causes a vasculature normalization or an increase in pericyte coating index by at least 10% (*e.g.,* by at least 15%, or by at least 20%) in the tumor tissue that allows access of anti-cancer drugs in the tumor tissue and reduces hypoxia (hypoxia facilitate tumor growth/malignancy and immune resistance).

Additionally, there is nothing in the art that indicates that bolus administration of long acting CNP, very long acting CNP, long acting CNP derivative, very long acting CNP derivative, long acting NPRB agonist, or very long acting NPRB agonist would not cause a significant drop in blood pressure while providing a sustained plasma cyclic-GMP elevation that can be used to treat cancer. The present disclosure discloses a treatment of cancer including administering bolus dose of long acting CNP, very long acting CNP, long acting CNP derivative, very long acting CNP derivative, long acting NPRB agonist, or very long acting NPRB agonist that does not cause a significant drop in blood pressure while providing a sustained plasma cyclic-GMP elevation. Furthermore, sustained activation of NPRB by bolus administration of long acting CNP, very long acting CNP, long acting CNP derivative, very long acting CNP derivative, long acting NPRB agonist, or very long acting NPRB agonist can activate the immune system to attack tumor tissue and/or cause infiltration of tumor area with cytotoxic T cells.

There is nothing in the art that would suggest to a person of skill in the art to treat cancer to use a combination of immune checkpoint inhibitors or CAR T-cell therapy with any of long acting CNP, very long acting CNP, long acting CNP derivative, very long acting CNP derivative, long acting NPRB agonist, or very long acting NPRB agonist to improve the efficacy of other immune checkpoint inhibitors or CAR T-cell therapy for the treatment of cancer or malignancy. In some embodiments, the present disclosure features the use of long acting CNP, very long acting CNP, long acting CNP derivative, very long acting CNP derivative, long acting NPRB agonist, or very long acting NPRB agonist in combination with other immune checkpoint inhibitor(s) in general and/or CAR T-cell therapy in general. For example, the immune check point inhibitor can be from a blocker of CTLA-4, PD-1, or PD-L1, such as but not limited to ipilimumab, tremelimumab, labrolizumab, nivolumab, pidilizumab, AMP-244, MEDI 4736, and/or MPDL3280A.

The present disclosure features the use of long acting derivatives of endogenous peptide or long acting NPRB agonists. In use, the long acting derivatives of endogenous peptide or long acting NPRB agonists are safe and act as enhancers of the anti-tumor function of the immune system and/or vascular normalization. The methods of the present disclosure provide a treatment of cancer by activating the immune system in general without limiting its use to a specific cancer.

Unlike radiation or chemotherapy treatments that have severe side effects with limited efficacy, the long acting NPRB activator of present disclosure has no measurable or observable undesirable side effects that aggravate suffering of the cancer patients or those suffering from malignancy. The present disclosure demonstrates that the bolus administration of long acting CNP or long acting NPRB agonist is effective in the treatment of various cancers or malignancies and when used combination with other immune checkpoint inhibitors, there is an improvement in the overall efficacy in the treatment without additional side effects when compared to the same treatment without the bolus administration of long acting CNP or long acting NPRB agonist. It is believed that a synergistic effect can be observed when immune checkpoint inhibitors are used in combination with bolus administration of long acting CNP or long acting NPRB agonist of the present disclosure, such that the therapeutic effect of the combination of immune checkpoint inhibitors with the long acting CNP or long acting NPRB agonist is greater than the cumulative therapeutic effects of treatment with only an immune checkpoint inhibitors or with only the long acting CNP or long acting NPRB agonist (*see, e.g.,* FIGURES 13, 14, 16, 17, 18, 19, 20, 26B-26D, and 28A-28D). In some embodiments, the dosage of the immune checkpoint inhibitors can be decreased when used in conjunction with the bolus administration of long acting CNP or long acting NPRB agonist. Furthermore, the present disclosure demonstrates the bolus administration of long acting CNP or long acting NPRB agonist can provide improvement in the overall efficacy of CAR T-cell therapy in the treatment various cancers or malignancies without additional side effects in the overall treatment, none of which has been shown by the state of the art.

It is believed that nothing is known regarding improving efficacy of immune checkpoint inhibitors such as drugs or antibodies that blocks CTLA-4 (cytotoxic T lymphocyte associated protein 4), PD-1 (programmed cell death protein 1), or PD-L1 (programmed death ligand 1), when administered in conjunction with a bolus dose of long acting CNP, long acting CNP derivative, very long acting CNP, very long acting CNP derivative, long acting NPRB agonist, or very long acting NPRB agonist. The present disclosure presents the following surprising discoveries: 1) CNP can be modified, derivatized, or formulated such that the resulting formulation, when administered as a bolus dose, can increase plasma cyclic GMP in a sustained manner that does not cause a drop in blood pressure at therapeutically effective doses or higher; 2) bolus administration of a NPRB agonist (*i.e.,* long acting CNP, long acting CNP derivative, very long acting CNP, very long acting CNP derivative, long acting NPRB agonist, or very long acting NPRB agonist) can increase plasma cyclic-GMP in a sustained manner for at least 6 hours; 3) bolus administration of a NPRB agonist as defined above can normalize cancer tissue vasculature that can reduce hypoxic inflammation and immune resistance (hypoxia promotes tumor malignancy and immune resistance); 4) bolus administration of a NPRB agonist as defined above can increase the number of cancer-killing cytotoxic T cells within cancer tissue that can assist in eliminating cancer, 5) bolus administration of a NPRB agonist as defined above can decrease the number of immune suppressor cells (Treg cell and myeloid-derived suppressor cells) that hinder cancer elimination by the immune system, 6) bolus administration of a NPRB agonist as defined above suppresses expression of TGFβ, Foxp3, and Bv8 (a marker of myeloid-derived suppressor cell or MDSC) indicating that immune suppression against cancer is reduced, and 7) bolus administration of a NPRB agonist as defined above in combination with other immune checkpoint inhibitors can significantly improve the efficacy of immune checkpoint inhibition and efficacy against cancer.

### Definitions

As used herein, the term "cancer" refers to a malignant tissue mass. Malignant tumor cells can "metastasize" (*i.e*., spread) to other parts of the body through the blood and lymphatic system.

As used herein, the term "carcinoma" refers to a cancer that begins in the skin or in tissues that line or cover internal organs.

As used herein, the term "sarcoma" refers to a cancer that begins in bone, cartilage, fat, muscle, blood vessels, or other connective or supportive tissue.

As used herein, the term "lymphoma" refers to cancers that begin in the cells of the immune system.

As used herein, the term "central nervous system or (CNS) cancers" refers to malignancies that begin in the tissues of the central nervous system.

As used herein, the term "vascular normalization" refers to a restoration to a structurally and functionally normal vasculature with a more ordered pattern compared to an abnormal vasculature, in any tissue or organ, which in turn is characterized by a random vasculature where a significant number of arterioles anastomose directly to the venules bypassing the capillary bed, causing local hypoxia. In a variety of diseases, such as cancer, there is uncontrolled new blood vessel formation resulting in a microvascular network characterized by vessel immaturity, with profound structural and functional abnormalities that alter a microenvironment to fuel or disease progression, such as cancer, and that attenuate response to conventional therapies. Vascular normalization (*e.g.,* in tumor vessels) can be detected by histological staining. For example, colocalization of staining for α-smooth muscle actin (α-SMA) for pericyte and vascular endothelium for CD31 can be used to obtain a pericyte coating index (*see, e.g.,* the Examples and FIGURE 9). In some embodiments, normalization is defined as at least a 20% increase in pericyte coating index compared to the pericyte coating index prior to treatment; or defined as an increase in tumor perfusion indirectly measured by at least a 20% increase in CD31-positive and lectin-positive structure per microscope field compared to that taken or observed prior to treatment. In some embodiments, normalization is defined as at least a 10% (*e.g.,* at least a 15%, or at least a 20%) increase in pericyte coating index compared to the pericyte coating index prior to treatment. Blood vessels with higher perfusion have greater CD31 and lectin co-staining. In some embodiments, normalization is defined as at least a 20% increase in microvessel density (MVD) compared to that taken or observed prior to treatment. MVD can be measured by several methods. *See* Goddard et al., Angiogenesis 2002; 5(1-2): 15-20, incorporated herein by reference in its entirety. In some embodiments, normalization can be measured by vascular morphology, and permeability (by MRI imaging using a contrast agent such as gadolinium compounds, ultrasound, PET, and CT scanning). *See,* Li et al., Cancer Management and Research 2018:10 4163-4172, incorporated herein by reference in its entirety. In some embodiments, a transient decrease in plasma sVEGFR1 is used as a candidate biomarker for predicting normalization of tumor blood vessels. In certain embodiments, plasma Angiopoietin-1/Angiopoietin-1 ratio is correlated with the degree of vascular normalization. Apelin expression both in tumor tissue and in plasma can be transiently decreased during the vessel normalization. Vascular normalization can be manifested as an increase or decrease (as applicable) in one more of the above parameters following treatment with a composition of the present disclosure.

As used herein, the term "abnormal vasculature" refers to a vasculature in any tissue or organ characterized by a random blood vessel pattern where a number of arterioles anastomose directly to the venules, bypassing the capillary bed causing local hypoxia. This is the result of uncontrolled new vessel formation, resulting in a microvascular network characterized by vessel immaturity, with profound structural and functional abnormalities that alter a microenvironment to fuel disease progression, such as cancer, and that attenuate response to conventional therapies.

As used herein, the term "cytotoxic T cells" refers to an T lymphocyte (a type of white blood cell) that has direct cytotoxic effect. The cytotoxic T cells kill cancer cells, cells that are infected (particularly with viruses), or cells that are damaged in other ways. Cytotoxic T cell is also known as Tc, cytotoxic T lymphocyte, CTL, T-killer cell, cytolytic T cell, CD8+ T-cell or killer T cell. CD8(+) T-cells are adept at killing virally infected cells and cancer cells, releasing cytokines (*e.g.,* IFN-γ) to aid this response. The term "low number of cytotoxic T cells" refers to less than 2% percent of total CD8 positive cells that are also positive for CD3 and/or also positive for CD3 and INFg, especially in the tumor tissue, *see, e.g.,* FIGURES 3, 4 and 5. Similarly, the term "increase in the number of cytotoxic T cells" refers to least a 10% increase in the percent of CD8 positive cells that are also positive for CD3 and/or also positive for CD3 and INFg, compared to the number prior to the treatment of the same cancer patient. *See, e.g.,* FIGURES 3, 4 and 5.

As used herein, the term "TIM-3" refers to "glycoprotein T cell Ig and mucin domain-containing protein 3" that is upregulated in tumor and virus-infected cells that can interfere with T-cell (including CAR T-cell) response. This upregulation progressively impairs cytotoxic T cell response against cancer and chronic viral infections, such as with HIV, resulting in T cell exhaustion. Reducing TIM-3 aids in restoring cytotoxic function of CD8(+) T-cells. *See, e.g.,* J Immunol. 2014 Jan 15;192(2):782-91.

As used herein, the term "natural killer cells" or "NK cells" refers to a type of cytotoxic lymphocyte critical to the innate immune system similar cytotoxic T cells, but unlike cytotoxic T cells, does not need MHC and antibodies. Thus, NK cells can respond rapidly (~3 days) to stressed cells, tumor formation, and/or virus-infected cells. Additionally, the term "low number of activated NK cells" in the context of cancer refers to less than 1.2% percent of total NK1.1 (or CD16 or CD57 for human) being also positive for Perforin, especially in the tumor tissue. *See, e.g.,* FIGURES 3, 4 and 5 and their corresponding Examples, below. The term "increase the number of activated NK cells" refers to least a 20% increase in the percent of total NK1.1 (or CD16 or CD57 for human) being also positive for perforin compared to the level prior to the treatment of the same cancer patient.

As used herein, the term "Treg cells" or "Tregs" refers to regulatory T cells or immune suppressor T cells that maintain tolerance to self-antigens and decrease the likelihood of autoimmune disease. Tregs are immunosuppressive and generally suppress or downregulate induction and proliferation of effector T cells such cytotoxic T cells. Tregs express the biomarkers CD4, FOXP3, and CD25. Recent research has found that the cytokine TGFβ is essential for Tregs to differentiate from naive CD4+ cells and is important in maintaining Treg homeostasis. The term "high number of Treg cells" refers to greater than 2% of total CD4 positive cells being also positive for CD25 and Fox3, especially in the tumor tissue. The term "reducing the number of Treg cells" refers to at least 20% reduction in the percent of total CD4 positive cells being also positive for CD25 and Fox3 compared to the level prior to the treatment of the same cancer patient.

As used herein, the term "TGFb," "TGFβ," or "Transforming growth factor beta" refers to a multifunctional cytokine belonging to the transforming growth factor superfamily that includes three different mammalian isoforms (TGFβ 1 to 3, HGNC symbols TGFB1, TGFB2, TGFB3) and many other signaling proteins. TGFβ is expressed by all white blood cell lineages. An increase in the expression of TGFβ is correlated with malignancy in many cancers. Additionally, the term "high level of TGFβ expression" refers to at least 1.2 times the expression relative to the level observed in normal tissue. A high level of TGFβ expression indicates high level of Treg cells. The term "decreasing TGFβ expression" refers to decreasing expression by at least 20% relative to the expression prior to the treatment of the same cancer patient.

As used herein, the term "Foxp3" or "forkhead box P3" is a protein involved in immune system responses and acts as a master regulator in the development and function of regulatory T cells that generally turn the immune response down. In cancer, an excess of regulatory T cell activity can inhibit the immune system from destroying cancer cells. In autoimmune disease, a deficiency of regulatory T cell activity can allow other autoimmune cells to attack the body's own tissues. The term "high level of Foxp3 expression" refers to at least 1.2 times the expression relative to the level observed in normal non-cancerous tissue. A high level of Foxp3 expression indicates high level of Treg cells. The term "decreasing Foxp3 expression" refers to decreasing expression by at least 20% relative to the expression prior to the treatment of the same cancer patient.

As used herein, the term "myeloid-derived suppressor cells" or "MDSCs" refers to a heterogenous group of immune cells from the myeloid lineage that strongly expand in pathological situations such as chronic infections and cancer. Cancer tissues with high infiltration of MDSCs are associated with poor patient prognosis and resistance to therapies. MDSCs possess strong immunosuppressive activities rather than immunostimulatory properties and regulate functions of T cells, dendritic cells, macrophages and natural killer cells. MDSCs can also be detected in the blood and the level can be up to 10-fold higher than normal in cancer. As used herein, the term "high number of myeloid-derived suppressor cells" or "high number of MDSCs" refers to at least 1.5 times the number of MDSCs relative to the level observed in normal healthy tissue. The average MDSC from 67 healthy adult blood is about 50 +/- 30 MDSC cells per microliter of blood for ages 20-93 years and for both sexes. *See,* Apodaca et al., Journal for Immunotherapy of Cancer (2019) 7:230. The term "decreasing number of myeloid-derived suppressor cells" refers to at least a 20% (preferably 50%) reduction in the number of myeloid-derived suppressor cells relative to the number prior to the treatment of the same cancer patient.

As used herein, the term "Bv8" refers to a protein also known as prokineticin that promotes both tissue-specific angiogenesis and hematopoietic cell mobilization. Bv8 modulates mobilization of MDSCs from the bone marrow during tumor development and promotes angiogenesis locally. Bv8 is a surrogate marker for MDSCs. The term "high level of Bv8 expression" refers to at least 1.5 times the level of Bv8 expression relative to the level observed in normal healthy tissue refers to a higher level of Bv8 expression relative to a normal individual without cancer. Similarly, the term "decreased level of Bv8 expression" refers to a decrease level of Bv8 expression relative to the level observed prior to the treatment of the same cancer patient.

As used herein, the term "cytotoxic cell immunostimulant(s)" refers to immunostimulatory(s) that decreases the number of "Treg cells" and/or "MDSCs" and increases the number of "cytotoxic T cell" and/or the "NK cells." Examples of cytotoxic cell immunostimulants include immune checkpoint inhibitors. Immune checkpoint inhibitors bind directly to immune checkpoint proteins allowing the immune system to be activated by decreasing the number of "Treg cells" and /or "MDSCs" and increasing the number of "cytotoxic T cell" and/or the "NK cells" that destroy or cause damage to cancer cells or tissues. As used herein, the term "immunostimulants" or "immunostimulators" refers to substances (drugs and nutrients) that stimulate or facilitate activation of any component of the immune system.

At various places in the present specification, substituents of compounds of the disclosure are disclosed in groups or in ranges. It is specifically intended that the disclosure include each and every individual subcombination of the members of such groups and ranges. For example, the term "C₁₋₆ alkyl" is specifically intended to individually disclose methyl, ethyl, C₃ alkyl, C₄ alkyl, C₅ alkyl, and C₆ alkyl.

One letter codes for amino acids are used herein. For example, alanine is A, arginine is R, asparagine is N, aspartic acid is D, cysteine is C, glutamic acid is E, glutamine is Q, glycine is G, histidine is H, isoleucine is I, leucine is L, lysine is K, methionine is M, phenylalanine is F, proline is P, serine is S, threonine is T, tryptophan is W, tyrosine is Y, valine is V, and γE is glutamic acid where the R-group (*i.e.,* side chain) carboxyl (gamma, γ) is the moiety used to link to any of the primary amino group of a peptide or to the N-terminal portion of a peptide rather than the alpha-carboxyl. For the purpose of the present application, the one letter codes for amino acids includes L and/or D amino acid stereoisomers. It is understood that when the amino acids combine to form a peptide, the amino acids are referred to as amino acid residues where the elements of water are removed. Furthermore, where the present disclosure refers to an amino acid in a peptide sequence, it is understood to be an amino acid residue.

As used herein, the term "aliphatic" refers to a compound or group containing carbon and hydrogen joined together in straight chains, branched chains, or non-aromatic rings. Aliphatic compounds or groups may be saturated (*e.g.,* an alkane such as hexane and other alkanes, an alkyl such as hexyl and other alkyls) or unsaturated (*e.g.,* hexene and other alkenes, as well as alkynes, hexenyl and other alkenyl, as well as alkynyl). The aliphatic compound or group (*e.g.,* an alkyl, alkenyl, or alkynyl) can be substituted, for example, with 1, 2, 3, 4, 5, 6, 7, or 8 substituents such as (=O), hydroxyl, carboxyl, carbonyl, and/or an ester group. For example, the aliphatic group can have a carboxyl group as a substituent as a pendant group and/or at a terminus. When the aliphatic group is part of a compound, it is understood that the aliphatic group can be covalently bound to the compound via a chemical linkage, such as a carbonyl (C=O, also represented by C(O) or C(=O)) (*e.g.,* as part of an amide or an ester linkage), a thioether, an ether, a thioether, a carbamate moiety, a bond, or the like. It is understood that the number of carbons in the aliphatic chain includes the backbone carbons in the chemical linkage. For example, a saturated C8 aliphatic chain that includes a C(=O) linkage, when linear, can be represented by CH₃(CH₂)₆C(=O). As another example, a saturated C8 aliphatic chain that has a carboxyl group at a first terminus and that includes a C(=O) linkage at a second terminus, when linear, can be represented by HOC(=O)(CH₂)₆C(=O). For example, a saturated C18 aliphatic chain that includes a C(=O) linkage, when linear, can be represented by CH₃(CH₂)₁₆C(=O). As another example, a saturated C18 aliphatic chain that has a carboxyl group at a first terminus and that includes a C(=O) linkage at a second terminus, when linear, can be represented by HOC(=O)(CH₂)₁₆C(=O). The aliphatic group can be derived from a fatty acid and/or the aliphatic group can be derived from a diacid.

As used herein, the term "alkyl" refers to a saturated hydrocarbon group which is straight-chained (*e.g.,* linear) or branched. Example alkyl groups include methyl (Me), ethyl (Et), propyl (*e.g.,* n-propyl and isopropyl), butyl (*e.g.,* n-butyl, isobutyl, t-butyl), pentyl (*e.g.,* n-pentyl, isopentyl, neopentyl), and the like. An alkyl group can contain from 1 to about 30, from 1 to about 24, from 2 to about 24, from 1 to about 20, from 2 to about 20, from 1 to about 10, from 1 to about 8, from 1 to about 6, from 1 to about 4, or from 1 to about 3 carbon atoms.

As used herein, the term "fatty acid" refers to an aliphatic chain that is substituted with a carboxyl group, which is either saturated or unsaturated. Examples of fatty acids includes caprylic acid, capric acid, lauric acid, myristic acid, palmitic acid, stearic acid, arachidic acid, behemic acid, and/or lignoceric acid.

As used herein, the term "fatty acid ester" refers to a long aliphatic chain (saturated or unsaturated) having a -C(=O)O- moiety at an end of the chain.

As used herein, the term "fatty acid amide" refers to a long aliphatic chain (saturated or unsaturated) having a -C(=O)NR- moiety at an end of the chain.

As used herein, the term "individual," "subject," or "patient," used interchangeably, refers to any animal, including mammals, preferably mice, rats, other rodents, rabbits, dogs, cats, swine, cattle, sheep, horses, or primates, and most preferably humans.

As used herein, the phrase "therapeutically effective amount" refers to an amount of a therapeutic agent (i.e., drug, or therapeutic agent composition) that elicits the biological or medicinal response that is being sought in a tissue, system, animal, individual or human by a researcher, veterinarian, medical doctor or other clinician, which includes one or more of the following:
(1) preventing the disease; for example, preventing a disease, condition or disorder in an individual who may be predisposed to the disease, condition or disorder but does not yet experience or display the pathology or symptomatology of the disease;
(2) inhibiting the disease; for example, inhibiting a disease, condition or disorder in an individual who is experiencing or displaying the pathology or symptomatology of the disease, condition or disorder; and
(3) ameliorating the disease; for example, ameliorating a disease, condition or disorder in an individual who is experiencing or displaying the pathology or symptomatology of the disease, condition or disorder (*i.e.,* reversing the pathology and/or symptomatology) such as decreasing the severity of disease, prolonging survival time, and/or preventing death.

As used herein, a "bolus dose" is a single dose of a drug or other substance given or administered over a short period of time, for example, less than 10 minutes (*e.g.,* less than 8 minutes, less than 5 minutes, less than 3 minutes, or less than 1 minute). In some embodiments, a bolus dose is administered in less than 5 minutes. In some embodiments, a bolus dose is administered in less than 3 minutes. In some embodiments, a bolus dose is administered in less than 1 minute. Administration includes one of: injection in any part of the body (including but not limited to blood vessels, subcutaneous, intrathecal, or intradermal), enterally (*e.g.,* orally, as a dosage form), inhalation (*e.g.,* by intratracheal inhalation administration, where a subject is exposed to high aerosol concentrations such that the active pharmaceutical ingredient is deposited directly in the lower respiratory tract), or nasally (*e.g.,* as an aerosol, liquid, or powder).

As used herein, the terms "a blood pressure drop," "a drop in blood pressure," or "hypotension" are used interchangeably, and refer to a statistically significant decrease in blood pressure in a subject below a baseline blood pressure. The baseline blood pressure is the average mean blood pressure measured prior to treatment or administration of any drug to a subject, or the mean blood pressure of a normal healthy subject. The standard deviation of most blood pressure measuring device can be between 5-15% depending on the method of measurement and position, state of mind, or movement of the subject during measurement. For the clarity of the present specification the change in blood pressure will be expressed as statistically significant percent increase, decrease, or drop in blood pressure from the mean/average baseline blood pressure prior to drug or test article administration. Statistically significant means that P<0.05 as known to those skilled in the art of statistics.

As used herein, the term "C-type natriuretic peptide" or "CNP" is a peptide including 22 amino acid residues, having a 17 amino acid residue ring structure formed by a disulfide bond, and an additional 5-amino acid residue extension at the N-terminal (GLSKGCFGLKLDRIGSMSGLGC [SEQ ID NO. 10]; where the letters are in accordance with conventional amino acid nomenclature, and the amino acid residues C-6 (at position 6) and C-22 (at position 22) are linked by a disulfide bond). *See*, *e.g.,* Sudoh et al., Biochem. Biophys. Res. Commun. 1989; 159:1427-1434.

As used herein, a "NPRB receptor," "natriuretic peptide receptor B (NPRB)," or "NPR2," "guanylate cyclase B (GC-B)," or "B-type natriuretic peptide receptor 2" (NPR2) are used interchangeably. In humans, a NPRB receptor is encoded by NPR2 gene, which is located on chromosome 9 and in mouse on chromosome 4. *See, e.g.,* Nuglozeh et al., Genome. 1997; 8:624-625. The expression of NPRB is reported in various organs such as heart, brain, uterus, ovary, kidney, lungs, liver and adipocytes and in some cancers. Schulz et al., Cell. 1989; 58:1155- 1162; Nagase et al., J. Hypertens. 1997; 15:1235-1243; Chrisman, et al., J. Biol. Chem. 1993; 268:3698-3703. NPRB is selectively activated by CNP and not by ANP or BNP (the other known natriuretic peptides). The ubiquitous expression of NPRB signifies its role in many physiological functions. While the other natriuretic peptide receptor, NPRA, is activated by physiological concentrations of ANP and BNP, NPRA is not activated by CNP.

As used herein, the term "long-acting C-type natriuretic peptide" or "long acting CNP" refers to a CNP formulation that when administered as a single bolus dose to a mammalian subject (human, non-human, primate, dogs, rats, mice, etc.), the resulting elevation of CNP level in the plasma or elevation of plasma cyclic-GMP level above the baseline will be sustained for a duration of greater than 4 hours or greater than 6 hours depending on the species. A long-acting C-type natriuretic peptide or a long acting CNP encompasses a very long-acting C-type natriuretic peptide or a very long acting CNP. The elevation of plasma cyclic-GMP is a result of CNP structure activity itself, or from the combination of the CNP with one or more components of a formulation containing the CNP. The presence in the plasma (or elevation) means a detectable presence over and above the analytical baseline level, wherein the baseline level is the level measured in the absence of long-acting CNP formulation administration. The length of sustained plasma cyclic-GMP elevation is the duration of biological activity of the CNP formulation. A CNP formulation refers to a composition containing a CNP peptide with one or more excipient or carrier such as a polymer, protein, sugar, detergent, and/or buffer. The CNP in the CNP formulation may or may not be covalently linked to excipient or carrier. The sustained presence in the blood can be evaluated by pharmacokinetic/pharmacodynamic analysis after administration.

As used herein, a formulation containing a "very long-acting C-type natriuretic peptide" or "very long acting CNP" refers to a long-acting CNP formulation containing the 22 amino acid residue CNP formulated in such a way that when administered as a single bolus dose to subject, will have sustained presence in the plasma or sustained plasma cyclic-GMP elevation over the baseline of 24 hours or greater (*e.g.,* up to 2-3 days or up to 1-4 weeks). Thus, a very long-acting C-type natriuretic peptide or very long acting CNP is a subset of a long-acting C-type natriuretic peptide or long acting CNP. The presence in the plasma means a detectable presence over and above the endogenous native agonist that are normally made by the subject or an analytical baseline level in the absence of administration of a therapeutic CNP formulation. The duration (*i.e.,* length of time) of plasma cyclic-GMP elevation or the presence of detectable CNP over the baseline can be from 24 to 192 hours, or 24 to 48 hours, or 48 to 72 hours, or 72 to 96 hours, or 96-120 hours, or 120 to 144 hours, 144 to 168 hours, or 168 to 192 hours. As described above, a CNP formulation is a composition containing CNP peptide with one or more excipient or carrier such as polymer, protein, sugar, detergent, and/or buffer. The CNP in CNP formulation may or may not be covalently linked to excipient or carrier. The sustained presence in the blood can be evaluated by pharmacokinetic/pharmacodynamic analysis after administration.

As used herein, the term "long acting CNP derivative" is a CNP derivative that when administered as a single bolus dose to a mammalian subject or patient has sustained presence in the plasma or sustained plasma cyclic-GMP elevation over the baseline of greater than 4 hours, or greater than 6 hours, depending on the species. A long acting CNP derivative encompasses a very long-acting CNP derivative. The long-acting nature can result from the CNP derivative structure itself, or from the combination of the CNP derivative with one or more components of a formulation containing the CNP derivative. The presence in the plasma or blood refers to a detectable presence over the endogenous native agonist that are normally made by the mammals or above an analytical baseline level in the absence of administration of a therapeutic compound, peptide, protein or formulation. The sustained presence in the blood can be evaluated by pharmacokinetic/ pharmacodynamic analysis after administration. In some embodiments, the CNP derivative is a modified CNP with at least 72% (*e.g.,* at least 80%, at least 85%, at least 90%, at least 95%, or at least 98%) sequence homology or identity to native CNP. In some embodiments, the CNP derivative is an addition derivative where a native CNP is modified by covalent addition of a chemical moiety, such as one or more additional amino acids and/or fatty acids and/or any chemical moiety and/or moieties at the N-terminal, C-terminal, or in the R-group of any amino acid residue in the CNP peptide. In some embodiments, the CNP derivative includes a substitution derivative where 1 to 6 amino acid residues (or 5 to 28% of the amino acid residues) in native CNP is replaced by different or unnatural amino acid residues. In certain embodiments, the CNP derivative includes a subtraction derivative where 1 to 6 amino acid residues (or 5 to 28% of the amino acid residues) in a native CNP are deleted. In certain embodiments, the CNP derivative includes a subtraction derivative where 1 to 6 amino acid residues (or 5 to 28% of the amino acid residues) in a native CNP are deleted and/or substituted. A CNP derivative formulation refers to a composition containing a CNP derivative with one or more excipient or carrier such as polymer, protein, sugar, detergent, or buffer.

As used herein, the term "very long acting CNP derivative" refers to a long acting CNP derivative or CNP derivative, that when administered as a single bolus dose to mammalian subject or patient, has sustained presence in the plasma or sustained plasma cyclic-GMP elevation over the baseline that has a duration of 24 hours or greater. Thus, a very long acting CNP derivative is a subset of long acting CNP derivative. The very long acting CNP derivative can result from the CNP derivative structure itself, or from the combination of the CNP derivative with one or more components of a formulation containing the CNP derivative. The presence in the plasma refers to a detectable presence over an analytical baseline plasma level in the absence of administration of the very long acting CNP derivative. The duration of plasma cyclic-GMP elevation or the presence of detectable CNP derivative over the baseline can be from 24 to 192 hours, or 24 to 48 hours, or 48 to 72 hours, or 72 to 96 hours, or 96-120 hours, or 120 to 144 hours, 144 to 168 hours, or 168 to 192 hours. The sustained presence in the blood can be evaluated by pharmacokinetic/pharmacodynamic analysis after administration. In some embodiments, the CNP derivative includes a modified CNP with 72% (*e.g.,* at least 80%, at least 85%, at least 90%, at least 95%, or at least 98%) sequence identity to native CNP. In some embodiments, the CNP derivative is an addition derivative where native CNP is modified by covalent addition of chemical moiety such as additional amino acids and/or fatty acid and/or any chemical moiety and/or moieties at N-terminal, C-terminal, or in the R-group of any amino acid residue in the CNP peptide. In some embodiments, the CNP derivative is a substitution derivative where 1-6 amino acid residues (or 5-28% of the amino acid residues) in native CNP is replaced by different or unnatural amino acid residues. In certain embodiments, the CNP derivative is a subtraction derivative where 1-6 amino acid residues (or 5-28% of the amino acid residues) in native CNP were deleted. In certain embodiments, the CNP derivative includes a subtraction derivative where 1 to 6 amino acid residues (or 5 to 28% of the amino acid residues) in a native CNP are deleted and/or substituted. CNP derivative formulation is a composition containing CNP derivative with one or more excipient or carrier such as polymer, protein, sugar, detergent, or buffer

As used herein, a "formulation of a CNP" or a "formulation of a CNP derivative" refers to a composition containing CNP peptide or its derivative that may or may not be covalently linked to an excipient or carrier such as polymer, protein, and/or lipid.

As used herein, the term "NPRB agonist" or "NPR2 agonist" refers to any compound, peptide or protein that does not contain the 22 amino acid residue CNP sequence in its structure and that can bind to NPRB, a cell catalytic receptor, and stimulate its intracellular guanylyl cyclase activity to increase intracellular or blood cyclic-GMP level, but with limited or no capability to bind and stimulate NPRA receptor. Since not all cells express similar levels of NPRB, the NPRB agonist is tailored to primarily affect those cells expressing NPRB. This selectivity can be readily measured by those skilled in the art by measuring the activity in cells that expresses NPRB, compared to activity in cells that expresses NPRA.

As used herein, the term "long acting NPRB agonist" refers to an NPRB agonist defined above, that, when administered as a single bolus dose to a mammalian subject or patient has sustained presence in the plasma or sustained plasma cyclic-GMP elevation over the baseline of greater than 4 hours or greater than 6 hours depending on the species. A long acting NPRB agonist encompasses a very long acting NPRB agonist. The long acting nature of the NPRB agonist can result from the NPRB agonist structure itself, or from the combination of the NPRB agonist with one or more components of a formulation containing the NPRB agonist. The presence in the plasma means a detectable presence over an analytical baseline level in the absence of administration of a long acting NPRB agonist. A formulation of a long acting NPRB agonist or a long acting NPRB agonist formulation is a composition containing a long acting NPRB agonist, or a long acting NPRB agonist with one or more an excipient or carrier such as a polymer, protein, sugar, lipid, or buffer. The long acting NPRB agonist may or may not be covalently linked to excipient or carrier. The sustained presence in the blood can be evaluated by pharmacokinetic/pharmacodynamic analysis after administration. The sustained plasma elevation of cyclic-GMP above the baseline can be evaluated by pharmacodynamic analysis after administration.

As used here, the term "very long acting NPRB agonist" refers to a long NPRB agonist that, when administered as a single bolus dose to a mammalian subject or patient, will have sustained presence in the plasma or sustained plasma cyclic-GMP elevation over the baseline of 24 hours or greater. A very long acting NPRB agonist is a subset of a long acting NPRB agonist. The very long acting nature of the NPRB agonist can result from the NPRB agonist structure itself, or from the combination of the NPRB agonist with one or more components of a formulation containing the NPRB agonist. The presence in the plasma means its detectable presence over an analytical baseline level in the absence of administration of a very long acting NPRB agonist. The duration of plasma cyclic-GMP elevation or the presence of detectable NPRB agonist over the baseline can be from 24 to 192 hours, 24 to 48 hours, or 48 to 72 hours, or 72 to 96 hours, or 96-120 hours, or 120 to 144 hours, 144 to 168 hours, or 168 to 192 hours. A formulation of a very long acting NPRB agonist or a very long acting NPRB agonist formulation refers to a composition containing a very long acting NPRB agonist, or a very long acting NPRB agonist with one or more an excipient or carrier such as a polymer, protein, sugar, lipid, or buffer. The very long acting NPRB agonist may or may not be covalently linked to excipient or carrier. The sustained presence in the blood can be evaluated by pharmacokinetic/ pharmacodynamic analysis after administration. The sustained presence in the blood can be evaluated by pharmacokinetic/pharmacodynamic analysis after administration. The sustained plasma elevation of cyclic-GMP above the baseline can be evaluated by pharmacodynamic analysis after administration.

As used herein, the phrase "NPRB agonist with limited or no agonistic activity against NPRA" refers to an NPRB agonist that has greater than 5-fold binding affinity (or lower EC50) for NPRB than NPRA.

As used herein, the term "polymer" refers to a macromolecule formed chiefly or entirely of many similar repeating units covalently bonded together. The term polymer include cellulose derivatives, poly(ethylene glycol) (PEG), methoxy poly(ethylene glycol) (MPEG), poly(lactic-co-glycolic acid), and poly(N-vinyl pyrrolidone) and derivatives thereof. These polymers can be branched or linear. As used herein, a polymer can be attached to peptides, protein or a linker group by amide, ester, ether, thioether, thioester, or carbamate bond or by linkers containing one of those bonds. Polymer can also be grafted with each other for make a protected graft co-polymer excipient that, when mixed with an active pharmaceutical ingredient, can enhance pharmacokinetic and pharmacodynamics performance of active pharmaceutical ingredient by extending its presence in the blood or plasma after administration *in vivo.*

The term "amino acids" as used herein are organic compounds with molecular weight of less than 500Da that contain amino (-NH₂) and carboxyl (-COOH) functional groups, along with a side chain (R group) specific to each amino acid. The key elements of an amino acid are carbon (C), hydrogen (H), oxygen (O), and nitrogen (N), although other elements are found in the side chains of certain amino acids. About 500 naturally occurring amino acids are known as of 1983 (though only 20 appear in the mammalian genetic code, these 20 amino acids are also referred to herein as "natural amino acids)). Amino acids can be alpha amino acids, where the amino group is bonded directly to the alpha carbon. Amino acids can be non-alpha amino acid, where the primary amino group is linked to a carbon other than the alpha position. The alpha carbon is the carbon directly adjacent to the carboxyl group.

The term "derivative" or "analog" as used herein includes compounds whose core structures are the same as, or closely resemble that of, a parent compound, but which have a chemical or physical modification, such as different or additional groups; the term includes co-polymers of parent compounds that can be linked to other atoms or molecules. The term also includes a peptide or protein with at least 72% (*e.g.,* at least 80%, at least 85%, at least 90%, at least 95%, or at least 98%) sequence identity with the parent peptide or protein. The term also includes a peptide with additional groups attached to it, such as additional label or tag, compared to the parent peptide. The term also includes a polymer with additional group attached to it, such as alkoxy or methoxy group, compared to the parent polymer.

As used herein, an "addition derivative" or "expansion derivative" refers to a peptide derivative where the main backbone amino acid sequence for a peptide remains the same, but the addition of extra functional groups and/or amino acid to the main amino acid sequence using one or more reactive moieties in the main amino acid sequence provides the addition derivative or the expansion derivative. The addition derivative or expansion derivative is different from a truncation and/or substitution peptide derivative where one or more amino acid residues in the main backbone amino acid residue sequence of the peptide have been removed and/or replaced by different functional groups and/or amino acid residues, respectively.

As used herein, the term "linker group" or "linking group" or "linker" refers to atoms or chemical moieties that covalently link or bond two entities (*e.g.,* portions of two molecules) together. For example, a linker precursor such as an amino acid, a peptide, or non-amino acid molecule derived from commercially available crosslinkers can be reacted with two entities, linking the two entities together via the linker group. Once the two moieties are linked together, the linker group is the portion that remains from the linker precursor in the final linked entities. For example, if molecule A is to be linked to molecule B, a linker group can have two chemical functional groups where one functional group will react with A and the other functional group will react with B resulting in "A-linker group-B". In this case, the linker group is the portion of the linker precursor that remains after the covalent linking of A and B.

As used herein, the term "polypeptide" refers to a polymer of amino acids.

As used herein, the term "peptide" refers to a polypeptide with three or more amino acids covalently linked together by amide bonds through alpha amino and alpha carboxyl. The number of amino acids in a peptide can be 3 to about 100 units.

As used herein, the term "protein" refers to a polypeptide large enough to have a 3-dimensional structure, such as a β-barrel, or an α-helix.

As used herein, the term "antibody" refers to a protein produced by the immune cells that recognize a specific antigen. It is a protein produced in response to and counteracting a specific antigen in the blood. Antibodies combine chemically with substances which the body recognizes as alien, such as bacteria, viruses, and foreign substances in the blood

As used herein, the term "humanized antibody" refers to an antibody from non-human species whose protein sequences have been modified to increase their similarity to antibody variants produced naturally in humans.

As used herein, the term "subcutaneous administration," "s.c.", "s.c. administration," "SC," or "SC administration" refers to a delivery of drug, usually in liquid form, directly into the fatty tissues just beneath the skin. The delivery is usually carried out by direct injection. These injections are shallower than those injected into muscle tissues. Providers often use subcutaneous injections for medications that are suitable for absorption into the bloodstream slowly and steadily,

As used herein, the term "intravenous administration," "IV administration," or "IV injection" refers to a delivery of drug, typically in liquid form, directly into a vein of an animal or human. The delivery methods are usually by direct injection. The intravenous route of administration can be used both for injections, using a syringe at higher pressures; as well as for infusions, for example, using the pressure supplied by gravity.

As used herein, the term "intramuscular administration," "IM administration," or "IM injection" refers to an intramuscular delivery of drug, usually in liquid form, directly into the muscles of an animal or human. The delivery is usually by direct injection. This allows the medication to be absorbed into the bloodstream quickly. In some instances, a person may also self-administer an IM injection. In some embodiments, IM injections can be used instead of intravenous injections, for example, when certain therapeutic agents are irritating to veins, or when a suitable vein cannot be located.

As used herein, the term "nasal administration" refers to a delivery of a therapeutic agent (*e.g.,* in form of gel, liquid, aerosol, gas, or powder) by topical application, dropping as a liquid, insufflation (or blown or sprayed), into the nose of an animal or a human. This form of administration can be used, depending on the formulation, for example, to deliver a therapeutic agent to the nasal cavity or the lungs (depending on the device used), and/or may not be absorbed systemically (purely local administration), and/or may be totally absorbed systemically (purely systemic), and/or more frequently partially absorbed (both local and systemic). Nasal sprays can include locally acting drugs such as decongestants for cold and allergy treatment, whose systemic effects are typically minimal. Examples of systemically active drugs available as nasal sprays include, for example, migraine drugs, nicotine replacement, and hormone treatments.

As used herein, the term "parenteral" or "non-gastrointestinal" administration refers to a route of administration that is not through enteral or gastrointestinal routes. Examples of parenteral administration include subcutaneous (under the skin), intravenous (into a vein), intra-arterial (into an artery), intramuscular (into a muscle), intraperitoneal (infusion or injection into the peritoneum), inhalation (*e.g.,* by intratracheal inhalation administration, where a subject is exposed to high aerosol concentrations of the active pharmaceutical ingredient such that the active pharmaceutical ingredient is deposited directly in the lower respiratory tract), nasal administration (through the nose), sublingual and buccal medication, intrathecal (into the spinal canal), intracerebral (into the cerebrum), intracerebroventricular (into the cerebral ventricles), intradermal (into the skin itself), or any other administration routes not involving the gastrointestinal tract. As used herein, the term "enteral" means administration to any region of the alimentary tract and including mouth (oral), pharynx (throat), esophagus, stomach, small intestine, large intestine, rectum, and anus or through an artificial opening in any of these regions.

As used herein, the term "therapeutic agent," "drug," or "active pharmaceutical ingredient" refers to a substance or a molecule capable of producing a curative effect in a disease state.

As used herein, the term "excipient" refers to a substance that is formulated together with or mixed with an active pharmaceutical ingredient for the purpose of long-term stabilization, to bulk up formulations that contain potent active ingredients in small amounts (thus often referred to as "bulking agents", "fillers", or "diluents"), and/or to confer a therapeutic enhancement on the active pharmaceutical ingredient in the final dosage form, such as to facilitate drug absorption and/or potency/dose, reduce viscosity, enhance solubility, and/or prolong the action or presence of the active pharmaceutical ingredient in the blood. The selection of appropriate excipients depends upon the route of administration and the dosage form, the active pharmaceutical ingredient, and other factors. The excipient can include, for example, sugar, amino acid, buffer, antioxidant, chelating agent, solvent or vehicle, and/or a complex polymer that binds and stabilizes an active pharmaceutical ingredient *in vitro* and/or *in vivo.* Though excipients were at one time assumed to be "inactive" ingredients, it is now understood that they can sometimes be "a key determinant of dosage form performance." In other words, the effects of an excipient on pharmacodynamics and pharmacokinetics can be important and can require extensive research and study. How an excipient influences delivery of an active pharmaceutical ingredient is often unpredictable.

As used herein, the term "healthy subject" refers to an individual (human and/or mammalian animals) with no indications of or other known significant health problems as evaluated by those skilled in the art (physician and/or clinician). For the purpose of the present disclosure these are individuals without tumor cancer as evaluated by those skilled in the art (physician and/or clinician). As an example, A healthy human subject has the following characteristic laboratory values for hematology test results: absolute neutrophil count (male): 1780-5380/µL (1.78-5.38 x 10⁹/L), (female): 1560-6130/µL (1.56-6.13 x 10⁹/L); activated partial thromboplastin time: 25-35 s; bleeding time: less than 10 min; erythrocyte count : 4.2-5.9 x 10⁶/µL (4.2-5.9 x 10¹²/L); erythrocyte sedimentation rate (male) 0-15 mm/h, (female): 0-20 mm/h; erythropoietin: less than 30 mU/mL (30 units/L); D-dimer: less than 0.5 µg/mL (0.5 mg/L); ferritin, serum: 15-200 ng/mL (15-200 µg/L); haptoglobin, serum: 50-150 mg/dL (500-1500 mg/L); hematocrit (male): 41%-51%, (female): 36%-47%; hemoglobin, blood (male): 14-17 g/dL (140-170 g/L), (female): 12-16 g/dL (120-160 g/L); leukocyte alkaline phosphatase: 15-40 mg of phosphorus liberated/h per 10¹⁰ cells, score = 13-130/100 polymorphonuclear neutrophils and band forms; leukocyte count: 4000-10,000/µL (4.0-10 x 10⁹/L); mean corpuscular hemoglobin: 28-32 pg; mean corpuscular hemoglobin concentration: 32-36 g/dL (320-360 g/L); mean corpuscular volume: 80-100 fL; platelet count: 150,000-350,000/µL (150-350 x 10⁹/L); prothrombin time: 11-13 s; reticulocyte count: 0.5%-1.5% of erythrocytes; absolute: 23,000-90,000/µL (23-90 x 10⁹/L)

A healthy human subject has the following characteristic laboratory values for blood, plasma, and serum chemistry results, albumin, serum: 3.5-5.5 g/dL (35-55 g/L); alkaline phosphatase, serum: 36-92 units/L; α-fetoprotein, serum: 0-20 ng/mL (0-20 µg/L); aminotransferase, alanine (ALT): 0-35 units/L; aminotransferase, aspartate (AST): 0-35 units/L; ammonia, plasma: 40-80 µg/dL (23-47 µmol/L); amylase, serum: 0-130 units/L bicarbonate, serum: 23-28 meq/L (23-28 mmol/L); bilirubin, serum total: 0.3-1.2 mg/dL (5.1-20.5 µmol/L) direct: 0-0.3 mg/dL (0-5.1 µmol/L); blood gases, arterial (ambient air) pH: 7.38-7.44 Pco2: 35-45 mm Hg (4.7-6.0 kPa) Po2: 80-100 mm Hg (10.6-13.3 kPa); oxygen saturation: 95% or greater; blood urea nitrogen: 8-20 mg/dL (2.9-7.1 mmol/L); C-reactive protein: 0.0-0.8 mg/dL (0.0-8.0 mg/L); calcium, serum: 9-10.5 mg/dL (2.2-2.6 mmol/L); chloride, serum: 98-106 meq/L (98-106 mmol/L); cholesterol, plasma Total: 150-199 mg/dL (3.88-5.15 mmol/L), desirable low-density lipoprotein (LDL): less than or equal to 130 mg/dL (3.36 mmol/L), desirable; high-density lipoprotein (HDL): greater than or equal to 40 mg/dL (1.04 mmol/L), desirable; complement, serum C3: 55-120 mg/dL (550-1200 mg/L); total (CH50): 37-55 U/mL (37-55 kU/L); creatine kinase, serum: 30-170 units/L; creatinine, serum: 0.7-1.3 mg/dL (61.9-115 µmol/L); electrolytes, serum sodium: 136-145 meq/L (136-145 mmol/L), potassium: 3.5-5.0 meq/L (3.5-5.0 mmol/L), chloride: 98-106 meq/L (98-106 mmol/L), bicarbonate: 23-28 meq/L (23-28 mmol/L); fibrinogen, plasma: 150-350 mg/dL (1.5-3.5 g/L); folate, red cell: 160-855 ng/mL (362-1937 nmol/L); folate, serum: 2.5-20 ng/mL (5.7-45.3 nmol/L); glucose, plasma: fasting, 70-100 mg/dL (3.9-5.6 mmol/L); γ-glutamyltransferase, serum: 0-30 units/L; homocysteine, plasma male: 0.54-2.16 mg/L (4-16 µmol/L), female: 0.41-1.89 mg/L (3-14 µmol/L); immunoglobulins globulins, total: 2.5-3.5 g/dL (25-35 g/L), IgG: 640-1430 mg/dL (6.4-14.3 g/L), IgA: 70-300 mg/dL (0.7-3.0 g/L), IgM: 20-140 mg/dL (0.2-1.4 g/L), IgD: less than 8 mg/dL (80 mg/L), IgE: 0.01-0.04 mg/dL (0.1-0.4 mg/L); Iron Ferritin, serum: 15-200 ng/mL (15-200 µg/L), iron, serum: 60-160 µg/dL (11-29 µmol/L), iron-binding capacity, total, serum: 250-460 µg/dL (45-82 µmol/L), transferrin saturation: 20%-50%; lactate dehydrogenase, serum: 60-100 units/L; lactic acid, venous blood: 6-16 mg/dL (0.67-1.8 mmol/L); lipase, serum: less than 95 units/L; magnesium, serum: 1.5-2.4 mg/dL (0.62-0.99 mmol/L); methylmalonic acid, serum: 150-370 nmol/L; osmolality, plasma: 275-295 mosm/kg H2O; phosphatase, alkaline, serum: 36-92 units/L; phosphorus, serum: 3-4.5 mg/dL (0.97-1.45 mmol/L); potassium, serum: 3.5-5.0 meq/L (3.5-5.0 mmol/L); prostate-specific antigen, serum - less than 4 ng/mL (4 µg/L); protein, serum Total: 6.0-7.8 g/dL (60-78 g/L), albumin: 3.5-5.5 g/dL (35-55 g/L), globulins, total: 2.5-3.5 g/dL (25-35 g/L); rheumatoid factor: less than 40 U/mL (40 kU/L); sodium, serum: 136-145 meq/L (136-145 mmol/L); transferrin saturation: 20%-50%; triglycerides: less than 150 mg/dL (1.69 mmol/L), desirable; troponins, serum troponin I: 0-0.5 ng/mL (0-0.5 µg/L), troponin T: 0-0.10 ng/mL (0-0.10 µg/L); urea nitrogen, blood: 8-20 mg/dL (2.9-7.1 mmol/L); uric acid, serum: 2.5-8 mg/dL (0.15-0.47 mmol/L); vitamin B12, serum: 200-800 pg/mL (148-590 pmol/L).

A healthy human subject has the following characteristic laboratory values for endocrine test panels results: adrenocorticotropic hormone (ACTH), serum: 9-52 pg/mL (2-11 pmol/L); aldosterone, serum supine: 2-5 ng/dL (55-138 pmol/L) standing: 7-20 ng/dL (194-554 pmol/L); aldosterone, urine: 5-19 µg/24 h (13.9-52.6 nmol/24 h); catecholamines epinephrine, plasma (supine): less than 75 ng/L (410 pmol/L), norepinephrine, plasma (supine): 50-440 ng/L (296- 2600 pmol/L), catecholamines, 24-hour, urine: less than 100 µg/m2 per 24 h (591 nmol/m² per 24 h); cortisol, free, urine - less than 50 µg/24 h (138 nmol/24 h); dehydroepiandrosterone sulfate (DHEA), plasma male: 1.3-5.5 µg/mL (3.5-14.9 µmol/L) female: 0.6-3.3 µg/mL (1.6-8.9 µmol/L); epinephrine, plasma (supine): less than 75 ng/L (410 pmol/L); estradiol, serum male: 10-30 pg/mL (37-110 pmol/L), female: day 1-10, 14-27 pg/mL (50-100 pmol/L); day 11- 20, 14-54 pg/mL (50-200 pmol/L); day 21-30, 19-41 pg/mL (70-150 pmol/L); Follicle-stimulating hormone, serum male (adult): 5-15 mU/mL (5-15 units/L), female: follicular or luteal phase, 5-20 mU/mL (5-20 units/L); midcycle peak, 30-50 mU/mL (30-50 units/L); postmenopausal, greater than 35 mU/mL (35 units/L); growth hormone, plasma: after oral glucose: less than 2 ng/mL (2 µg/L); response to provocative stimuli: greater than 7 ng/mL (7 µg/L); luteinizing hormone, serum male: 3-15 mU/mL (3-15 units/L) female: follicular or luteal phase, 5-22 mU/mL (5-22 units/L); midcycle peak, 30-250 mU/mL (30-250 units/L); postmenopausal, greater than 30 mU/mL (30 units/L); metanephrine, urine: less than 1.2 mg/24 h (6.1 mmol/ 24 h); norepinephrine, plasma (supine): 50-440 ng/L (296-2600 pmol/L); parathyroid hormone, serum: 10-65 pg/mL (10-65 ng/L); progesterone, blood male (adult): 0.27-0.9 ng/mL (0.9-2.9 nmol/L) female: follicular phase, 0.33-1.20 ng/mL (1.0-3.8 nmol/L); luteal phase, 0.72-17.8 ng/mL (2.3-56.6 nmol/L); postmenopausal, <0.2-1 ng/mL (0.6-3.18 nmol/L); oral contraceptives, 0.34-0.92 ng/mL (1.1-2.9 nmol/L); prolactin, serum male: less than 15 ng/mL (15 µg/L) female: less than 20 ng/mL (20 µg/L); testosterone, serum male (adult): 300-1200 ng/dL (10-42 nmol/L) female: 20-75 ng/dL (0.7-2.6 nmol/L); thyroid function tests, thyroid iodine (¹³¹I) uptake: 10%-30% of administered dose at 24 h, thyroid-stimulating hormone (TSH): 0.5-5.0 µU/mL (0.5-5.0 mU/L), thyroxine (T4), serum total: 5-12 µg/dL (64-155 nmol/L) free: 0.9-2.4 ng/dL (12-31 pmol/L) free T4 index: 4-11; triiodothyronine, free (T3): 3.6-5.6 ng/L (5.6-8.6); triiodothyronine, resin (T3): 25%-35%; triiodothyronine, serum (T3): 70-195 ng/dL (1.1-3.0 nmol/L); vanillylmandelic acid, urine: less than 8 mg/24 h (40.4 µmol/24 h); vitamin D 1,25-dihydroxy, serum: 25-65 pg/mL (60-156 pmol/L) 25-hydroxy, serum: 25-80 ng/mL (62-200 nmol/L);

A healthy human subject has the following characteristic laboratory values for urinalysis test panels results: albumin-creatinine ratio: less than 30 mg/g; calcium: 100-300 mg/24 h (2.5-7.5 mmol/24 h) on unrestricted diet; creatinine: 15-25 mg/kg per 24 h (133-221 mmol/kg per 24 h); glomerular filtration rate (GFR) normal male: 130 mL/min/1.73 m² female: 120 mL/min/1.73 m²; 5-hydroxyindoleacetic acid (5-HIAA): 2-9 mg/24 h (10.4-46.8 µmol/24 h); protein-creatinine ratio - less than or equal to 0.2 mg/mg; sodium: 100-260 meq/24 h (100-260 mmol/24 h) (varies with intake); uric acid: 250-750 mg/24 h (1.48-4.43 mmol/24 h) (varies with diet).

A healthy human subject has the following characteristic laboratory values for gastrointestinal test panels results. gastrin, serum: 0-180 pg/mL (0-180 ng/L); stool fat: less than 5 g/d on a 100-g fat diet; stool weight: less than 200 g/d.

A healthy human subject has the following characteristic values for pulmonary test. Forced expiratory volume in 1 second (FEV1): greater than 80% of predicted; Forced vital capacity (FVC): greater than 80% of predicted; FEV1/FVC: greater than 75%.

A healthy human subject has the following characteristic laboratory values for cerebrospinal fluid test panels results: cell count: 0-5/µL (0-5 x 10⁶/L); glucose: 40-80 mg/dL (2.2-4.4 mmol/L); less than 40% of simultaneous plasma concentration is abnormal; pressure (opening): 70-200 mm H₂O; protein: 15-60 mg/dL (150-600 mg/L).

A healthy human subject has the following characteristic hemodynamic test values: cardiac index: 2.5-4.2 L/min/m2; left ventricular ejection fraction: greater than 55%; pressures: pulmonary artery systolic: 20-25 mm Hg, diastolic: 5-10 mm Hg Mean: 9-16 mm Hg; pulmonary capillary wedge: 6-12 mm Hg, right atrium: mean 0-5 mm Hg, right ventricle systolic: 20-25 mm Hg, diastolic: 0-5 mm Hg.

In addition, a generally normal healthy human subject has a resting pulse rate in the range of from 50 to 90 beats/min, while wider ranges are acceptable for non-human subjects.

As used herein, the term "treatment" refers to a procedure performed after diagnosis of the condition.

As used herein, the term "mitigation" refers a procedure that is performed to prevent, or decrease the likelihood, of an anticipated injury or disease.

As used herein, the term "immune checkpoint proteins" refers to proteins present on the surface of normal cells that can engage with the corresponding ligand in immune cells to stop the immune system (through apoptosis) from destroying normal cells or tissues of the body and thus inhibit or prevent autoimmune disease. Well-known immune checkpoint proteins that are targeted for inhibition to treat cancers include CTLA-4, PD1, PD-L1, PD-L2. In addition to these, LAG-3, BTLA, B7H3, B7H4, TIM-3, MR are also recognized in the art to constitute immune checkpoint protein similar to the CTLA-4 and PD-1 (*see, e.g.,* Pardoll, 2012, Nature Rev Cancer 12:252-264; Mellman et al., 2011, Nature 480:480-489, incorporated herein by reference in its entirety). These immune checkpoint proteins are present on the surface of cells that provides the immune system with inhibitory signals that stop the immune system from destroying normal cells or tissues of the body. However, cancer cell also expresses immune checkpoint proteins to evade the immune system and allow for unlimited tumor growth. Masking or inhibiting the immune checkpoint proteins using an immune checkpoint inhibitor allows activation of the immune system to destroy cancer cells.

As used herein, the term "immune checkpoint inhibitor" or "ICPI" refers any compound that that can activate the immune system to attack a tumor or cancer; and/or can inhibit the overall function of an immune checkpoint protein, either directly or indirectly, allowing cytotoxic T cells to be activated within the tissue (or tumor) and/or reducing Treg immune cells and causing the proliferation of cytotoxic T cells in the tissue (or tumor) to damage or eliminate the tissue (or tumor). Most of the ICPI developed thus far bind directly with an immune checkpoint protein. There are many examples of ICPI proteins or polypeptides that can bind directly to immune checkpoint proteins or its ligands on the surface of cells and facilitate activation of the immune system. Known immune checkpoint proteins that can be inhibited by direct antibody or protein binding includes CTLA-4, PD1, PD-L1, PD-L2. In addition to these, LAG-3, BTLA, B7H3, B7H4, TIM-3, MR are also recognized in the art to constitute immune checkpoint protein similar to the CTLA-4 and PD-1 (*see, e.g.,* Pardoll, 2012, Nature Rev Cancer 12:252-264; Mellman et al., 2011, Nature 480:480-489, incorporated herein in its entirety) and can potentially be inhibited in a similar manner. There are also ICPI that may not bind directly to these immune checkpoint proteins but can activate cytotoxic T cells within the tumor tissue and/or causes reduction of Treg immune cells and the proliferation of cytotoxic T cells in the tumor tissue to damage or eliminate the tumor tissue. The present disclosure concerns the latter category of ICPI.

Direct blockers of immune checkpoint protein include polypeptides or compounds that specifically bind or mask the action of the immune checkpoint protein, thereby facilitating immunity or destruction of cancer. Several antibody checkpoint inhibitors have been approved by the Food and Drug Administration for the treatment of cancer, including pembrolizumab (against PD-1; marketed as Keytruda by Merck), Nivolumab (against PD-1 marketed as Opdivo by Bristol-Myers Squibb (*see, e.g.,* Topalian et al., 2012, N. Eng. J. Med. 366:2443-2454, U.S. Pat. No. 8,008,449 B2)), Ipilimumab (against CTLA4, marketed as Yervoy by Bristol-Myers Squib), Atezolizumab (against PD-L1, marketed as Tecentriq by Roche & Genentech), Avelumab (against PD-L1, marketed as Bavencio by Merck Serono & Pfizer), Durvalumab (against PD-L1, marketed as Imfinzi by AstraZeneca), and Cemiplimab (against PD-L1, marketed as Libtayo by Sanofi). Other immune checkpoint inhibitors include tremelimumab (inhibits CTLA-4) (*see, e.g.,* Ribas et al., 2013, J Clin. Oncol. 31:616-22), lambrolizumab (inhibits PD-1) (WO2008/156712; Hamid et al., 2013, N Engl. J. Med. 369: 134-144), pidilizumab (inhibits PD-1) (*see, e.g.,* Rosenblatt et al., 2011. J Immunother: 34:409-18). Other PD-1 inhibitors may include soluble PD-1 ligand including without limitation PD-L2 Fc fusion protein, also known as B7-DC-Igor AMP-244 (*see, e.g.,* Mkrtichyan M. et al. J Immunol. 189:2338-47 2012). Immune checkpoint inhibitors may include without limitation humanized or fully human antibodies blocking PD-L1 such as MEDI-4736 (*see, e.g.,* WO2011066389 A1), and MPDL3280A (U.S. Pat. No. 8,217,149 B2). Other PD-L1 inhibitors are presently under investigation.

As used herein, a "liquid" is a substance which flows freely at room temperature, such that its shape changes but its volume retains constant, *e.g*., as would water or an oil.

As used herein, "room temperature" denotes a typical ambient indoor temperature of about 25°C.

Unless defined otherwise, any feature within any aspect or embodiment of the disclosure may be combined with any feature within any other aspect or embodiment of the invention, and such combination are encompassed in the present disclosure. This also applies, but not exclusively, to endpoints of ranges disclosed herein. For instance, if a given substance is disclosed as existing in a composition in a concentration range of X-Y% or A-B%, the present disclosure is to be understood as explicitly disclosing not only the ranges X-Y% and A-B%, but also the ranges X-B%, A-Y% and, in as far as numerically possible, Y-A% and B-X%. Each of these ranges, and range combinations, are contemplated, and are to be understood as being directly and unambiguously disclosed in the present application.

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from X to Y". Accordingly, the expressions of ranges as "X-Y", "of X to Y", "from X to Y", "of X-Y" and "from X-Y" are to be understood equivalently as meaning and disclosing a range encompassing the end value X, all values (including decimals) between X and Y, as well as the end value Y.

As used herein the term "about" when referring to a particular value, *e.g*., an endpoint or endpoints of a range, encompasses and discloses, in addition to the specifically recited value itself, a certain variation around that specifically recited value. Such a variation may for example arise from normal measurement variability, *e.g*., in the weighing or apportioning of various substances by methods known to the skilled person. The term "about" shall be understood as encompassing and disclosing a range of variability above and below an indicated specific value, said percentage values being relative to the specific recited value itself, as follows: The term "about" may encompass and disclose variability of ± 5.0%. The term "about" may encompass and disclose variability of ± 4.5%. The term "about" may encompass and disclose variability of ± 4.0%. The term "about" may encompass and disclose variability of ± 3.5%. The term "about" may encompass and disclose variability of ± 3.0%. The term "about" may encompass and disclose variability of ± 2.5%. The term "about" may encompass and disclose variability of ± 2.0%. The term "about" may encompass and disclose variability of ± 1.5%. The term "about" may encompass and disclose variability of ± 1.0%. The term "about" may encompass and disclose variability of ± 0.5%. The term "about", in reference to the particular recited value, may encompass and disclose that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed in the present application. Unless stated otherwise, where the term "about" is recited before the first endpoint of a numerical range, but not before the second endpoint of that range, this term, and the variability it implies in scope and disclosure, refers to both the first endpoint of the range and the second endpoint of the range. For instance, a recited range of "about X to Y" should be read as "about X to about Y". The same applies for a recited range of ratios. For instance, a recited range of weight ratios of "about X:Y - A:B" should be read as a weight ratio of "(about X):(about Y) - (about A):(about B)".

Unless otherwise defined, all technical and scientific terms used herein have the same meaning as commonly understood by one of ordinary skill in the art. Although methods and materials similar or equivalent to those described herein can be used in the practice or testing of the present disclosure, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting. It will be readily understood that the aspects of the present disclosure, as generally described herein, and illustrated in the figures, can be arranged, substituted, combined, separated, and designed in a wide variety of different configurations, all of which are explicitly contemplated herein.

Furthermore, the particular arrangements shown in the FIGURES should not be viewed as limiting. It should be understood that other embodiments may include more or less of each element shown in a given FIGURE. Further, some of the illustrated elements may be combined or omitted. Yet further, an example embodiment may include elements that are not illustrated in the FIGURES.

### Treatment methods

The methods of the present disclosure are made possible by the surprising discovery that CNP can be modified, derivatized, and/or formulated in such a way that it can induce/cause increase and/or maximized cyclic-GMP production without the associated detrimental drop in blood pressure. In particular, the blood pressure effect of CNP can be minimized or eliminated at a therapeutic bolus dose that increases plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, depending on the peptide. In the present disclosure, the baseline plasma cyclic-GMP level is defined as the plasma level prior to administration of the bolus dose, the plasma level of the healthy subject which is 4 +/- 1 pmol/ml, or about 1.4 mg/ml in humans (but which can vary between species). *See, e.g.,* Shotan et al., Plasma cyclic guanosine monophosphate in chronic heart failure: hemodynamic and neurohormonal correlations and response to nitrate therapy. Clin Pharmacol Ther, 1993. 54(6): p. 638-44, incorporated herein in its entirety. In a preferred embodiment, the baseline level is the measured level prior to drug administration for the same subject to which treatment is provided, and that level can vary from one subject to the next. In practicing the present disclosure, any baseline parameter that used as a reference parameter to evaluate the effect of the treatment is established by measurement prior to treatment. Typically, but not exclusively, the baseline plasma cyclic-GMP level varies depending on the time of the day with lower level during day-time wakefulness, higher soon after bedtime, and can vary from 2-8 pmol/ml throughout the day in human. Thus, the measured baseline plasma cyclic GMP level prior to administration of the composition and the measured plasma cyclic GMP level after administration of the compositions of the present disclosure can occur at the same predetermined time every day. Where an average baseline is described, the average baseline can be the average baseline measurement taken at least 3 times at an interval of at least 4 hours for a given parameter within 24 hour period for a given subject. This controls for inter-subject or inter-individual variability. In patients with congestive heart failure the baseline plasma cyclic-GMP level may be 2 to 3-fold higher and the baseline is established prior to treatment for each individual subject or group of subjects. Similarly for blood pressure, the baseline will be measured level prior to drug administration and is used as reference to evaluate the effect of the treatment. The baseline cGMP level in healthy mouse with no known symptoms of any health condition is 20 (3.7) pmol/mL [mean (SEM); n=8] or 7 (1.3) ng/mL [mean (SEM); n=8]. The baseline cGMP level in dogs with no known symptoms of any health condition is 5-12 ng/ml.

As will be demonstrated in the Examples, when subjects with cancer were administered with a bolus dose of long acting CNP derivative, the growth of cancer was stopped and/or reversed, accompanied by a surprising vascular normalization and activation of the immune system against cancer. Furthermore, when subjects with cancer were treated with by administering a bolus dose of long acting CNP derivative, the following were observed: a vasculature normalization or an increase in pericyte coating index by at least 10% (*e.g.,* by at least 15%, or by at least 20%) (*e.g.,* within the tumor tissue), decreased tumor size, improved survival, reduced hypoxia within the tumor tissue, increased number of cancer-killing cytotoxic T cells, increased number of activated NK cells, decreased number of Treg cells, decreased number of myeloid-derived suppressor cells, decreased TGFβ expression, decreased Foxp3 expression, inhibition of immune checkpoint activity within tumor tissue, and/or decreased Bv8 expression. When subjects with cancer that is resistant or partially resistant to immune checkpoint inhibitors were co-treated with a composition including a long acting CNP derivative and an immune checkpoint inhibitor, a significant improvement in efficacy was observed, indicating that the long acting CNP derivative can increase the efficacy of immune checkpoint inhibitors.

The present disclosure features methods of treating a subject (*e.g.,* a mammalian subject, a patient in need thereof) with abnormal vasculature, in any tissue or organ, by normalizing vasculature, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. In some embodiments, the present disclosure relates to a method of increasing cytotoxic T-cells and/or activated NK cells, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. The subject can additionally have one or more of a low number of cytotoxic T cells, a low number of activated NK cells, a high number of Treg cells, a high level of expression of TGFβ, a high level or expression of Foxp3, a high number of myeloid-derived suppressor cells or MDSCs; and/or a high level or expression of Bv8. For example, the subject can have abnormal vasculature, a low number of cytotoxic T cells, and/or a low number of activated NK cells. In some embodiments, the subject has a condition (i) to (viii): (i) a low number of cytotoxic T cells, (ii) a low number of activated NK cells, (iii) a high number of Treg cells, (iv) a high level of expression of TGFβ, (v) a high level or expression of Foxp3, (vi) a high number of myeloid-derived suppressor cells or MDSCs, (vii) a high level or expression of Bv8; or (viii) any combination thereof; or the subject is in need of (ix) to (xvi): (ix) an increase in a number of cytotoxic T-cells; (x) an increase in activated NK cells; (xi) a decrease in a number of Treg cells; (xii) a decrease in TGF-β expression; (xiii) a decrease in Foxp3 expression; (xiv) a decrease in a number of myeloi d-derived suppressor cells (MDSCs); (xv) a decrease in Bv8 expression, or (xvi) any combination thereof. The present disclosure also features methods of treating a subject (*e.g.,* a patient in need thereof) with cancer by activating the subject's immune system to attack tumor or cancer, by increasing the number of cytotoxic T cells and/or activated NK cells, by reducing the number of immune suppressor cells (Treg cells), by decreasing immune suppressor cytokines (Transforming growth factor beta or TGFb or TGFβ), by decreasing Foxp3 (Treg marker) and/or Bv8 (marker for myeloid-derived suppressor cells or MDSCs marker), and/or by normalizing vasculature in the tumor tissue that allows access to anti-cancer drugs and reduces hypoxia (hypoxia facilitate tumor growth/malignancy and immune resistance). The methods include administering to the subject a therapeutically effective bolus dose of cytotoxic cell immunostimulant composition including a long acting CNP, a very long acting CNP, a long acting CNP derivative, a very long acting CNP derivative, a long acting NPRB agonist, and/or a very long acting NPRB agonist. In some embodiments, when administered to the subject in need thereof, the therapeutically effective bolus dose of the composition provides an increase in the number of cytotoxic T cells and/or NK cells of at least 15% (*e.g.,* of at least 20%, or of at least 30%) above the level prior to administration of the composition or above the level in a healthy subject. The therapeutically effective bolus dose is a dose that does not decrease or cause a decrease in blood pressure (or mean arterial pressure) by more than 20% (*e.g.,* by more than 15%, by more than 9%, or by more than 5%) of a baseline blood pressure measurement, where the baseline blood pressure measurement is an average blood pressure prior to administration of the composition, but the dose can increase plasma cyclic-GMP level at from 1 hour to 12 hours after administration (*e.g.,* 2 to 12 hours, 4 to 12 hours, 1 hour to-24 hours, 2 to 24 hours, 4-24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject.

In some embodiments, the present disclosure features methods of treating a subject (*e.g.,* a mammalian subject, a patient in need thereof) with abnormal vasculature, in any tissue or organ, by normalizing the vasculature, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. In some embodiments, the present disclosure relates to a method of increasing cytotoxic T-cells and/or activated NK cells, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. The subject can additionally have one or more of a low number of cytotoxic T cells, a low number of activated NK cells, a high number of Treg cells, a high level of expression of TGFβ, a high level or expression of Foxp3, a high number of myeloid-derived suppressor cells or MDSCs; and/or a high level or expression of Bv8. In some embodiments, the subject can additionally has a condition (i) to (viii): (i) a low number of cytotoxic T cells, (ii) a low number of activated NK cells, (iii) a high number of Treg cells, (iv) a high level of expression of TGFβ, (v) a high level or expression of Foxp3, (vi) a high number of myeloid-derived suppressor cells or MDSCs, (vii) a high level or expression of Bv8; or (viii) any combination thereof, or the subject is in need of (ix) to (xvi): (ix) an increase in a number of cytotoxic T-cells; (x) an increase in activated NK cells; (xi) a decrease in a number of Treg cells; (xii) a decrease in TGF-β expression; (xiii) a decrease in Foxp3 expression; (xiv) a decrease in a number of myeloid-derived suppressor cells (MDSCs); (xv) a decrease in Bv8 expression, or (xvi) any combination thereof. For example, the subject can have abnormal vasculature, a low number of cytotoxic T cells, and/or a low number of activated NK cells. In some embodiments, the present disclosure features methods of treating a subject (*e.g.,* a mammalian subject, a patient in need thereof) with cancer with one or more of the following conditions: a tumor; a low number or an absence of cytotoxic T cells and/or activated NK cells within the tumor tissue; a presence or a high number of immune suppressor cells (*e.g.,* Treg cells), a presence or high level of immune suppressor cytokines (*e.g*., transforming growth factor beta or TGFβ), a high level of cells positive for Foxp3 (*e.g.,* a Treg marker), a high level of cells positive for Bv8 (marker for myeloid-derived suppressor cells or MDSCs marker), a high number of myeloid-derived suppressor cells or MDSCs, and/or abnormal vasculature in the tumor tissue that allows access to anti-cancer drugs and reduces hypoxia (hypoxia facilitate tumor growth/malignancy and immune resistance). The methods include administering to the subject a therapeutically effective bolus dose of a composition (*e.g.,* a cytotoxic cell immunostimulant composition) including a long acting CNP, a very long acting CNP, a long acting CNP derivative, a very long acting CNP derivative, a long acting NPRB agonist, and/or a very long acting NPRB agonist. In some embodiments, when administered to the subject in need thereof, the therapeutically effective bolus dose of the composition provides an increase in the number of cytotoxic T cells and/or NK cells of at least 15% (*e.g.,* of at least 20%, or of at least 30%) above the level prior to administration of the composition or above the level in a healthy subject. The therapeutically effective bolus dose is a dose that does not decrease or cause a decrease in blood pressure (or mean arterial pressure) by more than 20% (*e.g.,* by more than 15%, by more than 10%, or by more than 5%) of a baseline blood pressure measurement, where the baseline blood pressure measurement is an average blood pressure prior to administration of the composition, but the dose can increase plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the plasma level prior to administration of the bolus dose or the plasma level of the healthy subject which is 4 +/- 1 pmol/ml or about 1.4 mg/ml in humans but can vary between species and the assay used. *See, e.g.,* Shotan, et al., Plasma cyclic guanosine monophosphate in chronic heart failure: hemodynamic and neurohormonal correlations and response to nitrate therapy. Clin Pharmacol Ther, 1993. 54(6): p. 638-44, incorporated herein in its entirety. The subject treated with the composition can have a lifespan or survival that is increased compared to a subject that has not been treated with the therapeutically effective bolus dose of the composition (*see, e.g.,* FIGURE 20).

In a preferred embodiment, the baseline level for a given marker or parameter is the measured level prior to drug administration and that level can vary from one subject to the next. In practicing the present disclosure, any baseline parameter that used as a reference parameter to evaluate the effect of the treatment is established by measurement prior to treatment. Typically, but not exclusively, the baseline plasma cyclic-GMP level varies depending on the time of the day with lower level during day-time wakefulness, higher soon after bedtime, and can vary from 2-8 pmol/ml throughout the day in human. Thus, the measured baseline plasma cyclic GMP level prior to administration of the composition and the measured plasma cyclic GMP level after administration of the compositions of the present disclosure can occur at the same predetermined time every day. Where an average baseline is described, the average baseline can be the average baseline measurement taken at least 3 times at least 4 hours apart for a given parameter within 24 hour period for a given subject. This controls for inter-subject or inter-individual variability. In patients with congestive heart failure the baseline plasma cyclic-GMP level may be 2 to 3-fold higher and the baseline is established prior to treatment for each individual subject or group of subjects. Similarly for blood pressure, the baseline will be measured level prior to drug administration and is used as reference to evaluate the effect of the treatment. The baseline cGMP level in healthy mouse with no known symptoms of any health condition is 20 (3.7) pmol/mL [mean (SEM); n=8] or 7 (1.3) ng/mL [mean (SEM); n=8]. The baseline cGMP level in dogs with no known symptoms of any health condition is 5-12 ng/ml.

In some embodiments, the present disclosure features methods of treating a subject (*e.g.,* a mammalian subject, a patient in need thereof) with abnormal vasculature, in any tissue or organ, by normalizing the vasculature, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. In some embodiments, the present disclosure relates to a method of increasing cytotoxic T-cells and/or activated NK cells, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. The subject can additionally have one or more of a low number of cytotoxic T cells, a low number of activated NK cells, a high number of Treg cells, a high level of expression TGFβ, a high level or expression of Foxp3, a high number of myeloid-derived suppressor cells or MDSCs; and/or a high level or expression of Bv8. For example, the subject can have abnormal vasculature, a low number of cytotoxic T cells, and/or a low number of activated NK cells. In some embodiments, the subject can additionally have a condition (i) to (viii): (i) a low number of cytotoxic T cells, (ii) a low number of activated NK cells, (iii) a high number of Treg cells, (iv) a high level of expression of TGFβ, (v) a high level or expression of Foxp3, (vi) a high number of myeloid-derived suppressor cells or MDSCs, (vii) a high level or expression of Bv8; or (viii) any combination thereof; or the subject is in need of (ix) to (xvi): (ix) an increase in a number of cytotoxic T-cells; (x) an increase in activated NK cells; (xi) a decrease in a number of Treg cells; (xii) a decrease in TGF-β expression; (xiii) a decrease in Foxp3 expression; (xiv) a decrease in a number of myeloid-derived suppressor cells (MDSCs); (xv) a decrease in Bv8 expression, or (xvi) any combination thereof. In some embodiments, the present disclosure features methods of treating a subject (*e.g.,* a patient in need thereof) with cancer by activating the subject's immune system to attack tumor or cancer, increase the number of cytotoxic T cells and/or activated NK cells, reduce the number of immune suppressor cells (*e.g.,* Treg cells), decrease immune suppressor cytokines (*e.g.,* transforming growth factor beta or TGFβ), decrease Foxp3 (*e.g.,* a Treg marker), decrease Bv8 (a marker for myeloid-derived suppressor cells or MDSCs marker), decrease the number of myeloid-derived suppressor cells (MDSCs), normalize vasculature in the tumor tissue that allows access to anti-cancer drugs and reduces hypoxia (hypoxia facilitate tumor growth/malignancy and immune resistance). The treatment can increase a subject's lifespan or survival compared to a subject that has not been treated with the therapeutically effective bolus dose of the composition. The methods include administering to the subject a therapeutically effective bolus dose of a composition (*e.g.,* a cytotoxic cell immunostimulant composition) including a very long acting CNP derivative. In some embodiments, when administered to the subject in need thereof, the therapeutically effective bolus dose of the composition provides an increase in the number of cytotoxic T cells and/or NK cells of at least 15% (*e.g.,* of at least 20%, or of at least 30%) above the level prior to administration of the composition or above the level in a healthy subject. The therapeutically effective bolus dose is a dose that does not decrease or cause a decrease in blood pressure (or mean arterial pressure) by more than 20% (*e.g.,* by more than 15%, by more than 10%, or by more than 5%) of a baseline blood pressure measurement, where the baseline blood pressure measurement is an average blood pressure prior to administration of the composition, but the dose can increase plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the present disclosure features methods of treating a subject (*e.g.,* a patient in need thereof) with abnormal vasculature, in any tissue or organ, by normalizing the vasculature, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. In some embodiments, the present disclosure relates to a method of increasing cytotoxic T-cells and/or activated NK cells, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. The subject can additionally have one or more of a low number of cytotoxic T cells, a low number of activated NK cells, a high number of Treg cells, a high level of expression TGFβ, a high level or expression of Foxp3, a high number of myeloid-derived suppressor cells or MDSCs; and/or a high level or expression of Bv8. In some embodiments, the subject can additionally have a condition (i) to (viii): (i) a low number of cytotoxic T cells, (ii) a low number of activated NK cells, (iii) a high number of Treg cells, (iv) a high level of expression of TGFβ, (v) a high level or expression of Foxp3, (vi) a high number of myeloid-derived suppressor cells or MDSCs, (vii) a high level or expression of Bv8; or (viii) any combination thereof; or the subject is in need of (ix) to (xvi): (ix) an increase in a number of cytotoxic T-cells; (x) an increase in activated NK cells; (xi) a decrease in a number of Treg cells; (xii) a decrease in TGF-β expression; (xiii) a decrease in Foxp3 expression; (xiv) a decrease in a number of myeloid-derived suppressor cells (MDSCs); (xv) a decrease in Bv8 expression, or (xvi) any combination thereof. For example, the subject can have abnormal vasculature, a low number of cytotoxic T cells, and/or a low number of activated NK cells. In some embodiments, the present disclosure features methods of treating a subject (*e.g.,* a patient in need thereof) with cancer with one or more of the following conditions: a tumor; a low number or an absence of cytotoxic T cells and/or activated NK cells within the tumor tissue; a presence or a high number of immune suppressor cells (*e.g.,* Treg cells), a presence or high level of immune suppressor cytokines (*e.g.,* transforming growth factor beta or TGFβ), a high level of cells positive for Foxp3 (*e.g.,* a Treg marker), a high level of cells positive for Bv8 (marker for myeloid-derived suppressor cells or MDSCs marker), a high number of myeloid-derived suppressor cells or MDSCs, and/or abnormal vasculature in the tumor tissue that allows access to anti-cancer drugs and reduces hypoxia (hypoxia facilitate tumor growth/malignancy and immune resistance). The methods include administering to the subject a therapeutically effective bolus dose of cytotoxic cell immunostimulant composition including a long acting CNP derivative, and/or a very long acting CNP derivative. In some embodiments, when administered to the subject in need thereof, the therapeutically effective bolus dose of the composition provides an increase in the number of cytotoxic T cells and/or NK cells of at least 15% (*e.g.,* of at least 20%, or of at least 30%) above the level prior to administration of the composition or above the level in a healthy subject. The therapeutically effective bolus dose is a dose that does not decrease or cause a decrease in blood pressure (or mean arterial pressure) by more than 20% (e*.g.,* by more than 15%, by more than 10%, or by more than 5%) of a baseline blood pressure measurement, where the baseline blood pressure measurement is an average blood pressure prior to administration of the composition, but the dose can increase plasma cyclic-GMP level at from 1 hour to 12 hours after administration (*e.g.,* 2 to 12 hours, 4 to 12 hours, 1 hour to 24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the present disclosure features methods of treating a subject (*e.g.,* a patient in need thereof) with fibrosis associated with tumors, by administering to a subject in need thereof a therapeutically effective bolus dose of a composition including a long acting C-type natriuretic peptide (CNP), CNP derivative, long acting CNP derivative, long acting CNP receptor (NPRB) agonist, or any combination thereof. Expression of markers associated with fibrosis (*e.g.,* α-SMA, TGFβ, and/or Ang 2) can be decreased after administration.

In some embodiments, the subject (*e.g.,* a mammalian subject, a patient in need thereof) having abnormal vasculature can have cancer. In some embodiments, the subject with cancer has one or more of the following cancers: mantle cell lymphoma, primary CNS lymphoma, Burkitt's lymphoma, marginal Zone B cell lymphoma, polycythemia vera lymphoma, Hodgkin's disease, non-Hodgkin's disease, solid tumors, sarcomas, carcinomas, fibrosarcoma, myxosarcoma, liposarcoma, chrondrosarcoma, osteogenic sarcoma, osteosarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon sarcoma, colorectal carcinoma, pancreatic cancer, breast cancer, ovarian cancer, prostate cancer, squamous cell carcinoma, basal cell carcinoma, adenocarcinoma, Sweat gland carcinoma, Sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular tumor, lung carcinoma, Small cell lung carcinoma, non-small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, retinoblastoma, nasopharyngeal carcinoma, esophageal carcinoma, basal cell carcinoma, biliary tract cancer, bladder cancer, bone cancer, brain and central nervous system (CNS) cancer, cervical cancer, choriocarcinoma, colorectal cancers, connective tissue cancer, cancer of the digestive system, endometrial cancer, esophageal cancer, eye cancer, head and neck cancer, gastric cancer, intraepithelial neoplasm, kidney cancer, larynx cancer, liver cancer, lung cancer (small cell and/or large cell), melanoma, neuroblastoma; oral cavity cancer (for example lip, tongue, mouth and pharynx), ovarian cancer, pancreatic cancer, retinoblastoma, rhabdomyosarcoma, rectal cancer, cancer of the respiratory system, sarcoma, skin cancer, stomach cancer, testicular cancer, thyroid cancer, uterine cancer, and cancer of the urinary system.

In some embodiments, the subject (*e.g.,* a mammalian subject, a patient in need thereof) with cancer has one or more of cancers in the following organs: skin, breast, bone, prostate, colon, head, neck, liver, kidney, cervix, lung, stomach, urethra, bladder, ureters, renal pelvis, rectum, esophagus, lymph node, pancreas, stomach, ovary, central nervous system, soft tissue, and/or endocrine glands. The subject is administered with a therapeutically effective bolus dose of a composition (*e.g.,* a cytotoxic cell immunostimulant composition) including a long acting CNP, a very long acting CNP, a long acting CNP derivative, a very long acting CNP derivative, a long acting NPRB agonist, and/or a very long acting NPRB agonist. The therapeutically effective bolus dose is a dose as described above.

In some embodiments, the subject (*e.g.,* a mammalian subject, a patient in need thereof) with cancer has one or more of cancers in the following organs: skin, breast, bone, prostate, colon, head, neck, liver, kidney, cervix, lung, stomach, urethra, bladder, ureters, renal pelvis, rectum, esophagus, lymph node, pancreas, stomach, ovary, central nervous system, soft tissue, and/or endocrine glands. The subject is administered with a therapeutically effective bolus dose of a composition (*e.g.,* a cytotoxic cell immunostimulant composition) including a long acting CNP derivative. The therapeutically effective bolus dose is a dose as described above.

In some embodiments, the subject (*e.g.,* a mammalian subject, a patient in need thereof) with cancer has one or more of cancers in the following organs: skin, breast, bone, prostate, and/or colon. The subject is administered with a therapeutically effective bolus dose of a composition (*e.g.,* a cytotoxic cell immunostimulant composition) including a long acting CNP, a very long acting CNP, a long acting CNP derivative, a very long acting CNP derivative, along acting NPRB agonist, and/or a very long acting NPRB agonist. The therapeutically effective bolus dose is a dose as described above.

In some embodiments, the subject (*e.g.,* a mammalian subject, a patient in need thereof) with cancer has one or more of cancers in the following organs: skin, breast, bone, prostate, and/or colon. The subject is administered with a therapeutically effective bolus dose of a composition (*e.g.,* a cytotoxic cell immunostimulant composition) including a long acting CNP derivative. The therapeutically effective bolus dose is a dose as described above.

In some embodiments, the cancer in any one of the above methods includes a solid tumor in one or more of the following organs: pancreas, bladder, colon rectum, breast, prostate, kidney, liver, lung, ovary, cervix, stomach, esophagus, head, neck, skin, endocrine glands, central nervous system, bone, and/or soft tissue.

The long acting NPRB agonist or the very long acting NPRB agonist can include a polypeptide, such as an antibody. In some embodiments, the long acting NPRB agonist or the very long acting NPRB agonist includes a molecule having a molecular weight of less than 2kDa.

In some embodiments, in any one of the above methods, the composition has limited or no agonistic activity against NPRA and/or has greater than 5-fold greater binding affinity (or 5-fold lower EC50) for NPRB receptor than NPRA receptor.

In some embodiments, the therapeutically effective bolus dose of a composition of the present disclosure is a dose that does not decrease or cause a decrease in blood pressure (or mean arterial pressure) by more than 20% (*e.g.,* by more than 15%, by more than 10%, or by more than 5%) of a baseline blood pressure measurement, where the baseline blood pressure measurement is an average blood pressure prior to administration of the composition, but the dose increases plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the therapeutically effective bolus dose of a composition of the present disclosure is a dose that does not decrease or cause a decrease in blood pressure (or mean arterial pressure) by more than 15% of a baseline blood pressure measurement, where the baseline blood pressure measurement is an average blood pressure prior to administration of the composition, but the dose increases plasma cyclic-GMP level at from 1 hour to 12 hours after administration (*e.g.,* 2 to 12 hours, 4 to 12 hours, 1 hour to 24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the therapeutically effective bolus dose of a composition of the present disclosure does not decrease blood pressure by more than 10% but the dose increases plasma cyclic-GMP level at from 1 hour to 12 hours after administration (*e.g*., 2 to 12 hours, 4 to 12 hours, 1 hour to 24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the therapeutically effective bolus dose of a composition of the present disclosure does not decrease blood pressure by more than 5% but the dose increases plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, for any one of the above methods, administering to a subject includes an administration method such as enteral (*e.g.,* oral) administration or parenteral administration. Examples of parenteral administration are subcutaneous, intravenous, intramuscular, by inhalation, nasal, or any combination thereof. In some embodiments, the methods above can include enteral (*e.g.,* oral) administration and/or subcutaneous administration. In certain embodiments, the methods above include intravenous administration. In some embodiments, the methods above include intramuscular administration. In some embodiments, the methods above include administration by inhalation (*e.g.,* by intratracheal inhalation administration, where a subject is exposed to high aerosol concentrations so that the active pharmaceutical ingredient is deposited directly in the lower respiratory tract). In certain embodiments, the methods above include nasal administration. In some embodiments, the methods above include enteral (*e.g.,* oral) administration.

In some embodiments, for any one of the above methods, administering to a subject consists essentially of, or consists of, administering the compositions of the present disclosure as a bolus dose. In some embodiments, for any one of the above methods, administering to a subject does not include administration of the compositions of the present disclosure by infusion over a sustained period of time (*e.g.,* by continuous infusion). In some embodiments, for any of the above methods, administering to a subject does not include administering the compositions of the present disclosure as a bolus dose followed by an infusion over a sustained period of time. In some embodiments, for any one of the above methods, administering to a subject does not include enteral or oral administration of the compositions of the present disclosure. In some embodiments, for any one of the above methods, administering to a subject does not include oral administration of the compositions of the present disclosure.

### Active pharmaceutical ingredients

For any of the above methods described above, the long acting CNP derivative or very long acting CNP derivative, can include U-GLSKGCFGLKLDRIGSMSGLGC [SEQ ID NO. 2]; U-GLSKGCFGLK(U)LDRIGSMSGLGC [SEQ ID NO. 3]; GLSKGCFGLK(U)LDRIGSMSGLGC [SEQ ID NO. 4]; and/or U-CFGLKLDRIGSxSGLGC, where x is a natural or unnatural amino acid residue [SEQ ID NO. 12], where U is attached to the N-terminal G, C and/or to the epsilon amino of K residue.

In some embodiments, U in the sequences above is a moiety of Formula (I) or (II), where Formula (I) is

(aliphatic)ₐ-(X)-; (1)

wherein:
a is 0 or 1 (preferably a is 1);
aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
X is a 1-10 amino acid residue or peptide sequence, wherein each amino acid residue is independently selected from lysine (K), arginine (R), glycine (G), alanine (A), glutamic acid (E), and aspartic acid (D); or
X is a linker (γE)ₘ-(B)ₙ,
   wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
   m is 0, 1, 2, or 3;
   n is 0, 1, 2, or 3; and
   the sum of m and n is at least 1,
and Formula (II) is

   (polymer)ₐ-(Y)-; (II)
wherein a is 0 or 1 (preferably a is 1);
polymer is cellulose, poly(ethylene glycol) (PEG), methoxy poly(ethylene glycol) (MPEG), poly(lactic-co-glycolic acid), poly(N-vinyl pyrrolidone), or a derivative thereof;
Y is:
   a 1-10 amino acid residue or peptide sequence, wherein each amino acid residue is independently selected from lysine (K), arginine (R), glycine (G), alanine (A), glutamic acid (E), and aspartic acid (D);
   a non-amino acid linker comprising an ester, an amide, a thioether, an ether, a thioether, a carbamate moiety, or a combination thereof;
   an amino acid residue-containing linker, wherein the amino acid residue is covalently attached to (polymer)ₐ; or
   a peptide linker different from the 1-10 amino acid residue or peptide sequence.

In some embodiments, in the above Formula (II), Y is a linker (γE)ₘ-(B)ₙ, wherein B is a 1-8 amino acid residue or peptide sequence wherein each amino acid is independently selected from 2-[2-(2-aminoethoxy)ethoxy]acetic acid, Gly, Ala, Leu, Ser, Arg, and Lys; m is 0, 1, 2, or 3; n is 0, 1, 2, or 3; and the sum of m and n is at least 1.

In the present disclosure, lower case "x" refers to a natural or unnatural amino acid residue in the peptide sequence where it appears. Upper case X refers to a linker in Formula (I) and (II). In some embodiments, x is not a methionine residue (M), is not an asparagine residue (N), or is neither a methionine (M) nor an asparagine residue (N). In some embodiments, x is not any one of the 20 natural amino acid residues encoded by the mammalian genetic code, such as amino acids A, C, D, E, F, G, H, I, K, L, M, N, P, Q, R, S, T, V, W, and Y. In some embodiments, x is an unnatural amino acid residue (*i.e.,* an amino acid residue not encoded by the mammalian genetic code). In some embodiments, x is homoglutamine (also referred to herein as homoQ).

In some embodiments, the long acting CNP derivative or very long acting CNP derivative includes U-GLSKGCFGLKLDRIGSMSGLGC [SEQ ID NO. 2]; where U is attached to the N-terminal G of GLSKGCFGLKLDRIGSMSGLGC, and U is (aliphatic)ₐ-(X)-; wherein a is 1; aliphatic is an optionally substituted C₄₋₂₄ chain (*e.g.,* an optionally substituted C₁₀₋₂₄ chain, an optionally substituted C₁₀₋₁₈ chain, or an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; X is a 1-10 amino acid residue or peptide sequence, wherein each amino acid residue is independently selected from lysine (K), arginine (R), glycine (G), alanine (A), glutamic acid (E), and aspartic acid (D).

In some embodiments, the long acting CNP derivative or very long acting CNP derivative includes U-CFGLKLDRIGSxSGLGC [SEQ ID NO. 12] where x is a natural or unnatural amino acid residue and U has formula (aliphatic)ₐ-(X)- (Formula I); wherein 0 or 1 (preferably a is 1); aliphatic is an optionally substituted C₄₋₂₄ chain (*e.g.,* an optionally substituted C₁₀₋₂₄ chain, an optionally substituted C₁₀₋₁₈ chain, or an optionally substituted C₁₂₋₁₈ chain) covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the long acting CNP derivative or very long acting CNP derivative includes U-CFGLKLDRIGSxSGLGC [SEQ ID NO. 30] where x is a natural or unnatural amino acid residue, and provided that x is not M (methionine); U has formula (aliphatic)ₐ-(X)- (Formula I); wherein 0 or 1 (preferably a is 1); aliphatic is an optionally substituted C₄₋₂₄ chain (*e.g.,* an optionally substituted C₁₀₋₂₄ chain, an optionally substituted C₁₀₋₁₈ chain, or an optionally substituted C₁₂₋₁₈ chain) covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, x is not a methionine residue, is not an asparagine residue, or is neither a methionine nor an asparagine residue. In some embodiments, x is not any one of the 20 natural amino acid residues encoded by the mammalian genetic code, such as amino acids A, C, D, E, F, G, H, I, K, L, M, N, P, Q, R, S, T, V, W, and Y. In some embodiments, x is an unnatural amino acid residue (*i.e.,* an amino acid residue not encoded by the mammalian genetic code). In some embodiments, x is homoglutamine (also referred to herein as homoQ).

In some embodiments, the long acting CNP derivative or very long acting CNP derivative can include U-GLSKGCFGLKLDRIGSMSGLGC [SEQ ID NO. 2]; U-GLSKGCFGLK(U)LDRIGSMSGLGC [SEQ ID NO. 3]; GLSKGCFGLK(U)LDRIGSMSGLGC [SEQ ID NO. 4]; and/or U-CFGLKLDRIGSxSGLGC, where x is a natural or unnatural amino acid residue [SEQ ID NO. 12], or any combination thereof;
wherein:
U is a moiety of Formula (I), where Formula (I) is

   (aliphatic)ₐ-(X)-; (I)
wherein
   a is 0 or 1 (preferably a is 1);
   aliphatic is an optionally substituted C ₁₀₋₂₄ chain (*e.g.,* an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a 1-10 amino acid residue or peptide sequence, wherein each amino acid residue is independently selected from lysine (K), arginine (R), glycine (G), alanine (A), glutamic acid (E), and aspartic acid (D); or
   X is a linker (γE)ₘ-(B)ₙ,
   wherein B is a 1-8 amino acid residue or peptide sequence wherein each amino acid residue is independently selected from 2-[2-(2-aminoethoxy)ethoxy] acetic acid residue, Gly, Ala, Leu, Ser, Arg, and Lys;
   m is 0, 1, 2, or 3;
   n is 0, 1, 2, or 3; and
   the sum of m and n is at least 1.

In some embodiments, x in U-CFGLKLDRIGSxSGLGC [SEQ ID NO. 12] is not a methionine residue, is not an asparagine residue, or is neither a methionine nor an asparagine residue. In some embodiments, x is not any one of the 20 natural amino acid residues encoded by the mammalian genetic code, such as amino acids A, C, D, E, F, G, H, I, K, L, M, N, P, Q, R, S, T, V, W, and Y. In some embodiments, x is an unnatural amino acid residue (*i.e.,* an amino acid residue not encoded by the mammalian genetic code). In some embodiments, x is homoglutamine (also referred to herein as homoQ).

In some embodiments, X is a 4-7 amino acid residue or peptide sequence, wherein each amino acid residue is independently selected from lysine (K), arginine (R), and glycine (G).

In some embodiments, the long acting CNP derivative or the very long acting CNP derivative includes U-GLSKGCFGLKLDRIGSMSGLGC [SEQ ID NO. 2],
wherein:
U is (aliphatic)ₐ-(X)-;
wherein:
   a is 1;
   aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a 1-10 amino acid residue or peptide sequence, wherein each amino acid residue is independently selected from lysine (K), arginine (R), glycine (G), alanine (A), glutamic acid (E), and aspartic acid (D).

In some embodiments, the long acting CNP derivative or the very long acting CNP derivative includes U-CFGLKLDRIGSxSGLGC [SEQ ID NO. 13], x is homoglutamine; U is (aliphatic)ₐ-(X)-, wherein a is 0 or 1 (preferably a is 1), aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is Gly; m is 0, 1, or 2; and n is 1.

In some embodiments, the long acting CNP derivative or the very long acting CNP derivative includes U-CFGLKLDRIGSxSGLGC [SEQ ID NO. 14], x is homoglutamine; U is (aliphatic)ₐ-(X)-, wherein a is 0 or 1 (preferably a is 1), aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is Gly; m is 1; and n is 1.

In some embodiments, the long acting CNP derivative or the very long acting CNP derivative includes U-CFGLKLDRIGSxSGLGC [SEQ ID NO. 15], x is homoglutamine; U is (aliphatic)ₐ-(X)-, wherein a is 0 or 1 (preferably a is 1), aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; m is 1; and n is 0.

In some embodiments, the long acting CNP derivative or the very long acting CNP derivative includes U-CFGLKLDRIGSxSGLGC, where x is a natural or unnatural amino acid residue [SEQ ID NO. 12], wherein U is (aliphatic)ₐ-(X)-; a is 0 or 1 (preferably a is 1); aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X, or aliphatic is HOC(=O)(CH₂)₁₆C(=O)); X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1. In some embodiments, x in U-CFGLKLDRIGSxSGLGC [SEQ ID NO. 12] is not a methionine residue, is not an asparagine residue, or is neither a methionine nor an asparagine residue. In some embodiments, x is not any one of the 20 natural amino acid residues encoded by the mammalian genetic code, such as amino acids A, C, D, E, F, G, H, I, K, L, M, N, P, Q, R, S, T, V, W, and Y. In some embodiments, x is an unnatural amino acid residue (*i.e.,* an amino acid residue not encoded by the mammalian genetic code). In some embodiments, x is homoglutamine (also referred to herein as homoQ).

In some embodiments, the long acting CNP derivative or the very long acting CNP derivative includes U-CFGLKLDRIGSxSGLGC, where x is homoglutamine (homoQ) [SEQ ID NO. 16], U is (aliphatic)ₐ-(X)-; wherein a is 0 or 1 (preferably a is 1); aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 0, and n is 2.

In some embodiments, the long acting CNP derivative or the very long acting CNP derivative includes U-CFGLKLDRIGSxSGLGC, where x is homoglutamine (homoQ) [SEQ ID NO. 17], U is (aliphatic)ₐ-(X)-; wherein a is 0 or 1 (preferably a is 1); aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 1, and n is 2.

In some embodiments, the long acting CNP derivative or the very long acting CNP derivative includes U-CFGLKLDRIGSxSGLGC, where x is homoglutamine (homoQ) [SEQ ID NO. 18], U is (aliphatic)ₐ-(X)-; wherein a is 0 or 1 (preferably a is 1); aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is (2-[2-(2-aminoethoxy)ethoxy]acetic acid)-(2-[2-(2-aminoethoxy)ethoxy]acetic acid)-(Gly), m is 0, and n is 1.

In some embodiments, the long acting CNP derivative or the very long acting CNP derivative includes U-CFGLKLDRIGSxSGLGC, where x is homoglutamine (homoQ) [SEQ ID NO. 19], U is (aliphatic)ₐ-(X)-; wherein a is 0 or 1 (preferably a is 1); aliphatic is a branched or straight optionally substituted C₁₈ chain covalently bound to X via a carbonyl (*e.g.,* CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is (2-[2-(2-aminoethoxy)ethoxy]acetic acid)-(2-[2-(2-aminoethoxy)ethoxy]acetic acid)-(Gly), m is 1, and n is 1.

In some embodiments, the long acting CNP derivative or the very long acting CNP derivative U-CFGLKLDRIGSxSGLGC is HOC(=O)(CH₂)₁₆C(=O)-γE-Aeea-Aeea-GCFGLKLDRIGShomoQSGLGC with a disulfide bond between the cysteine residues (homoQ: homoGlutamine; Aeea: 2-[2-(2-aminoethoxy)ethoxy]acetic acid residue; HOC(=O)(CH₂)₁₆C(=O): octadecadioic acid reacted with γE so that a carbonyl (C(=O)) remains from one of the original octadecadioic acid carboxylic acid terminus; γE: glutamic acid conjugated through gamma-carboxy group [SEQ ID NO. 20].

In some embodiments, the long acting CNP derivative or the very long acting CNP derivative U-CFGLKLDRIGSxSGLGC is HOC(=O)(CH₂)₁₆C(=O)-Aeea-Aeea-GCFGLKLDRIGShomoQSGLGC with a disulfide bond between the cysteine residues (homoQ: homoGlutamine; Aeea: 2-[2-(2-aminoethoxy)ethoxy]acetic acid residue; HOC(=O)(CH₂)₁₆(CO): octadecadioic acid reacted with the amino terminus of Aeea so that a carbonyl (C(=O)) remains from the original octadecadioic acid carboxylic acid terminus; [SEQ ID NO. 21.].

In some embodiments, in any of the definitions herein, aliphatic does not include one or more of a straight or branched optionally substituted C₄₋₉ chain (*e.g.,* an optionally substituted C₃₋₈ chain-C(=O)- moiety, and/or an optionally substituted C₄₋₉ chain that is covalently bound to the peptide via a linkage such as a thioether, an ether, a thioether, a carbamate moiety, a bond, or the like). In certain embodiments, aliphatic is not a straight or branched C₈ chain (*e.g.,* a straight or branched C₈ chain covalently bound to the peptide via a linkage such as a carbonyl, thioether, an ether, a thioether, a carbamate moiety, a bond, or the like).

In some embodiments, U as described above includes CH₃(CH₂)₁₄C(=O)KKKKGGG- [SEQ ID NO. 22]; CH₃(CH₂)₁₆C(=O)KKKKGGG-[SEQ ID NO. 23]; CH₃(CH₂)₁₈C(=O)KKKKGGG- [SEQ ID NO. 24]; CH₃(CH₂)₂₀C(=O)KKKKGGG- [SEQ ID NO. 25]; or CH₃(CH₂)₂₂C(=O)KKKKGGG [SEQ ID NO. 26].

In some embodiments, the long acting CNP derivatives of the present disclosure includes CH₃(CH₂)₁₄C(=O)KKKKGGGGLSKGCFGLKLDRIGSMSGLGC [SEQ ID NO. 5]; CH₃(CH₂)₁₆C(=O)KKKKGGGGLSKGCFGLKLDRIGSMSGLGC [SEQ ID NO. 6]; CH₃(CH₂)₁₈C(=O)KKKKGGGGLSKGCFGLKLDRIGSMSGLGC [SEQ ID NO. 7]; CH₃(CH₂)₂₀C(=O)KKKKGGGGLSKGCFGLKLDRIGSMSGLGC [SEQ ID NO. 8]; CH₃(CH₂)₂₂C(=O)KKKKGGGGLSKGCFGLKLDRIGSMSGLGC [SEQ ID NO. 9]; HOC(=O)(CH₂)₁₆C(=O)-γE-Aeea-Aeea-GCFGLKLDRIGShomoQSGLGC including a disulfide bond between the cysteine residues [SEQ ID NO. 20]; and/or HOC(=O)(CH₂)₁₆C(=O)-Aeea-Aeea-GCFGLKLDRIGShomoQSGLGC including a disulfide bond between the cysteine residues [SEQ ID NO. 21].

In certain embodiment, the long acting CNP derivatives of the present disclosure includes CH₃(CH₂)₁₆C(=O)KKKKGGGGLSKGCFGLKLDRIGSMSGLGC [SEQ ID NO.6].

In some embodiments, the long acting CNP derivative or the very long acting CNP derivative includes U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof;
wherein U is a moiety of Formula (11), where Formula (II) is

   (polymer)ₐ-(Y)-; (II)
wherein a is 0 or 1 (preferably a is 1);
polymer is cellulose, poly(ethylene glycol) (PEG), methoxy poly(ethylene glycol) (MPEG), poly(lactic-co-glycolic acid), or poly(N-vinyl pyrrolidone);
Y is:
   a 4-10 amino acid residue or peptide sequence, wherein each amino acid residue is independently selected from lysine (K), arginine (R), and glycine (G);
   a non-amino acid linker comprising an ester, an amide, a thioether, an ether, a thioether, a carbamate moiety, or a combination thereof; or
   a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the long acting CNP derivative or the very long acting CNP derivative includes U-GLSKGCFGLKLDRIGSMSGLGC [SEQ ID NO. 2], U-GLSKGCFGLK(U)LDRIGSMSGLGC [SEQ ID NO. 3], or any combination thereof;
wherein U is a moiety of Formula (II), where Formula (II) is

   (polymer)ₐ-(Y)-; (II)
wherein a is 0 or 1 (preferably a is 1);
polymer is cellulose, poly(ethylene glycol) (PEG), methoxy poly(ethylene glycol) (MPEG), poly(lactic-co-glycolic acid), poly(N-vinyl pyrrolidone), or a derivative thereof;
Y is:
   a 1-10 amino acid residue or peptide sequence, wherein each amino acid residue is independently selected from lysine (K), arginine (R), glycine (G), alanine (A), glutamic acid (E), and aspartic acid (D);
   a non-amino acid linker comprising an ester, an amide, a thioether, an ether, a thioether, a carbamate moiety, or a combination thereof;
   an amino acid residue-containing linker, wherein the amino acid residue is covalently attached to (polymer)ₐ;
   a peptide linker different from the 1-10 amino acid residue or peptide sequence.

In some embodiments, Y in Formula (II) above is a linker -(γE)ₘ-(Bₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the polymer does not include poly(ethylene glycol), MPEG, or both poly(ethylene glycol) and MPEG.

In some embodiments, Y is a 4-10 amino acid residue or peptide sequence, wherein each amino acid residue is independently selected from lysine (K), arginine (R), and glycine (G); or a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, Y is a 4-10 amino acid residue sequence, wherein each amino acid residue is independently selected from lysine (K), arginine (R), and glycine (G).

In some embodiments, Y is a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the CNP or derivatives thereof of the present disclosure does not include CNP that is modified with polyalkylene glycol at the lysine residues at positions 4 and 10 of SEQ ID NO. 10 and/or at the N-terminus of the CNP of SEQ ID NO. 10.

In some embodiments, the formulations including long acting CNP derivatives of the present disclosure includes one or more CNP or derivatives thereof formulated with a polymer excipient including a poly(amino acid) grafted with polyethylene glycol, fatty acid, and/or anionic moieties. The polymer is adapted to sequester or non-covalently bind to the CNP derivative(s).

In some embodiments, the formulations including very long acting CNP derivatives of the present disclosure includes one or more long acting CNP derivatives formulated with a polymer excipient including a poly(amino acid) grafted with polyethylene glycol, fatty acid, and/or anionic moieties. The polymer is adapted to sequester or non-covalently bind to the CNP derivative(s).

In some embodiments, the formulations including long acting NPRB agonist(s) of the present disclosure includes one or more CNP or derivatives thereof formulated with a polymer excipient including a poly(amino acid) grafted with polyethylene glycol, fatty acid, and/or anionic moieties. The polymer is adapted to sequester or non-covalently bind to the NPRB agonist(s).

In some embodiments, the formulations including very long acting NPRB agonist of the present disclosure includes one or more long acting CNP derivatives formulated with a polymer excipient including a poly(amino acid) grafted with polyethylene glycol, fatty acid, and/or anionic moieties. The polymer is adapted to sequester or non-covalently bind to the NPRB agonist(s).

The poly(amino acid) that is grafted with polyethylene glycol, fatty acid, and/or anionic moieties can include a poly(amino acid) which may have D- or L-chirality or both and is a straight chain homopolymer. In one specific embodiment, straight chain homopolymers include polylysine, polyomithine, polyarginine, polyglutamate, polyaspartate, polyserine, polytyrosine, or any other amide linked homopolymer made from amino acids. In another preferred embodiment, straight chain hydrophobic homopolymers comprise polyalanine, polyvaline, polyleucine, polyisoleucine, polyglycine, or polyphenylalanine. In some embodiments, the poly(amino acid) is polylysine.

### Methods of making the active pharmaceutical ingredients

The peptides of the present disclosure, such as the long acting CNP, long acting CNP derivative, and long acting NPRB agonist can be synthesized by solid phase peptide synthesis (SPPS) using methods known to a person of ordinary skill in the art. For example, a starting solid support, such as H-Cys(Trt)-2-Cl-Trt Resin (BLDPharm, Shanghai, China) could be used in a peptide synthesizer, such as an automated microwave peptide synthesizer (*e.g.,* LibertyBlue HT12, CEM, Matthews, NC). Each amino acid, fatty acid, or protected alkyl carboxylic (di)acid can be anchored sequentially onto the peptide resin using Fmoc chemistry, known to those of ordinary skill in the art, resulting in a linear protected peptide linked to the resin. Linear crude peptide can be deprotected and released from the resin by acidolysis with trifluoroacetic acid in the presence of carbocation scavengers and ether precipitation. The resulting linear peptide can be cyclized, for example, by dissolving in DMSO and acetonitrile aqueous solution and reacted to form disulfide bond. Finally, the peptide can be purified and characterized by reversed phase HPLC (*e.g.,* 1260 Infinity II Preparative LC Systems, Santa Clara, CA). Fractions with >90% purity of the final peptide product can be collected and dried as white powder.

In some embodiments, the formulations including the active pharmaceutical ingredient(s) ("APIs") of the present disclosure has a weight ratio of a polymer excipient relative to APIs such that the resulting mixture is a long acting, or very long acting. For example, the weight ratio of the polymer excipient to total API can be from 5:1 to 100:1, 10:1 to 50:1 or 20:1 to 5:1. The polymer excipient is adapted to sequester or non-covalently bind to the APIs. Examples of polymer excipients are described, for example, in Castillo et al., Pharm. Res., (2012) 29(1); p 306-18; Castillo et al., PLoS One, (2017) 12(2); e0171703; US patent nos. 10,507,248; 10,035,885; and 10,010,613, each of which is herein incorporated by reference in its entirety. The polymer excipient can be, but is not limited to polylysine grafted with PEG at the epsilon amino to a level between 10-55% (*e.g*., 10-35%, or 30-55%) of total epsilon amino and the remainder amino groups grafted with alkyl group and/or anionic moieties such as sulfate, sulfonate, carboxyl, phosphate, or phosphonate. Methods of making polymer excipients are known in the art.

Briefly, in some embodiments, the polymer excipient is a polymer made by the following procedure. Poly-L-lysine (20PL), hydrobromide (21 µmol or 1 g; Sigma, Average Mw=26 kDa; d.p.126) was dissolved and the amount of NH₂-groups determined by TNBS titration. Methoxy polyethylene glycol carboxymethyl (MPEG-CM; 10 g; Mw=5 kDa; 2 mmol; Laysan Bio) was coupled to the polylysine using NHSS and EDC to provide the polymer excipient intermediate. The percent amino groups remaining was determined by TNBS. The hydrodynamic diameter was determined by size exclusion chromatography. The crude product can be lyophilized. Stearyl-NHS (C18-NHS) was prepared by activating stearic acid with NHS. DCC coupling of stearyl-NHS to the polymer excipient intermediate can be conducted. Excess reagents and side products can be removed standard techniques. Additional C18-NHS (3.6 mmol) was added and allowed to react with the polymer intermediate overnight. The reaction mixture was concentrated by rotary evaporation under vacuum to remove volatile components until an oil is isolated. The oil can be dissolved in alcohol and water. The solution can be filtered, washed repeatedly to provide a retentate containing the polymer excipient (polylysine with C18 hydrophobic side chains and MPEG hydrophilic side chains) was collected, 0.2 um filtered (polysulfone filter, Nalgene, Rochester, NY) and lyophilized, to provide the dry polymer excipient.

While a polymer excipient having C₁₈ hydrophobic side chains is described above, it is understood that other hydrophobic side chain lengths (*e.g.,* C₁₀₋₂₄, C₁₂₋₂₀, C₁₂₋₁₈, C₁₄₋₁₈, C₁₆₋₁₈, or C₁₈) and hydrophilic side chains (*e.g.,* PEG, mPEG) can be adapted to make polymer excipients having other hydrophobic side chains and hydrophilic side chains.

The poly(amino acid) that is grafted with polyethylene glycol, fatty acid, and/or anionic moieties can include a poly(amino acid) which may have D- or L-chirality or both and is a straight chain homopolymer. In one specific embodiment, straight chain homopolymers include polylysine, polyomithine, polyarginine, polyglutamate, polyaspartate, polyserine, polytyrosine, or any other amide linked homopolymer made from amino acids. In another preferred embodiment, straight chain hydrophobic homopolymers comprise polyalanine, polyvaline, polyleucine, polyisoleucine, polyglycine, or polyphenylalanine. In some embodiments, the poly(amino acid) is polylysine.

Examples of hydrophilic side chains include poly(ethylene glycol), which may be esterified by dicarboxylic acid to form a poly(ethylene glycol) monoester; methoxy poly(ethylene glycol) monoester (MPEG) or a co-polymer of poly(ethylene glycol) and poly(propylene glycol) monoester in a form of an ester with a dicarboxylic acid giving the terminal of this co-polymers a carboxyl group that can be used to covalently link it to a poly(amino acid). Other forms include poly(ethylene glycol)-carboxyl; methoxy poly(ethylene glycol)-carboxyl; poly(ethylene glycol)-carboxymethyl; methoxy poly(ethylene glycol)-carboxymethyl; poly(ethylene glycol) monoamine; methoxy poly(ethylene glycol) monoamine; poly(ethylene glycol) hydrazide; methoxy poly(ethylene glycol) hydrazide; methoxy poly(ethylene glycol) imidazolide block-co-polymer of poly (ethylene glycol) and one or several polymers represented by polyaminoacid, polysaccharide, polyamidoamine, polyethyleneimine where these blocks are alternated to give a linear block-co-polymer. In one embodiment, the overall molecular weight of a protective chain may be larger than 300 Daltons but not exceeding 10,000 Daltons. In one embodiment, one or more protective chains are linked to the poly(amino acid) backbone by a single linkage.

Without wishing to be bound by theory, it is believed that the higher the weight ratio of polymer excipient to APIs, the more sustained the presence in the plasma and the more sustained the plasma cyclic-GMP elevation over the baseline, when the API composition is administered to a subject.

In some embodiments, the formulations including the long acting CNP, long acting CNP derivative, and/or the long acting NPRB agonist of the present disclosure has a weight ratio of a polymer excipient relative to CNP, CNP derivative, and/or NPRB agonist such that the resulting mixture is a long acting CNP, long acting CNP derivative, and/or long acting NPRB agonist. For example, the weight ratio of the polymer excipient to CNP, CNP derivative, and/or the NPRB agonist can be from 5:1 to 100:1, 10:1 to 50:1 or 20:1 to 5:1. The polymer excipient can include a poly(amino acid) grafted with polyethylene glycol, fatty acid, and/or anionic moieties. *See, e.g.,* Castillo et al., Pharm. Res., (2012) 29(1); p 306-18; Castillo et al., PLoS One, (2017) 12(2); e0171703; US patent nos. 10,507,248; 10,035,885; 10,010,613, each of which is herein incorporated by reference in its entirety. The polymer excipient is adapted to sequester or non-covalently bind to the CNP, CNP derivative, and/or the NPRB agonist. The polymer excipient can be, but is not limited to polylysine grafted with PEG at the epsilon amino to a level between 30-55% or 10-35% of total epsilon amino and the remainder amino groups grafted with alkyl group and/or anionic moieties such as sulfate, sulfonate, carboxyl, phosphate, or phosphonate. Methods of making polymer excipients are known in the art. Without wishing to be bound by theory, it is believed that the higher the weight ratio of polymer excipient to CNP , CNP derivative, and/or the NPRB agonist, the more sustained the CNP, CNP derivative, or NPRB agonist presence in the plasma and the more sustained the plasma cyclic-GMP elevation over the baseline, when the CNP, CNP derivative, and/or NPRB agonist composition is administered to a subject.

In some embodiments, the very long acting CNP, very long acting CNP derivative, and/or very long acting NPRB agonist formulations include CNP, CNP derivative, and/or NPRB agonist and a polymer excipient at a weight ratio of polymer excipient relative to CNP, CNP derivative, and/or NPRB agonist such that the resulting mixture is a very long acting CNP, a very long acting CNP derivative, and/or a very long acting NPRB agonist. For example, the weight ratio of the polymer excipient to CNP, CNP derivative, and/or NPRB agonist can be from 5:1 to 100:1, 10:1 to 50:1 or 20:1 to 5:1. The polymer excipient can include a poly(amino acid) grafted with polyethylene glycol, fatty acid, and/or anionic moieties. *See, e.g.,* Castillo et al., Pharm. Res., (2012) 29(1); p 306-18; Castillo et al., PLoS One, (2017) 12(2); e0171703; US patent nos. 10,507,248; 10,035,885; 10,010,613 each of which is herein incorporated by reference in its entirety. The polymer excipient is adapted to sequester or non-covalently bind to the CNP, CNP derivative, and/or NPRB agonist. The polymer excipient can be, but is not limited to polylysine grafted with PEG at the epsilon amino to a level between 30-55% or 10-35% of total epsilon amino and the remainder amino groups grafted with alkyl group and/or anionic moieties such as sulfate, sulfonate, carboxyl, phosphate, or phosphonate. Methods of making polymer excipients are known in the art. Without wishing to be bound by theory, it is believed that the higher the weight ratio of polymer excipient to CNP, CNP derivative, and/or NPRB agonist, the more sustained the CNP, CNP derivative, and/or NPRB agonist presence in the plasma and the more sustained the plasma cyclic-GMP elevation over the baseline, when the CNP, CNP derivative, and/or NPRB agonist composition is administered to a subject.

### Combination cancer treatment

In further embodiment of the present disclosure, any one of the above methods for treating cancer includes further administering immune checkpoint inhibitor(s). The immune checkpoint inhibitor(s) can include antibody(ies)/protein(s) or compounds that binds and/or block the action of any one of CTLA-4 (cytotoxic T-lymphocyte-associated protein 4), PD1 (programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, is an immune checkpoint member of the B7 and CD28 families), B7H4 (a molecule of the B7 family, negatively regulates T cell immunity), and/or TIM-3 (a co-inhibitory receptor that is expressed on IFN-γ-producing T cells). The therapeutically effective bolus dose of a composition including a long acting C-type natriuretic peptide (CNP), CNP derivative, long acting CNP derivative, long acting CNP receptor (NPRB) agonist, or any combination thereof can be administered prior to, concurrently with, or subsequent to the immune checkpoint inhibitor(s).

In some embodiments, any one of the methods for treating cancer of the present disclosure further includes administering CAR-T cells. The therapeutically effective bolus dose of a composition including a long acting C-type natriuretic peptide (CNP), CNP derivative, long acting CNP derivative, long acting CNP receptor (NPRB) agonist, or any combination thereof; and/or the immune checkpoint inhibitor(s), can be administered prior to, concurrently with, or subsequent to intravenous administration of the CAR-T cells.

In some embodiments, any one of the methods for treating cancer of the present disclosure further includes administering antibodies. For example, the antibody can be an anti-PD1 antibody). In some embodiments, a synergistic effect can be observed between a composition including a long acting C-type natriuretic peptide (CNP), CNP derivative, long acting CNP derivative, long acting CNP receptor (NPRB) agonist, or any combination thereof, together with the antibody.

In some embodiments, any one of the methods for treating cancer of the present disclosure further includes administering one or more adjuvants, such as CpG oligodeoxynucleotides (ODN), a Toll-like receptor 9 agonist; or a CpG oligodeoxynucleotide. In some embodiments, a synergistic effect can be observed between a composition including a long acting C-type natriuretic peptide (CNP), CNP derivative, long acting CNP derivative, long acting CNP receptor (NPRB) agonist, or any combination thereof, together with the adjuvant.

In some embodiments, the present disclosure features a method of treating a subject (*e.g.,* a mammalian subject, a patient in need thereof) with one or more of cancers in the following organs: skin, breast, bone, prostate, colon, head, neck, liver, kidney, cervix, lung, stomach, urethra, bladder, ureters, renal pelvis, rectum, esophagus, lymph node, pancreas, stomach, ovary, central nervous system, soft tissue, and/or endocrine glands. The method includes administering to the subject a therapeutically effective bolus dose of a composition (*e.g.,* cytotoxic cell immunostimulant composition) including a long acting CNP, a very long acting CNP, a long acting CNP derivative, a very long acting CNP derivative, a long acting NPRB agonist, and/or a very long acting NPRB agonist. The method further includes administering an immune checkpoint inhibitor such as one or more antibody(ies)/protein(s) or compounds that binds and/or block the action of anyone of CTLA-4 (cytotoxic T-lymphocyte-associated protein 4), PD1 (Programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, is an immune checkpoint member of the B7 and CD28 families), B7H4 (a molecule of the B7 family, negatively regulates T cell immunity), and/or TIM-3 (a co-inhibitory receptor that is expressed on IFN-γ-producing T cells). In some embodiments, the method further includes administering to the subject an immune adjuvant, wherein the immune adjuvant modulates a toll-like receptor, or a cytotoxic cell immunostimulant including therapeutic agents (*e.g.,* protein and/or small molecule compounds) or antibodies targeting an immune checkpoint protein selected from CTLA-4 (cytotoxic T-lymphocyte-associated protein 4) , PD1 (programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, an immune checkpoint member of the B7 and CD28 families), B7H4 (a molecule of the B7 family, negatively regulates T cell immunity), and TIM-3 (a co-inhibitory receptor that is expressed on IFN-γ-producing T cells); wherein the cytotoxic cell immunostimulant inhibits an immune checkpoint protein; or further administering a cytotoxic cell immunostimulant including an antibody or portion of an antibody against an immune checkpoint protein, a soluble ligand of an immune checkpoint protein, pembrolizumab, Nivolumab, Ipilimumab, Atezolizumab, Avelumab, Durvalumab, Cemiplimab, tremelimumab, lambrolizumab, and/or pidilizumab; or further administering an immune adjuvant that is a toll-like receptor 9 agonist; or further administering an immune that is a CpG oligodeoxynucleotide. The therapeutically effective bolus dose of the composition is a dose that does not decrease or cause a decrease in blood pressure (or mean arterial pressure) by more than 20% (*e.g.,* by more than 15%, by more than 10%, or by more than 5%) of a baseline blood pressure measurement, where the baseline blood pressure measurement is an average blood pressure prior to administration of the composition, but the dose can increase plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the subject (*e.g.,* a mammalian subject, a patient in need thereof) with cancer has one or more of cancers in the following organs: skin, breast, bone, prostate, colon, head, neck, liver, kidney, cervix, lung, stomach, urethra, bladder, ureters, renal pelvis, rectum, esophagus, lymph node, pancreas, stomach, ovary, central nervous system, soft tissue, and/or endocrine glands. The methods include administering to the subject a therapeutically effective bolus dose of a composition (*e.g.,* a cytotoxic cell immunostimulant composition) including a long acting CNP, a very long acting CNP, a long acting CNP derivative, a very long acting CNP derivative, a long acting NPRB agonist, and/or a very long acting NPRB agonist. In some embodiments, the method further includes administering an antibody or portion of an antibody against an immune checkpoint protein and/or a soluble ligand of an immune checkpoint protein. For example, the method can further include administering one or more immune checkpoint protein antibodies including pembrolizumab, nivolumab, ipilimumab, atezolizumab, avelumab, durvalumab, cemiplimab, tremelimumab, lambrolizumab, and/or pidilizumab. The therapeutically effective bolus dose is a dose that does not decrease or cause a decrease in blood pressure (or mean arterial pressure) by more than 20% (*e.g.,* by more than 15%, by more than 10%, or by more than 5%) of a baseline blood pressure measurement, where the baseline blood pressure measurement is an average blood pressure prior to administration of the composition, but the dose can increase plasma cyclic-GMP level at from 1 hour to 12 hours after administration (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, 12 to 168 hours) to above 1.5x (*e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the subject (*e.g.,* a mammalian subject, a patient in need thereof) with cancer has one or more of cancers in the following organs: skin, breast, bone, prostate, colon, head, neck, liver, kidney, cervix, lung, stomach, urethra, bladder, ureters, renal pelvis, rectum, esophagus, lymph node, pancreas, ovary, central nervous system, soft tissue, and/or endocrine glands. The method includes administering to the subject a therapeutically effective bolus dose of cytotoxic cell immunostimulant composition including a long acting CNP derivative. The method further includes administering immune checkpoint inhibitor selected from one or more antibody(ies)/protein(s) or compounds that binds and/or block the action of anyone of CTLA-4 (cytotoxic T-lymphocyte-associated protein 4), PD1 (Programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, is an immune checkpoint member of the B7 and CD28 families), B7H4 (a molecule of the B7 family, negatively regulates T cell immunity), TIM-3 (a co-inhibitory receptor that is expressed on IFN-γ-producing T cells). The therapeutically effective bolus dose is a dose as described above.

In some embodiments, the subject (*e.g.,* a mammalian subject, a patient in need thereof) with cancer has one or more of cancers in the following organs: skin, breast, bone, prostate, colon, head, neck, liver, kidney, cervix, lung, stomach, urethra, bladder, ureters, renal pelvis, rectum, esophagus, lymph node, pancreas, stomach, ovary, central nervous system, soft tissue, and/or endocrine glands. The methods include administering to the subject a therapeutically effective bolus dose of cytotoxic cell immunostimulant composition including a long acting CNP derivative. In some embodiments, the method further includes administering an antibody or portion of an antibody against an immune checkpoint protein, and/or a soluble ligand of an immune checkpoint protein. For example, the method can further include administering one or more immune checkpoint protein antibodies including pembrolizumab, nivolumab, ipilimumab, atezolizumab, avelumab, durvalumab, cemiplimab, tremelimumab, lambrolizumab, and/or pidilizumab. The therapeutically effective bolus dose is a dose as described above.

In some embodiments, the subject (*e.g.,* a mammalian subject, a patient in need thereof) with cancer has one or more of cancers in the following organs: skin, breast, bone, prostate, and/or colon. The methods include administering to the subject a therapeutically effective bolus dose of cytotoxic cell immunostimulant composition including a long acting CNP, a very long acting CNP, a long acting CNP derivative, a very long acting CNP derivative, a long acting NPRB agonist, and/or a very long acting NPRB agonist. The method further includes administering immune checkpoint inhibitor selected from one or more antibody(ies)/protein(s) or compounds that binds and/or block the action of anyone of CTLA-4 (cytotoxic T-lymphocyte-associated protein 4), PD1 (programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, is an immune checkpoint member of the B7 and CD28 families), B7H4 (a molecule of the B7 family, negatively regulates T cell immunity), and/or TIM-3 (a co-inhibitory receptor that is expressed on IFN-γ-producing T cells). The therapeutically effective bolus dose is a dose as described above.

In some embodiments, the subject (*e.g.,* a mammalian subject, a patient in need thereof) with cancer has one or more of cancers in the following organs: skin, breast, bone, prostate, and/or colon. The methods include administering to the subject a therapeutically effective bolus dose of cytotoxic cell immunostimulant composition including a long acting CNP, a very long acting CNP, a long acting CNP derivative, a very long acting CNP derivative, a long acting NPRB agonist, and/or a very long acting NPRB agonist. In some embodiments, the methods further include administering an antibody or portion of an antibody against an immune checkpoint protein, and/or a soluble ligand of an immune checkpoint protein. For example, the method can further include administering one or more immune checkpoint protein antibodies including pembrolizumab, nivolumab, ipilimumab, atezolizumab, avelumab, durvalumab, cemiplimab, tremelimumab, lambrolizumab, and/or pidilizumab. The therapeutically effective bolus dose is a dose as described above.

In any of the above mentioned methods, the skin cancer can include Merkel cell carcinoma, squamous cell carcinoma, and/or melanoma; the liver cancer can include hepatocellular carcinoma; the kidney cancer can include renal cell carcinoma; the lung cancer can include small cell or non-small cell lung carcinoma; the breast cancer can include triple negative breast cancer; the stomach cancer can include gastric cancer adenocarcinoma of esophageal junction, and/or dMMR; the lymph node can include Hodgkin or non-Hodgkin PMBCL; the pancreatic cancer and/or ovarian cancer can each include dMMR; and/or the cancer of the surrounding organs to the renal pelvic area can include urothelial cancer.

The following examples are provided to illustrate, not limit, the disclosure.

### EXAMPLES

All peptides used in the Examples were synthesized by solid phase peptide synthesis (SPPS) with H-Cys(Trt)-2-Cl-Trt Resin (0.54 mmol/g) as the starting solid support (BLDPharm, Shanghai, China) in an automated microwave peptide synthesizer (LibertyBlue HT12, CEM, Matthews, NC). Each constituent molecule of the peptide, such as amino acid, fatty acid, or protected alkyl dioic acid were anchored sequentially onto the peptide resin using Fmoc chemistry, which is known to a person of ordinary skill in the art, resulting in a linear protected peptide linked to the resin. Linear crude peptide was deprotected and released from the resin by acidolysis with trifluoroacetic acid in the presence of carbocation scavengers and ether precipitation. The resulting linear peptide was cyclized by dissolving in 10% DMSO and 20% acetonitrile aqueous solution and allowed to react for at least two days to provide disulfide bond formation. Finally, the peptide was purified and characterized by reversed phase HPLC (1260 Infinity II Preparative LC Systems, Santa Clara, CA) using a gradient of 10% acetonitrile in water with 0.1% trifluoroacetic acid (TFA) and acetonitrile with 0.1% TFA. This gradient was run on a Waters 30 x 150mm XBridge C18 column (P/N 186003284) with a Waters C18 prep column (P/N 186006893) at 40 mL/min over 24 minutes at room temperature and was acquired at 214 nm. The peptide fractions with >90% purity were collected and dried as white powder to provide the final peptide product.

### Example 1: Superior in vivo performance of long acting CNP compared to native CNP when administered as a bolus

All mice used for this study were maintained under a 12-hour light/12-hour dark cycle with free access to water and standard mouse diet (MF diet, Oriental Yeast Co., Ltd. Tokyo, Japan or PicoLab Rodent Diet 20, LabDiet Corp., St. Louis, Missouri).

For the pharmacokinetic study, female CD-1 mice (6-8 weeks old from Charles river laboratory) were treated with 2.0 mg/Kg of native human CNP (Chempep Inc. Wellington, FL), long acting CNP derivative (dCNP, Chempep Inc. Wellington, FL), or very long acting CNP derivative (VLA-dCNP) via subcutaneous administration between the shoulder blades. All test articles were formulated or dissolved in 100 mM sorbitol, 100 mM methionine, 20 mM histidine, pH 6.0. Blood sampling at various times (0, 0.5, 1, 2, 3, 4, 5, and 24 for native CNP; 0, 1, 2, 4, 8, 12, 24, 48, and 72 for dCNP and VLA-dCNP) was performed by retro-orbital bleed, two bleeding per animal at two different timepoints. Blood samples were processed in K₂EDTA tubes to obtain plasma. Plasma was analyzed by commercially available CNP ELISA kit from Phoenix Pharmaceuticals (cat# EKE-012-03). CNP is a native human CNP (GLSKGCFGLKLDRIGSMSGLGC [SEQ ID NO. 10]) and dCNP is one of the addition derivatives of human CNP with the following sequence: CH₃(CH₂)₁₆C(=O)KKKKGGGGLSKGCFGLKLDRIGSMSGLGC [SEQ ID NO. 6]. The VLA-dCNP is a co-formulation of dCNP with PK extending polymer excipient at a dCNP: excipient weight ratio of 1:10. The details of the polymer are described in Castillo et al., Pharm. Res., (2012) 29(1); p 306-18, herein incorporated by reference in its entirety.

For pharmacodynamic study of cyclic-GMP response study, male C57BL/6J mice (6-week old from Kyudo; Saga, Japan) were treated with 1.0 mg/Kg of native human CNP, long acting CNP derivative (dCNP), and very long acting CNP derivative (VLA-dCNP) via subcutaneous bolus administration between the shoulder blades. All test articles were formulated or dissolved in 100 mM sucrose, 100 mM methionine, 50 mM histidine, pH 7.4. Blood sampling at various times (0, 1, 4, 8, 12, and 24 h for native CNP and dCNP; 0, 1, 2, 4, 8, 5, 24, and 48 h for dCNP and VLA-dCNP) was performed by abdominal aorta blood sampling after laparotomy, one bleeding per animal per timepoint. To obtain plasma, EDTA; final concentration 1.5 mg/mL (Dojindo, Kumamoto, Japan) and aprotinin; final concentration 500KIU/mL (Sigma Aldrich, St. Louis MO) were added to blood and centrifuged (x 2,000 g; 15 min, 4C). After supernatant was harvested, plasma samples were stored at -80 °C. Plasma samples were analyzed by commercially available cyclic-GMP kit from CisBio (Codolet, France). CNP is a native human CNP (GLSKGCFGLKLDRIGSMSGLGC [SEQ ID NO. 10]) and dCNP is an addition derivative of human CNP with the following sequence: CH₃(CH₂)₁₆(C=O)KKKKGGGGLSKGCFGLKLDRIGSMSGLGC [SEQ ID NO. 6]. The VLA-dCNP is a co-formulation of dCNP with PK extending excipient at a dCNP: excipient weight ratio of 1:10. The details of the polymer are described in Castillo et al., Pharm. Res., (2012) 29(1); p 306-18, herein incorporated by reference in its entirety. Specifically, the PK extending excipient was made using N-hydroxy sulfo-succinimide reagent and 1-Ethyl-3-(3-dimethylaminopropyl)carbodiimide to activate carboxyl group of polyetheylene glycol (PEG) for attachment to epsilon amino of linear polylysine ( at Epsilon amino: NHSS: EDC: PEG carboxyl group molar ratio of 0.2: 1: 1: 0.3) of 5kDa polyetheylene glycol (PEG) attached them to the epsilon amino group of the linear polylysine backbone with molecular weight ranging from 15 to 40kDa (polylysine average molecular weight of 25kDa, by multi-angle laser light scattering or MALLS). The product was characterized by trinitrobenzine sulfonic acid (TNBS) amino in process measurement. It is estimated to have 55% of epsilon amino group used up during the PEG addition reaction and the remaining epsilon amino groups was used up during the stearic acid addition reaction using NHS-stearic acid. Only trace amount of measurable amino groups (<5%) present at the end of the stearic acid addition as measured by TNBS. The PK extending excipient was purified by an ultrafiltration process that is well-known to those skilled in the art. Buffer formulation used for bolus administrations with and without PK extending excipient was 100 mM sucrose, 100 mM methionine, 50 mM histidine.

Referring to FIGURE 1A, the plasma CNP [mean (SD); n= 5] is shown for CD-1 mice after subcutaneous administration in an amount of 2.0 mg/Kg of a native CNP, a CNP derivative (dCNP), and a very long acting CNP derivative (VLA-dCNP). The inset is an enlarged scale of the left bottom corner to show the low plasma level of CNP (diamond) when native CNP is administered. Error bars represent standard deviation of n=5 plasma samples. Baseline CNP level prior to administration was 1.74 (0.6) ng/mL [mean (SD); n=15]. FIGURE 1B is a plot showing plasma cyclic-GMP in male C57BL/6J mice measured using a cyclic-GMP kit from CisBio (Codolet, France) after subcutaneous administration in an amount of 1.0mg/Kg of native CNP, long acting CNP derivative (dCNP), and very long acting CNP derivative (VLA-dCNP). Baseline plasma cyclic-GMP level was 20 (3.7) pmol/mL [mean (SEM); n=8] or 7 (1.3) ng/mL [mean (SEM); n=8]. At 2 hours and beyond, subcutaneous administration of native CNP did not show significant elevation of plasma cyclic-GMP compared to the baseline, while similar administration of long acting CNPs (dCNP and VLA-dCNP) showed significant elevation of cyclic-GMP for at least 24 hours.

### Example 2: Bolus administration of high dose of very long acting CNP derivative (VLA-dCNP) can increase plasma cyclic-GMP in the surprising absence of corresponding drop in blood pressure

For this study the cardiovascular and hemodynamic effects were assessed for three different long acting natriuretic peptides (very long acting ANP derivatives or VLA-dANP; ANP modified in a similar way as dCNP, where VLA-ANP was CH₃(CH₂)₁₆C(=O)KKKKGGG-SLRRSSCFGGRMDRIGAQSGLGCNSFRY [SEQ ID NO. 28] plus PK extending excipient and dANP was CH₃(CH₂)₁₆C(=O)KKKKGGG-SLRRSSCFGGRMDRIGAQSGLGCNSFRY [SEQ ID NO. 28] alone. The PK extending excipient was a polymeric excipient described in Example 1 above, and in Castillo et al., Pharm. Res., (2012) 29(1); p 306-18, and herein incorporated by reference in its entirety. The very long action BNP derivatives or VLA-DBNP was CH₃(CH₂)₁₆C(=O)KKKKGGG-SPKMVQGSGCFGRKMDRISSSSGLGCKVLRRH (dBNP) [SEQ ID NO. 29] plus PK extending excipient described above. dBNP, CH₃(CH₂)₁₆C(=O)KKKKGGG-SPKMVQGSGCFGRKMDRISSSSGLGCKVLRRH [SEQ ID NO. 29] is without the PK extending excipient described above. VLA-dCNP is dCNP as described in Example 1 plus PK extending polymeric excipient described above. dCNP is as described in Example 1 and is without the PK extending excipient. These formulations (in 100 mM Sucrose, 100 mM methionine, 50 mM histidine buffer) were administered to Beagle dogs [n=12 animals/test article; the same animals were for other test articles after a washout period of at least a week]. These test articles were administered by a single subcutaneous injection containing 25 µg/Kg of peptide and 1 mg/Kg of PK extending polymer (2.5% loading). 12 animals were previously instrumented with Data Sciences International (St. Paul, MN) telemetry transmitters to continuously record heart rate, mean arterial pressure, systolic arterial pressure, diastolic arterial pressure, PR interval, QRS duration, QT interval and body temperature. All animals were monitored for 7 days after each dose. At 4, 6, 8, 16, 20, 24, 28, 32, 40, 48, 66, 78, 90, 102, 114, 126, 138, 150, 162, and 174 hours after each dose, a 3mL blood sample was taken in a K₃ EDTA collection tube and then stored on wet ice until spun in a refrigerated centrifuge. Plasma was harvested and treated with plasma preservation reagent (phosphoric acid in deionized water, 15:85, v/v). The samples were inverted several times and then frozen on dry ice. The samples were stored in a freezer (-80C) then shipped on dry ice for LC-MS analysis of cyclic-GMP.

All natriuretic peptides act by causing an increase in cytoplasmic cyclic-GMP generation which is believed to cause a corresponding drop in blood pressure. However, referring to FIGURES 2A and 2B, when the bolus doses of very long acting versions of 3 main natriuretic peptides were compared, it was surprisingly found that a high bolus dose (sufficient to increase blood cyclic-GMP for 3 days) of very long acting CNP derivative of the present disclosure could increase in plasma cyclic-GMP without causing a dangerous drop in blood pressure; while a similarly developed very long acting ANP and BNP derivative, when given as a bolus dose (enough to increase blood cyclic-GMP for 3 days), caused a significant drop in blood pressure. For the very long acting ANP derivative, the blood pressure drop was as much as 45%, while for the very long acting BNP derivative, the blood pressure drop was as much as 20%. For all 3 long acting natriuretic peptide derivatives, the increase in cyclic-GMP was more than 1.5-fold and as much as 6-fold the baseline. The cyclic-GMP AUC are VLA-dANP 3,483 ng*h/mL, VLA-dBNP 2,585 ng*h/mL, VLA-dCNP 2,627 ng*h/mL.

FIGURE 2A shows the corresponding increase in plasma cyclic-GMP [mean (SEM); n=12] as monitored after a bolus administration of 25ug/Kg of very long acting CNP derivative (VLA-dCNP), very long acting BNP derivative (VLA-dBNP), and very long acting BNP derivative (VLA-dANP). Baseline plasma cyclic-GMP level was 8 (0.2) ng/mL [mean (SEM); n=12], a level which is similar to healthy human. *See, e.g.,* Igaki, et al., Hypertens Res 1998; 21: 7-13. All very long acting formulations of natriuretic peptide increased cyclic-GMP above the baseline of 8ng/ml. The cyclic-GMP AUC values were VLA-dANP 3,483 ng*h/mL, VLA-dBNP 2,585 ng*h/mL, VLA-dCNP 2,627 ng*h/mL. The very long acting CNP derivative (VLA-dCNP) increased plasma cyclic-GMP for 3 days without an associated drop in blood pressure.

FIGURE 2B shows the mean arterial pressure in dogs [mean (SEM); n=12] as monitored after a bolus administration of 25ug/Kg of very long acting CNP derivative (VLA-dCNP), very long acting BNP derivative (VLA-dBNP), or very long acting BNP derivative (VLA-dANP). VLA-dCNP did not cause significant drop in blood pressure from baseline (0 hr) after administration at a very high dose. In comparison, other very long acting natriuretic peptides such as VLA-dBNP and VLA-dANP derivatives caused more than a 15% drop in blood pressure. This was especially true for VLA-dANP where a drop in blood pressure could be as much as 50% for similar increase in cyclic-GMP. The very long acting CNP derivative (VLA-dCNP) increased plasma cyclic-GMP for 3 days without an associated drop in blood pressure.

### Example 3: VLA-dCNP increased cluster of differentiation 8 positive (CD8+) T Cell in breast tumor indicating that VLA-dCNP facilitated entry and/or activation of tumor killing cells and/or suppression of immune checkpoint inhibition against tumor,

Cluster of differentiation 8 (CD8) is a transmembrane glycoprotein that serves as a co-receptor for the T cell receptor (TCR). Like the TCR, CD8 binds to a major histocompatibility complex (MHC) molecule but is specific for the MHC class I protein (*see, e.g.,* Gao G, Jakobsen B (2000). Immunol Today. 21 (12): 630-6, incorporated herein by reference in its entirety). CD8 is a marker for cytotoxic T cells, and the fact it is abundant in tumors means that there are a large number of T cells that can attack the tumor cells. Since DAPI (4',6-diamidino-2-phenylindole) stains the nucleus regardless of cell type, some cells that were not stained with CD8 which indicates that the specificity of CD8 is secured. This indicates not only the potential enhancement of immune checkpoint inhibitors but also the possibility of enhancing the effects of other immunotherapy such as Chimeric antigen receptor (CAR)-T cell therapy.

In this study, female C57BL/6J mice (6 weeks old, female, n = 4/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with mouse breast cancer cell line E0771 (250,000 cells/mouse, subcutaneous implantation to left mammary gland). From 4th day after implantation, mice were treated with a bolus dose of 0.3 mg/kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous injection under isoflurane anesthesia, 5 times/week). At 2 weeks, mice were sacrificed, and tumor tissues were harvested and frozen. Frozen sections of tumor samples were prepared. CD8 and cellular nucleus in tumor samples were immunohistochemically stained and detected with a fluorescence microscope (BZ-X700, Keyence, Tokyo, Japan). Referring to FIGURE 3A, the numbers of CD8-positive cells per fields were counted; error bars are SEM. FIGURE 3B shows the fluorescence images of CD8 and DAPI. Magnification was low field power x4 for all the images. Statistical analysis was performed by Student t- tests by using GraphPad Prism 6.0 (*n* =4). **P* < 0.05.

### Example 4: VLA-dCNP increased activated T cell in breast tumor indicating that VLA-dCNP facilitated entry and/or activation of tumor killing cells and/or suppression of immune checkpoint inhibition against tumor

FIGURE 4A is a bar graph showing the amount of CD8 cells in a control mouse group and a group treated with VLA-dCNP (described in Example 1), FIGURE 4B is a bar graph showing the amount of activated CD8 cells in a control mouse group and a group treated with VLA-dCNP, and FIGURE 4C is a bar graph showing the amount of activated NK cells in a control mouse group and a group treated with VLA-dCNP. Referring to FIGURES 4A-4B, cytotoxic T cells cluster of differentiation 3 positive/cluster of differentiation 8 positive (CD3+/CD8+), the center of tumor immunity, showed an increasing trend. In addition, activated cytotoxic T cells (CD3+/CD8+/Interferon gamma positive (IFNG+)) were significantly elevated. This indicates that VLA-dCNP increased the number of T cells and induced their activation. Similarly, referring to FIGURE 4C, it was shown that the proportion of activated natural killer cells (NK cells) (NK1.1), which is important in tumor immunity, was increased. NK cells do not need their target cells to have MHC Class I for target cell antigen to be recognized for destruction/killing (T cell cannot kill cells because MHC Class I is required in tumor cells for antigen recognition and killing). From these, it is believed that VLA-dCNP induced activation of NK cells as well as CD8 positive T cells means that there is only a small blind spot in its immune enhancing effect. Without wishing to be bound be theory, it is believed that CD8+ cells in mammary glands is about 0.2%-2.8% (the healthy animal value was 2.0 (0.2; SEM ranging from 1.6-2.6%), CD8+/IFNg+ in mammary glands is about 3-4% (the healthy animal value was 4.1 (0.14; SEM)), and activated NK in mammary glands is 0.01-0.05%.

Female C57BL/6J mice (6 weeks old, female, n=4/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with mouse breast cancer cell line E0771 (ATCC, Old Town Manassas, VA) (250,000 cells/mouse, subcutaneous implantation to left mammary gland). From 4th day after implantation, mice were treated with a bolus dose of 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone as control (subcutaneous injection under isoflurane anesthesia, 5 times/week). At 2 weeks, mice were sacrificed and tumor tissues were harvested and obtained single cells by using BD Horizon^{™} Dri Tissue & Tumor Dissociation Reagent (BD, Franklin Lakes NJ). Cells were stained by each antibody set and analyzed by flow cytometer (Verse, BD, Franklin Lakes, NJ); error bars are SEM. After measuring all particles (around 10,000) using a flow cytometer based on particle size (FSC) and structural disorder (SSC) to gate a population are cells, this population was set to 100. Then, CD8 (CD3+ and CD8+), Activated CD8 (CD3+, CD8+, and IFNg+), and Activated NK (NK1.1+ (CD161) and Perforin+) were measured and calculated out the total population and converted to percentage. In this Example, NK1.1 is a marker for Natural Killer cells, and Perforin is a protein that activates NK cells and kills target cells. When the two are considered together, the cells can be counted as activated NK cells. Perforin alone does not tell if it is an NK cell. Statistical analysis was performed by Mann Whitney test by using GraphPad Prism **P* < 0.05; two-tailed; ** *P*=0.12; two-tailed.

### Example 5: VLA-dCNP eradicated regulatory T cell in breast cancer/tumor allowing the immune system to suppress tumor growth.

It is known that there are many regulatory T cells (Treg; cluster of differentiation 4 positive (CD4+)/cluster of differentiation 25 positive (CD25+)/forkhead box P3 (FOXP3+)) in cancers/tumors that suppress tumor immunity. Since Tregs are suppressed by VLA-dCNP, referring to FIGURES 5A and 5B, it was found that VLA-dCNP also showed a partial suppression of T-cell immunoglobulin and mucin-domain containing-3 (TIM-3), which is an index of T cell exhaustion (a state in which immunity is decreased). This indicated that VLA-dCNP not only enhances immune checkpoint inhibitors but may enhance the effects of other immunotherapy such as chimeric antigen receptor T-cell (CAR-T) therapy, since these cells are exogenously grown and administered and are likely more susceptible to exhaustion. In addition, VLA-dCNP alone has the potential to be used as immunotherapy. Without wishing to be bound be theory, it is believed that Treg in mammary glands is about 3%-4.3%, and Tim3 in mammary glands is about 290-350 fluorescence Intensity.

Female C57BL/6J mice (6 weeks old, female, n=4/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with mouse breast cancer cell line E0771 (ATCC, Old Town Manassas, VA) (250,000 cells/mouse, subcutaneous implantation to left mammary gland). From 4th day after implantation, mice were treated with 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone as control (subcutaneous injection under isoflurane anesthesia, 5 times/week). At 2 weeks, mice were sacrificed, and tumor tissues were harvested and single cells were obtained by using BD Horizon^{™} Dri Tissue & Tumor Dissociation Reagent (BD, Franklin Lakes NJ). Cells were stained by each antibody set and analyzed by flow cytometer (Verse, BD, Franklin Lakes, NJ); error bars are SEM. After measuring all particles (around 10,000) using a flow cytometer by based on particle size (FSC) and structural disorder (SSC) to gate a population that seems to be cells. This population is set to 100. Then, Regulatory T cell (Treg) (CD4+, CD25+, and Foxp3+; these are Treg marker molecules) were measured and calculated out the total population and converted to percentage. In healthy murine mammary gland, it is estimated about 2.5%. For Tim3, the value of Tim3 in the Cytotoxic T cell (CTL), CD3+ CD8+ population is shown by fluorescence intensity. In healthy murine mammary gland, it is estimated over 600 fluorescence intensity (TBD). Statistical analysis was performed by Mann Whitney test by using GraphPad Prism **P* < 0.05; two-tailed; ** *P*=0.24; two-tailed.

### Example 6: The Effect of VLA-dCNP on bone tumor volume growth with or without cluster of differentiation 8 (CD8) depletion showed that anti-tumor action of VLA-dCNP is directly related to CD8 activation since neutralization of CD8 resulted in loss of anti-tumor action of VLA-dCNP (FIGURE 6).

For this study, male Balb/c mice (6 week) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice (n=11, 10, and 10) were implanted with mouse osteosarcoma cell K7M2 (ATCC, Old Town Manassas, VA) (50,000 cells/mouse, subcutaneous implantation to right-side back). From 4^{th} day after implantation, mice were treated with a bolus dose of 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd., Tokyo, Japan); histidine 50 mM (Tokyo Chemical Industry Co., Ltd., Tokyo, Japan) in H₂O (Otsuka Pharmaceutical, Tokushima, Japan)) or buffer (for control group) 5 times a week via subcutaneous injection under isoflurane anesthesia. Mice of anti-CD8 group were intraperitoneally administered a bolus dose of 5 mg/Kg anti-CD8 antibody (YTS169.4 BioXcell; West Lebanon, NH) twice a week. Tumor size were measured with a caliper. Statistical analysis was performed by Student t-tests by using GraphPad Prism **P* < 0.05 vs. Control group (*n* =10) and vs. VLA-dCNP + Anti-CD8 group (n =10); error bars are SEM.

Referring to FIGURE 6, the anti-tumor action of VLA-dCNP is directly related to CD8 activation because neutralization of CD8 resulted in loss of anti-tumor action of VLA-dCNP.

### Example 7: VLA-dCNP suppressed the size of growth of bone cancer in subcutaneous implantation model in mouse.

In this study, Male Balb/c mice (6 week) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice (n = 17/group) were implanted with mouse osteosarcoma cell K7M2 (ATCC, Old Town Manassas, VA) (50,000 cells/mouse, subcutaneous implantation to right-side back). From 4th day after implantation, mice were treated with a bolus dose of 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone as control (subcutaneous injection under isoflurane anesthesia, 5 times/week). Statistical analysis was performed by Student t-tests by using GraphPad Prism **P* < 0.01 vs. Control group (*n* =17); error bars are SEM.

Referring to FIGURE 7, VLA-dCNP suppressed the size of growth of bone cancer in subcutaneous implantation model in mouse.

### Example 8A: The effect of VLA-dCNP on bone tumor volume growth

Referring to FIGURE 8A, the effect of VLA-dCNP on bone tumor volume growth with or without cluster of differentiation 8 (CD8) depletion in orthotopic implantation (femur) model in mice showed that anti-tumor action of VLA-dCNP is directly related to CD8 activation since neutralization of CD8 resulted in loss of anti-tumor action of VLA-dCNP. VLA-dCNP is shown to be an immune stimulator of cytotoxic T-cell, resulting in reduction of bone tumor volume growth.

In this study, Male CH3He mice (5 week) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice (n = 8, 8, 6, 6) were implanted with mouse osteosarcoma cell LM8 (RCB, Tsukuba, Japan) (1000,000 cells/mouse, orthotopic implantation to Femur bone). From 4^{th} day after implantation, mice were treated with a bolus dose of 0.3 mg/kg VLA-dCNP (described in Example 1) in buffer (Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H2O (Otsuka Pharmaceutical, Tokushima, Japan)) or buffer (for control group) (subcutaneous injection under isoflurane anesthesia, 5 times/week). Mice of anti-CD8 group/ anti-CD8 + VLA-dCNP group were intraperitoneally administrated 5 mg/kg anti-CD8 antibody (YTS 169.4 BioX cell; West Lebanon, NH) twice a week from 4 days before implantation. Tumor sizes were measured with caliper at 15 day. Statistical analysis was performed by Student t-test by using GraphPad Prism *<0.01 VLA-dCNP (n=8) vs. Control group (n=8), anti-CD8 (n=6), or anti-CD8 + VLA-dCNP (n=6); error bars are SEM.

### Example 8B: The effect of VLA-dCNP on activation of immunity in bone cancer subcutaneous implantation mouse model.

Transforming growth factor beta 1 (TGF-beta1) is a cytokine involved in immunosuppression. Forkhead box P3 (Foxp3) is a marker of regulatory T cells involved in suppression of tumor immunity, and Bv8 (Prokineticin proteins) is a factor secreted by Myeloid-derived suppressor cells (MDSCs) involved in immunosuppression (Neoplasia 2014, 16 501-510). Referring to FIGURES 8B-8D, the expression of all three markers are low suggested there are few cells that suppress immunity. Therefore, it can be interpreted that the tumor growth was suppressed by the administration of VLA-dCNP because the immune system was relatively activated. In this study, small intestine was used. Many mice in the VLA-dCNP administered group had tumors that completely disappeared. The combination of VLA-dCNP/PD-1 caused most of the tumors to disappear, so no sample was available to measure. Therefore, immune parameters were alternatively evaluated in the intestinal tract, which is the center of immunity. Without wishing to be bound be theory, it is believed that TGF-beta 1 in intestine is about 8.5%-9.7%, Foxp3 in intestine is about 7%-13%, and Bv8 in 8%-48%.

In this study, male Balb/c mice (6 week) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice (n = 17/group) were implanted with mouse osteosarcoma cell K7M2 (ATCC, Old Town Manassas, VA) (50,000 cells/mouse, subcutaneous implantation to right-side back). From 4th day after implantation, mice were treated with a bolus dose of 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone as control (subcutaneous injection under isoflurane anesthesia, 5 times/week). At 4 weeks, mice were sacrificed and small intestine were harvested and minced in Trireagent (Molecular Research Center, Inc. Cincinnati, OH) and kept at -80 °C until analysis. Total RNA was extracted from harvested lung tissue by the chloroform-phenol method. Complementary DNA (cDNA) was synthesized from extracted mRNA with cDNA kit (Qiagen, Hilden Germany). Quantitative RT-PCR analysis was performed by premix kit (Takara bio, Shiga Japan). Expression levels of target genes in synthesized cDNA were measured by real-time RT-PCR method. Actb gene was used as an internal standard. It was calculated using internal standard; the expression measurement (sample)/b-actin expression measurement = individual expression amount. Then, normalized the Control group to be 100% and individual group to be calculated against the Control group. Statistical analysis was performed by Student t-tests by using GraphPad Prism **P* < 0.01 vs. Control group (*n* =17); error bars are SEM.

### Example 9: VLA-dCNP normalized breast cancer tumor vascular structure

Cluster of differentiation 31 (CD31) is a marker for vascular endothelium and alpha smooth muscle actin (alpha-SMA) is a marker for pericytes. In normal blood vessels, pericytes are located near the blood vessels, so the presence of pericytes can be used to evaluate the existence of normal blood vessels. Referring to FIGURE 9A, because the overlapping areas of each fluorescence increased in the VLA-dCNP administration group, this indicates that blood vessels with sufficient function (healthy) were formed. In theory, normal healthy vascular structure will have 100% pericyte-coating index.

Female C57BL/6J mice (6 weeks old, female, n=4/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with mouse breast cancer cell line E0771 (ATCC, Old Town Manassas, VA) (250,000 cells/mouse, subcutaneous implantation to left mammary gland). From 4th day after implantation, mice were treated with a bolus dose of 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone as control (subcutaneous injection under isoflurane anesthesia, 5 times/week). At 2 weeks, mice were sacrificed and tumor tissues were harvested and frozen. Frozen section of tumor samples were prepared. CD31 and alpha-SMA in tumor samples were immunohistochemically stained by using each antibody (Cell Signaling Technology, Danvers MA) and detected with fluorescence microscope (BZ-X700, Keyence, Tokyo Japan).

FIGURE 9A shows the fluorescence microscope images of red CD31 and green alpha-SMA. Magnification was low field power x20 for all the images. FIGURE 9B shows the % index of pericyte-coating; error bars are SEM. In theory, normal healthy vascular structure will have 100% pericyte-coating index. The pericyte-coating index was calculated using alpha-SMA as an index, (CD31+ alpha-SMA+)/CD31+. Statistical analysis was performed by Student t-tests by using GraphPad Prism **P* < 0.05 vs. Control group (*n* =4).

### Example 10: VLA-dCNP normalized tumor vascular structure

Cluster of differentiation 31 (CD31) is a vascular endothelial marker that indicates the presence of blood vessels. The lectin indicates whether blood is actually flowing through the blood vessels (to exclude many non-functioning blood vessels that are present in the tumor tissue). In other word, using these two stains, the number of functional blood vessels can be determined. As shown on the previous example, administration of VLA-dCNP increased the number of functional blood vessels. The present Example confirms that blood passes through these blood vessels.

Female C57BL/6J mice (6 weeks old, female, n=3/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with mouse breast cancer cell line E0771 (ATCC, Old Town Manassas, VA) (250,000 cells/mouse, subcutaneous implantation to left mammary gland). From 4th day after implantation, mice were treated with a bolus dose of 0.3 mg/Kg VLA-dCNP (described in Example 1) in buffer (Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical ,Tokushima, Japan)) or buffer alone as control (subcutaneous injection under isoflurane anesthesia, 5 times/week). At 2 weeks, mice were intravenously injected 50 ug/mouse fluorescence-labeled tomato-lectin (FL-1171, VECTOR Laboratories, Inc., Burlingame, CA). After 5 minutes from the lectin injection, mice were sacrificed and tumor tissues were harvested and frozen. CD31 in tumor samples were immunohistochemically stained. Stained CD31 and lectin were detected with fluorescence microscope (BZ-X700, Keyence, Tokyo Japan).

FIGURE 10A shows the fluorescence microscope images of red CD31 and green lectin. Magnification was low field power x20 for all the images. FIGURE 10B shows the count of CD31 and lectin structure per field; error bars are SEM. Normal vascular structure shows higher structure number per field. Statistical analysis was performed by Student t-tests by using GraphPad Prism **P* < 0.05 vs. Control group (*n* =3)*.*

### Example 11: VLA-dCNP reduced hypoxia condition in tumor

Because pimonidazole binds to the thiol group (-SH) of hypoxic proteins, it was used as a marker to determine the oxygen status in tissues. Referring to FIGURE 11A, hypoxic regions were visualized by staining pimonidazole administered before dissection with an antibody. Since this fluorescence was reduced by administration of VLA-dCNP, it was found that the hypoxic condition was resolved. In addition to the results, it is understood that blood vessels are formed and oxygen is sufficiently transported. Because the hypoxic region is involved in tumor malignancy, anticancer drug resistance, and suppression of tumor immunity, it is believed based that VLA-dCNP can reduce these effects.

Female C57BL/6J mice (6 weeks old, female, n=4/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with mouse breast cancer cell line E0771 (ATCC, Old Town Manassas, VA) (250,000 cells/mouse, subcutaneous implantation to left mammary gland). From 4th day after implantation, mice were treated with a bolus dose of 0.3 mg/Kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone as control (subcutaneous injection under isoflurane anesthesia, 5 times/week). At 2 weeks, mice were intravenously injected 150 ug/mouse pimonidazole (Hypoxyprobe^{™}-1, Hypoxyprove, Inc., Burlington, Massachusetts). After 30 minutes from the pimonidazole injection, mice were sacrificed and tumor tissues were harvested and frozen. Frozen section of tumor samples were prepared. Pimonidazole in tumor samples were immunohistochemically stained as followed by manufacture's protocol. Stained pimonidazole were detected with a fluorescence microscope (BZ-X700, Keyence, Tokyo Japan).

FIGURE 11A shows the fluorescence microscope images of red pimonidazole. Magnification was low field power x20 for all the images. FIGURE 11B shows the percentage of relative intensity of red pimonidazole; error bars are SEM. In theory, the healthy tissue will have no hypoxia therefore, the relative intensity will be 0%. Statistical analysis was performed by Student t-tests by using GraphPad Prism **P* < 0.05 vs. Control group (*n* =4).

### Example 12: VLA-dCNP and anti-mouse cytotoxic T-lymphocyte-associated protein 4 (CTLA-4) antibody combination therapy suppressed growth of colon cancer in subcutaneous implantation model in mouse

Referring to FIGURE 12, the colon tumor size at various days after exposure to various therapeutic agents is shown. VLA-dCNP and anti-mouse cytotoxic T-lymphocyte-associated protein 4 (CTLA-4) antibody combination therapy greatly suppressed growth of colon cancer.

In this study, male C57BL/6J mice (6 weeks old, male, n=9-10/group) were purchased from Kyudo (Saga, Japan) and maintained under a 12-hour light/12-hour dark cycle with free access to water and standard mouse diet (MF diet; Oriental Yeast Co., Lt. Tokyo, Japan). Mice were implanted with MC38 mouse colon carcinoma cell (1 × 106 cells/mouse) (Donation) in the subcutaneous and treated with a bolus dose of the following: very long acting CNP derivative or VLA-dCNP (described in Example 1) (0.3 mg/kg s.c.) with anti-CTLA-4 Ab (BioX cell, clone 9H10; West Lebanon, NH) or with Isotype control Ab (BioX cell, BE0087; West Lebanon, NH), native C-type natriuretic peptide or CNP (0.3 mg/kg s.c.) with anti-CTLA-4 Ab or with Isotype control Ab, CNP derivative or dCNP (described in Example 1) (0.3 mg/kg s.c.) with anti-CTLA-4 Ab or with Isotype control Ab, B-Type natriuretic peptide or BNP (0.3 mg/kg s.c.) with anti-CTLA-4 Ab or with Isotype control Ab starting from 8th Day. Buffer control with Isotype control Ab and buffer with anti-CTLA-4 Ab were included. Mice were treated with 2.5 mg/kg anti-CTLA-4 Ab or Isotype control Ab via i.p. once a week. Tumor sizes were measured with caliper. The results showed that individual treatment (without anti-mouse CTLA-4 antibody or anti-mouse CTLA-4 antibody alone) is less effective than the combined treatment (with anti-mouse CTLA-4 antibody) for tumor volume reduction. Statistical analysis was performed by Student t-tests by using GraphPad Prism *P < 0.01 vs. Control group (n =10); 1 P<0.05 vs. Control group (n =10).

### Example 13: VLA-dCNP and anti-mouse (cytotoxic T-lymphocyte-associated protein 4) CTLA-4 antibody combination therapy suppressed the growth of colon cancer after developed tumor using control group from Example 12 in subcutaneous implantation model in mouse.

Referring to FIGURE 13A, the colon tumor size as a function of various therapeutic agents is shown as a graph. A corresponding table of tumor size at various days after exposure to various therapeutic agents is shown in FIGURE 13B. VLA-dCNP and anti-mouse (cytotoxic T-lymphocyte-associated protein 4) CTLA-4 antibody combination therapy greatly suppressed the growth of colon cancer. A synergistic effect can be observed.

In this study, male C57BL/6J mice (6 weeks old, male, n=5-7/group) were purchased from Kyudo (Saga, Japan) and maintained under a 12-hour light/12-hour dark cycle with free access to water and standard mouse diet (MF diet; Oriental Yeast Co., Lt. Tokyo, Japan). Mice were implanted with MC38 mouse colon carcinoma cell (1 × 10⁶ cells/mouse) (Donation) in the subcutaneous. After 22^{nd} day, control group were divided into three groups. From 22^{nd} day, mice were treated with a bolus dose of the following: very long acting CNP derivative or VLA-dCNP (described in Example 1) (0.3 mg/kg s.c.) with anti-CTLA-4 Ab (BioX cell, clone 9H10; West Lebanon, NH), native C-type natriuretic peptide or CNP (0.3 mg/kg s.c.) with anti-CTLA-4 Ab, CNP derivative or dCNP (described in Example 1) (0.3 mg/kg s.c.) with anti-CTLA-4 Ab. Buffer control with anti-CTLA-4 Ab was included. Mice were treated with 2.5 mg/kg anti-CTLA-4 Ab via i.p. twice a week. Tumor sizes were measured with caliper. Statistical analysis was performed by Student's t test by using GraphPad Prism 6.0 (*n* =5). **P* < 0.01 (n=5-7)

### Example 14: VLA-dCNP and anti-mouse Programmed cell death protein 1 (PD-1) antibody combination therapy suppressed growth (volume) of colon cancer in subcutaneous implantation model in mouse.

Referring to FIGURE 14, the colon tumor size at various days after exposure to various therapeutic agents is shown. VLA-dCNP and anti-mouse Programmed cell death protein 1 (PD-1) antibody combination therapy greatly suppressed growth (volume) of colon cancer. A synergistic effect can be observed.

In this study, male C57BL/6J mice (6 weeks old, male, n=10/group) were purchased from Kyudo (Saga, Japan) and maintained under a 12-hour light/12-hour dark cycle with free access to water and standard mouse diet (MF diet; Oriental Yeast Co., Lt. Tokyo, Japan). Mice were implanted with MC38 mouse colon carcinoma cell (1 × 10⁶ cells/mouse) (Donation) in the subcutaneous and treated with a bolus dose of the following: very long acting CNP derivative or VLA-dCNP (described in Example 1) (0.3 mg/kg s.c.) with anti-PD-1 Ab (BioX cell, clone J43; West Lebanon, NH) or with Isotype control Ab (BioX cell, BE0091; West Lebanon, NH), native C-type natriuretic peptide or CNP (0.3 mg/kg s.c.) with anti-PD-1 Ab or with Isotype control Ab, CNP derivative or dCNP (described in Example 1) (0.3 mg/kg s.c.) with anti-PD-1 Ab or with Isotype control Ab, B-Type natriuretic peptide or BNP (0.3 mg/kg s.c.) with anti-PD-1 Ab or with Isotype control Ab starting from 8^{th} Day. Buffer control with Isotype control Ab and buffer with anti-PD-1 Ab were included. Mice were treated with 5 mg/kg anti-PD-1 Ab or Isotype control Ab via i.p. once a week. Tumor sizes were measured with caliper. The results showed that individual treatment (without anti-mouse PD-1 antibody) is less effective than the combined treatment (with anti-mouse PD-1 antibody) for tumor volume reduction. Example group see between VLA-dCNP group against VLA-dCNP + PD-1 group and dCNP group against dCNP + PD-1 group. Statistical analysis was performed by Student t-tests by using GraphPad Prism **P* < 0.05 vs. PD-1 control group *(n* =10); 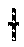 P<0.05 vs. Control group (n =10).

### Example 15: VLA-dCNP and anti-cytotoxic T-lymphocyte-associated protein 4 (CTLA-4) antibody combination therapy suppressed the growth of skin cancer in orthotopic transplantation model in mice

Referring to FIGURE 15, the skin tumor size at various days after exposure to various therapeutic agents is shown. VLA-dCNP and anti-cytotoxic T-lymphocyte-associated protein 4 (CTLA-4) antibody combination therapy greatly suppressed the growth of skin cancer.

In this study, male C57BL/6J mice (6 weeks old, male, n=8-10/group) were purchased from Kyudo (Saga, Japan) and maintained under a 12-hour light/12-hour dark cycle with free access to water and standard mouse diet (MF diet; Oriental Yeast Co., Lt. Tokyo, Japan). Mice were implanted with B16 melanoma cancer cells (25,000 cells/mouse) (ATCC Manassas, MA) in the subcutaneous and treated with a bolus dose of the following: very long acting CNP derivative or VLA-dCNP (described in Example 1) (0.3 mg/kg s.c.) with anti-CTLA-4 Ab (BioX cell, 9H10; West Lebanon, NH) or with Isotype control Ab (BioX cell, BE0087; West Lebanon, NH), native C-type natriuretic peptide or CNP (0.3 mg/kg s.c.) with anti-CTLA-4 Ab or with Isotype control Ab, CNP derivative or dCNP (described in Example 1) (0.3 mg/kg s.c.) with anti-CTLA-4 Ab or with Isotype control Ab, B-Type natriuretic peptide or BNP (0.3 mg/kg s.c.) with anti-CTLA-4 Ab or with Isotype control Ab starting from 7th Day. Buffer control with Isotype control Ab and buffer with anti-CTLA-4 Ab were included. Mice were treated with 10 mg/kg anti-CTLA-4 Ab or Isotype control Ab via i.p. once a week. Tumor sizes were measured with caliper. Statistical analysis was performed by Student t-tests by using GraphPad Prism *P < 0.05 vs. CTLA-4 group (n=8 or 10).

### Example 16: VLA-dCNP or dCNP and anti-mouse Programmed cell death protein 1 (PD-1) antibody therapy suppressed growth of breast cancer in orthotopic transplantation model in mice.

Referring to FIGURE 16, the breast tumor size at various days after exposure to various therapeutic agents is shown. VLA-dCNP or dCNP and anti-mouse Programmed cell death protein 1 (PD-1) antibody therapy greatly suppressed growth of breast cancer. A synergistic effect can be observed.

In this study, female C57BL/6J mice (6 weeks old, female, n=7 or 10/group) were purchased from Kyudo (Saga, Japan) and maintained under a 12-hour light/12-hour dark cycle with free access to water and standard mouse diet (MF diet; Oriental Yeast Co., Lt. Tokyo, Japan). Mice were implanted with E0771 breast cancer cells (250,000 cells/mouse) (Cosmo Bio Tokyo, Japan) in the left mammary gland. From the 4^{th} day, mice were treated with a bolus dose of the following: very long acting CNP derivative or VLA-dCNP (described in Example 1) (0.3 mg/kg s.c.) with anti-PD-1 Ab (BioX cell; clone RMP1-14, West Lebanon, NH) or with Isotype control Ab (BioX cell, BE0089; West Lebanon, NH), native C-type natriuretic peptide or CNP (0.3 mg/kg s.c.) with anti-PD-1 Ab or with Isotype control Ab, CNP derivative or dCNP (described in Example 1) (0.3 mg/kg s.c.) with anti-PD-1 Ab or with Isotype control Ab, B-Type natriuretic peptide or BNP (0.3 mg/kg s.c.) with anti-PD-1 Ab or with Isotype control Ab, anti-Tumor necrosis factor alpha antibody or TNFa ab (BioXcell, clone XT3.11; West Lebanon, NH) (0.3 mg/kg s.c.) with anti-PD-1 Ab or with Isotype control Ab, and cGMP degradation inhibitor or PDE5 inhibitor called Vardenafil (VDN, Cayman Chemicals Ann Arbor, MI) (0.3 mg/kg s.c.) with anti-PD-1 Ab or with Isotype control Ab starting from 4^{th} Day. Buffer control with Isotype control Ab and buffer with anti-PD-1 Ab were included. Mice were treated with 5 mg/kg anti-PD1 Ab or Isotype control Ab via i.p. twice a week. Tumor sizes were measured with caliper. The results showed that individual treatment (without anti-mouse PD-1 antibody or anti-mouse PD-1 antibody alone) is less effective than the combined treatment (with anti-mouse PD-1 antibody) for tumor volume reduction. Example group see between VLA-dCNP group against VLA-dCNP + PD-1 group and dCNP group against dCNP + PD-1. Statistical analysis was performed by Student t-tests by using GraphPad Prism **P* < 0.01 vs. PD-1 control group (*n* =7 or 10); 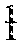 *P*<0.05 vs. Control group (n =7 or 10)

### Example 17: VLA-dCNP with anti-mouse PD-1 (Programmed cell death protein 1) antibody combination therapy suppressed the growth (volume) of breast cancer orthotopic transplantation model in mice showing dose response manner.

Referring to FIGURE 17, the breast tumor size at various days after exposure to various therapeutic agents is shown. VLA-dCNP with anti-mouse PD-1 (programmed cell death protein 1) antibody combination therapy greatly suppressed the growth (volume) of breast cancer. A synergistic effect could be observed.

In this study, female C57BL/6J mice (6 weeks old, female, n=7-9) were purchased from Kyudo (Saga, Japan) and maintained under a 12-hour light/12-hour dark cycle with free access to water and standard mouse diet (MF diet; Oriental Yeast Co., Ltd. Tokyo Japan). Mice were implanted with E0771 breast cancer cells (250,000 cells/mouse) (Cosmo Bio Tokyo, Japan) in the left mammary gland. From the 4^{th} day, mice were treated with a bolus dose of very long acting CNP derivative or VLA-dCNP (described in Example 1) (L: 0.1 mg/kg s.c., M: 0.3 mg/kg s.c., H: 1.0 mg/kg s.c.) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous injection under isoflurane anesthesia, 5 times/week). Mice of anti-PD1 Ab treated groups were intraperitoneally administrated with a bolus dose of 5 mg/kg anti-PD1 Ab (BioX cell, RMP1-14; West Lebanon, NH) twice a week. The other groups were treated with a bolus dose of isotype control Ab (BioX cell, BE0089; West Lebanon, NH). Tumor sizes were measured with caliper. Referring to FIGURE 17B, bold font at Day 14 shows VLA-dCNP and anti-PD-1 combination dose response and italicized font at Day 14 shows VLA-dCNP and isotype control Ab combination dose response. The results showed that individual treatment (without anti-mouse PD-1 antibody or anti-mouse PD-1 antibody alone) is less effective than the combined treatment (with anti-mouse PD-1 antibody) for tumor volume reduction. Example group see between VLA-dCNP (L, M, or H) group against VLA-dCNP (L, M, or H) + PD-1 group.

### Example 18: VLA-dCNP and anti-mouse Programmed death-ligand 1 (PD-L1) antibody combination therapy suppressed the growth (volume) of breast cancer in orthotopic transplantation model in mice.

The adaptive immune system reacts to antigens that are associated with immune system activation by exogenous or endogenous danger signals. In turn, clonal expansion of antigen-specific CD8+ T cells and/or CD4+ helper cells is propagated. The binding of PD-L1 to the inhibitory checkpoint molecule PD-L1 transmits an inhibitory signal based on interaction with phosphatases (SHP-1 or SHP-2) via Immunoreceptor Tyrosine-Based Switch Motif (ITSM). This reduces the proliferation of antigen-specific T-cells in lymph nodes, while simultaneously reducing apoptosis in regulatory T cells (anti-inflammatory, suppressive T cells) - further mediated by a lower regulation of the gene Bcl-2.

Up-regulation of PD-L1 may allow cancers to evade the host immune system. An analysis of 196 tumor specimens from patients with renal cell carcinoma found that high tumor expression of PD-L1 was associated with increased tumor aggressiveness and a 4.5-fold increased risk of death. Clinically available examples of PD-L1 inhibitors include durvalumab, atezolizumab and avelumab.

Referring to FIGURE 18A, the breast tumor size as a function of various therapeutic agents is shown as a graph. A corresponding table of tumor size at various days after exposure to various therapeutic agents is shown in FIGURE 18B. VLA-dCNP and anti-mouse Programmed death-ligand 1 (PD-L1) antibody combination therapy suppressed the growth (volume) of breast cancer. A synergistic effect could be observed.

In this study, female C57BL/6J mice (6 weeks old, female, n=8-9) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with E0771 mouse breast cancer cells (250,000 cells/mouse) (Cosmo Bio, Tokyo Japan) in the left mammary gland. From the 4^{th} day, mice were treated with a bolus dose of 0.3 mg/kg very long acting CNP derivative or VLA-dCNP (described in Example 1) in buffer (Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)) or buffer (for control group) (subcutaneous injection under isoflurane anesthesia, 5 times/week). Mice of anti-PDL1 Ab treated groups were intraperitoneally administrated with a bolus dose of 5 mg/kg anti-PDL1 Ab (BioX cell, 10F.9G2; West Lebanon, NH) twice a week. The other groups were treated with a bolus dose of isotype control Ab (BioX cell, BE0090; West Lebanon, NH). Tumor sizes were measured with caliper. The results showed that individual treatment (without anti-mouse PDL-1 antibody or anti-mouse PDL-1 antibody alone) is less effective than the combined treatment (with anti-mouse PD-1 antibody) for tumor volume reduction. See group between VLA-dCNP group against VLA-dCNP + PD-L1. Statistical analysis was performed by Student t-tests by using GraphPad Prism **P* < 0.01 vs. Control or PD-L1 group (*n* =8-9).

### Example 19: VLA-dCNP and anti-PD-1 antibody combination therapy suppressed the growth (volume) of breast cancer in orthotopic transplantation model in mice.

In syngeneic mice, EMT-6 cells form tumors and spontaneous metastases, primarily to the lungs. More recently, EMT-6 has emerged as a valuable pre-clinical model for immuno-oncology studies of triple negative breast cancer. EMT-6 tumors express PD-L1 and are moderately responsive to immunotherapies. Individual checkpoint inhibitors (anti-CTLA-4 or anti-PD-L1) generally have modest effect on tumor growth, while combination therapies exhibit greater success, making EMT6 a beneficial model for combination therapy studies.

Referring to FIGURE 19A, the breast tumor size as a function of various therapeutic agents is shown as a graph. A corresponding table of tumor size at various days after exposure to various therapeutic agents is shown in FIGURE 19B. VLA-dCNP and anti-PD-1 antibody combination therapy greatly suppressed the growth (volume) of breast cancer. A synergistic effect could be observed.

In this study, female Balb/c mice (6 weeks old, female, n=7-9) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with EMT-6 breast cancer cells (ATCC Manassas, VA) (200,000 cells/mouse, subcutaneous implantation to left mammary grant). At 4^{th} day after implantation, mice were treated with a bolus dose of 0.1 mg/kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous injection under isoflurane anesthesia, 5 times/week). Mice of anti-PD1 Ab treated groups were intraperitoneally administrated with a bolus dose of 5 mg/kg anti-PD1 Ab (BioX cell, RMP1-14; West Lebanon, NH) twice a week. The other groups were treated with a bolus dose of isotype control Ab (BioX cell, BE0089; West Lebanon, NH). Tumor sizes were measured with caliper. The results showed that individual treatment (without anti-mouse PD-1 antibody or anti-mouse PD-1 antibody alone) is less effective than the combined treatment (with anti-mouse PD-1 antibody) for tumor volume reduction. See group between VLA-dCNP group against VLA-dCNP + PD-1 or anti PD-1 group. Statistical analysis was performed by two-way ANOVA and Tukey's post hoc tests by using GraphPad Prism 6.0 **P* < 0.05.

### Example 20: VLA-dCNP and anti-PD-1 antibody combination therapy suppressed the growth of breast cancer and improve the survival in mice

In this study, female Balb/c mice (6 weeks old, female, n=7-9) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were implanted with EMT-6 breast cancer cells (ATCC Manassas, VA) (200,000 cells/mouse, subcutaneous implantation to left mammary grant). At 4^{th} day after implantation, mice were treated with a bolus dose of 0.1 mg/kg VLA-dCNP (described in Example 1) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous injection under isoflurane anesthesia, 5 times/week). Mice of anti-PD1 Ab treated groups were intraperitoneally administrated with a bolus dose of 5 mg/kg anti-PD1 Ab (BioX cell, RMP1-14; West Lebanon, NH) twice a week. The other groups were treated with a bolus dose of isotype control Ab (BioX cell, BE0089; West Lebanon, NH). The results showed that individual treatment (without anti-mouse PD-1 antibody or anti-mouse PD-1 antibody alone) is less effective than the combined treatment (with anti-mouse PD-1 antibody) for survival. VLA-dCNP vs VLA-dCNP + Anti-PD1 ab P<0.05.

Referring to FIGURE 20, VLA-dCNP and anti-PD-1 antibody combination therapy greatly suppressed the growth of breast cancer and improve the survival in mice. A synergistic effect could be observed.

### Example 21: Treatment with Very Long acting NPRB agonist (VLA-dCNP) alone improves the survival of mice with Osteosarcoma in the tibial bone during the terminal phase of the disease.

FIGURE 21 is a Kaplan-Meier curve showing that VLA-dCNP treatment improved the survival in mice with osteosarcoma in the tibial bone.

In this study, male CH3HeN mice (4 weeks old, male, n=8) were purchased from Kyudo (Saga, Japan) and maintained under a 12-hour light/12-hour dark cycle with free access to water and standard mouse diet (MF diet; Oriental Yeast Co., Ltd. Tokyo Japan). Mice were implanted with LM8 mouse osteosarcoma cells (1,000,000 cells/mouse) (donation, Japan) in the tibial bone (orthotopic implantation) at Day 0. From the 15^{th} day, the beginning of what is considered the "terminal phase" of the disease, mice were treated by subcutaneously administering a bolus dose of a very long acting CNP derivative or VLA-dCNP (described in Example 1) in buffer (Methionine 100 mM (Tokyo Chemical Industry Co., Ltd., Tokyo, Japan); sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)) or buffer alone (for control group). The subcutaneous injections were performed under isoflurane anesthesia, 5 times/week. The survival or number of deaths was monitored and recorded.

FIGURE 21 is a Kaplan-Meier curve for 27 days, and shows that treatment with VLA-dCNP alone improved the survival in mice with Osteosarcoma in the tibial bone. Implantation of LM8 cells was at Day 0. The terminal phase was considered to be after Day 15 (Black Arrow).

### Example 22: Long acting NPRB agonist (dCNP) enhances LM8-elicited IFNg production in splenocytes

Interferon gamma (IFNg) plays a key role in activation of cellular immunity and subsequently, stimulation of antitumor immune response. IFN-g may inhibit angiogenesis in tumor tissue, induce apoptosis of regulatory T-cell (a suppressor of immune response) and/or stimulate the activity of M1 proinflammatory macrophages to overcome tumor progression.

In this study, mouse osteosarcoma cancer cell line LM8 (donation from Kyushu) was maintained in alpha MEM supplemented (Fujifilm, Tokyo, Japan) with 10% Fetal Calf Serum (FCS; Sigma Aldrich, St Louis, MO) and inoculated into 96-well plates (10,000 cells/well in RPMI 1640 supplemented (Fujifilm, Tokyo, Japan) with 1,000,000 cells splenocytes derived from orthotopic LM8 bearing mice) and cells were treated with different concentration of long acting CNP derivatives or dCNP (described in Example 1) (0, 0.5 (1.6 ng/mL), and 5 nM (16 ng/mL) for 96 hours. Negative control in this study is has splenocytes only and no dCNP, and control has both LM8 and splenocytes but no dCNP to determine baseline. Supernatant was harvested and IFNg levels were measured by ELISA assay (R&D Systems, Minneapolis, MN). Statistical analysis was performed by Student t-tests by using GraphPad Prism **P* < 0.01 vs. Control (*n* =3 or 4).

FIGURE 22 shows that dCNP increased interferon gamma (IFNg) production in splenocytes exposed to cultured LM8 mouse osteosarcoma cancer cell line in a dose dependent manner.

### Example 23: Long acting NPRB agonist (dCNP) anti-tumor effect is mediated by CD8 activities in mice prostate cancer model and as effective as non-CD8 mediated cytotoxic chemotherapeutic agent.

FIGURE 23A shows graph of tumor size at Day 20 after 14-day treatment with various test articles starting at day 6. FIGURE 23B shows the tumor growth progress over the treatment period along with tumor eradication at Day 20. Error bars are SEM and individual dots represent individual animals in the group. FIGURES 23A and 23B show that dCNP (described in Example 1) at 0.3 and 1.0mg/kg was significantly effective in suppressing prostate cancer growth in mice and as effective as cytotoxic chemotherapeutic agent (Docetaxel) when evaluated at day 20. However, in the presence of Anti-CD8 antibody (Ab), the tumor growth suppressing effect of Docetaxel remained while the effect of dCNP was neutralized indicating that the mechanism of dCNP anti-tumor activity was mediated mainly by cytotoxic CD8 T-cells. CD8 serves as a co-receptor for the T-cell receptor (TCR). Along with the TCR, the CD8 co-receptor plays a role in T cell signaling and aiding with cytotoxic T cell antigen interactions to eliminate tumor. If mediated by CD8 then Anti-CD8 is expected neutralizes the effect as seen for dCNP but not for Docetaxel. Thus, FIGURES 23A and 23B show that the long acting NPRB agonist (dCNP) anti-tumor effect was mediated by CD8 activities in mice prostate cancer model and was effective as non-immune mediated cytotoxic chemotherapeutic agent.

In this study, male C57BL/6J mice (6 weeks old, male, n=10) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with TRAMPC-1 mouse prostate cancer cells (1,000,000 cells/mouse) (ATCC, Manassas, VA) in the subcutaneous. From the 6^{th} day (early stage of tumor growth), mice were treated with a bolus dose of dCNP (described in Example 1) (0.3 mg/kg s.c. or 1.0 mg/kg s.c.) with or without Anti-CD8 antibody (a-CD8; 5 mg/Kg i.p.; BioX cells, West Lebanon, NH) in buffer containing Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)], Docetaxel (1 mg/kg i.p.) in Saline with or without Anti-CD8 Ab (5 mg/kg i.p.) as reference control, and or with Phosphate buffered saline (PBS) alone via i.p. as control. Mice were treated total of 14 days (5 days of once daily, 2 days of rest, 5 days of once-daily treatment and 2 days rest). Anti-CD8 Ab (a-CD8) was administered on -2, 1, 4, 7, 10 and 13^{th} day. Tumor sizes were measured with caliper. Statistical analysis was performed by Student t-tests by using GraphPad Prism **P* < 0.0001 vs. Control or a-CD8 group (*n* =10). CD8 serves as a co-receptor for the T-cell receptor (TCR). Along with the TCR, the CD8 co-receptor plays a role in T cell signaling and aiding with cytotoxic T cell antigen interactions to eliminate tumor. If mediated by CD8 thenAnti-CD8 is expected neutralizes the effect.

### Example 24: Long acting NPRB agonist (dCNP) administered at much later stage had tumor-eliminating effect against much larger or severe prostate tumors, and was as effective as a cytotoxic chemotherapeutic agent (Docetaxel), and additionally had a mechanism of action that was mediated by CD8 cells that was different than Docetaxel.

FIGURE 24A shows a graph of prostate tumor eliminating action of dCNP (described in Example 1) (even when the when administration was started at much later stage (tumor size is ~70mm3; at day 19). Also shown is that dCNP was as effective as the cytotoxic chemotherapeutic agent in eliminating tumor. FIGURE 24B is a table showing prostate cancer growth over the treatment period along with tumor eradication at Day 30 (Bottom Panel). Error bars are SEM and individual dots represent individual animals in the group. The mechanism of action of dCNP was mediated by cytotoxic CD8 T-cells since it could be neutralized by anti-CD8 antibody whereas the anti-tumor effect of toxic chemotherapeutic agent could not be neutralized by Anti-CD8 consistent with direct cytotoxic effect of chemotherapeutic agent on cancer cells. Additionally, dCNP was less lethal than cytotoxic chemotherapeutic agent (Docetaxel). CD8 serves as a co-receptor for the T-cell receptor (TCR). Along with the TCR, the CD8 co-receptor plays a role in T cell signaling and aiding with cytotoxic T cell antigen interactions to eliminate tumor. If mediated by CD8 then Anti-CD8 antibody is expected neutralizes the effect.

In this study, male C57BL/6J mice (6 weeks old, male, n=10) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with TRAMPC-1 mouse prostate cancer cells (1,000,000 cells/mouse) (ATCC, Manassas, VA) in the subcutaneous. From the 19^{th} day (averaged tumor size was 70 mm3), mice were treated with a bolus dose of long acting CNP derivative or dCNP (described in Example 1) (0.3 mg/kg s.c. or 1.0 mg/kg s.c.) in buffer [Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)], Docetaxel (DTX1 mg/kg i.p.) in Saline, all with or without anti-CD8 (5 mg/Kg i.p.; BioX cell; West Lebanon, NH) or Phosphate buffered saline (PBS) alone via i.p. All Docetaxel groups were used as mechanism of action comparator chemotherapeutic agent. Mice were treated total of two weeks (5 days of once daily, 2 days of rest, 5 days of once-daily treatment and 2 days rest). Anti-CD8 Ab was administered every 3 days starting day 19^{th}. Tumor sizes were measured with caliper.

### Example 25: Even in healthy normal animals, long acting NPRB agonist (dCNP) could activate the immune system as seen by an increase in T-cells, specifically cytotoxic (CD8) T-cells and natural killer (NK) cells in blood and CD8, CD4, ICOS, and CD86 gene expression in spleen after administration.

FIGURES 25A-25C show that even in normal healthy mice, dCNP could activate the immune system as seen by an increase in T-cells (CD4), cytotoxic (CD8) T-cells and natural killer (NK) cells in blood. FIGURES 25D-25G show that in the spleen, there was a corresponding increase in CD4, CD8, ICOS, and CD86 gene expression that were significantly elevated indicating activation of the immune system. ICOS is inducible T-cell co-stimulator and is an immune checkpoint protein and its expression indicates immune activation. CD86, along with CD80 provides costimulatory signals necessary for T cell activation and survival and its expression confirms immune activation.

In this study, male C57BL/6J mice (6 weeks old n=3) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were treated with a bolus dose of 0, 0.1, 0.3, and 1.0 mg/kg CNP derivative or dCNP (described in Example 1) in buffer (Methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer alone (for control group). Administration is by bolus subcutaneous injection under isoflurane anesthesia, at day 1, 2, 3, 4, and 5. Blood was harvested on day 5 under the isoflurane vapor and plasma were obtained by added EDTA and red blood cells were removed by pharma lyse (BD, Franklin Lakes, NJ). Cells were analyzed by FACS (BD, Franklin lakes, NJ). The spleen was harvested on day 5 under isoflurane vapor, then placed in the Tri reagent (Cosmobio, Tokyo, Japan) for further analysis. Statistical analysis was performed by Student t-tests by using GraphPad Prism **P* < 0.05 vs. Control, **P<0.01 vs Control (*n* =5).

### Example 26: Surprising synergistic effect of VLA-dCNP and Anti-PD1 antibody combination in increasing Th1 cells and decreasing Treg cells of the immune system that facilitate anti -tumor immunity in mice with breast cancer. Additionally. VLA-dCNP was significantly more effective than Anti-PD1 antibody in doing the same.

FIGURE 26A shows a diagram of various immune cells, their mechanistic interaction for immune activation, and production of interferon gamma (IFNg) cytokine that triggers CD8 and NK cells anti-tumor activity or attack to eliminate of cancer cells. The grey arrows indicate increase (pointing up) or decrease (pointing down) in number of cells and dark arrows points to cells that it develops into in the presence of IFNg. Regulatory T cells (Tregs) are a specialized subpopulation of T cells that act to suppress T helper 1 (Th1) cells immune response (represented horizontal T), thereby maintaining homeostasis and self-tolerance. Treg inhibits T cell proliferation and cytokine production to suppress the immune system by inhibiting development of Th1 into CD8+ cytotoxic T cell (CTL) and under normal condition prevents autoimmunity. Additionally, Th1 cells provide helper functions to other cells of the immune system-especially the antigen-presenting cells (APCs) such as macrophages, dendritic cells, and B cells-and are important for their activation and maturation.

FIGURES 26B-26D show suppression of Tregs cells, increase of activated Th1 cells where VLA-dCNP alone was significantly more effective than Anti-PD1 alone (Top right graph), and the ratio of Th1/Treg cells where VLA-dCNP alone was significantly more effective than Anti-PD1 alone in an E0771 mouse breast cancer model. Surprisingly when both dCNP (described in Example 1) and Anti-PD1 antibody were combined they had synergistic effect much greater than the sum of the effects of administration of dCNP alone and Anti-PD1 alone. These graphs indicated dCNP and Anti-PD1 antibody enhanced anti-tumor immunity in a surprisingly synergistic manner by increasing Th1, CD8+, and NK cells while decreasing Treg cells.

In this study, female C57BL/6J mice (6 weeks old, female, n=6) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with E0771 mouse breast cancer cells (250,000 cells/mouse) (Cosmo Bio, Tokyo Japan) in the left mammary gland. From the 8^{th} day, mice were treated with a bolus dose of 0.3 mg/kg very long acting CNP derivative or VLA-dCNP (described in Example 1) via SC daily for 5 days in buffer (Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)) and/or 5 mg/Kg Anti-PD1 Ab via IP twice (day 8 and day 11). At 12^{th} day, tumor was harvested analyzed by FACS for Th1 cells and Treg cells and ratio were calculated. Statistical analysis was performed by Student t-tests by using GraphPad Prism **P <* 0.05 vs. Control or **P<0.01 vs. Control (*n* =6).

### Example 27: Adjuvant such as CpG oligodeoxynucleotides (ODN), a Toll-like receptor-9 agonist, enhanced dCNP tumor volume suppression activity against breast cancer.

CpG ODN adjuvant bind to and activate Toll-like receptor 9, initiating an innate immune response that supports the subsequent development of adaptive immunity. TLR9 agonists improve antigen presentation and the induction of vaccine- specific cellular and humoral responses. Clinical trials utilizing CpG ODNs have been conducted that evaluated their utility in preventing or treating allergy, infectious disease, and especially in cancer (Immunopotentiatory in Modern Vaccines, 2017 163-198; Chapter 9; Nature reviews urology 2013 10 537-545; Npj Vaccines 2020 vol 5 Article number 50.

FIGURE 27 shows the significant tumor suppression when dCNP was combined with adjuvant CpG ODN-TLR9 agonists in the mice breast cancer model. Adjuvant such as CpG oligodeoxynucleotides (ODN), a Toll-like receptor-9 agonist, enhanced dCNP tumor volume suppression activity against breast cancer in mice model.

In this study, female C57BL/6J mice (6 weeks old, female, n=6) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with E0771 mouse breast cancer cells (250,000 cells/mouse) (Cosmo Bio, Tokyo Japan) in the left mammary gland. From the 6^{th} day, mice were treated with a bolus dose of dCNP derivative or dCNP (described in Example 1) via SC daily for 5 days in buffer (Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)) or dCNP( 0.3 mg/kg S.C.) and 2 mg/Kg CpG ODN (ODN 1826; Type B: Adipogen Life sciences, San Diego, CA) via IP twice (Day 6 and Day 9). At 14^{th} day, animals were sacrificed. Statistical analysis was performed by Student t-tests by using GraphPad Prism **P* < 0.01 vs. Control group *(n* =5). CpG ODNs are short synthetic single-stranded DNA molecules containing unmethylated CpG dinucleotides.

### Example 28: dCNP, anti-PD1 antibody, and combination of both are effective immune activator that able to decrease tumor volume and Treg cells and increase of CD69+ cells in mice breast cancer model.

CD69 is a cell surface glycoprotein and is an activation marker for various immune cells. Consistent with general activation of T cells, activated tumor-infiltrating B cells (TIL-Bs) are CD69+ and associated with increase an effector T-cell (IFNg+CD4+TILs) response (Cancer Immunol Res. 2017 Oct 5(10) 898-907). In addition, CD69+ B cells were linked with Tertiary lymphoid structures (TLS) that is correlated with long term survival with NSCLC patients (Cancer Immunol Res. 2017 Oct 5(10) 898-907). The activation of T lymphocytes and NK cells, both in vivo and in vitro, induces expression of CD69 (Immunity 2018 48 4 702-715).

FIGURES 28A-28D show dCNP, anti-PD1 antibody, and combination of both were effective immune activators that were able to decrease tumor volume and Treg cells and increase CD69+ cells in mice breast cancer model. Suppression of Treg while increasing CD69+ cell population was consistent with immune activation causing tumor volume suppression.

In this study, female C57BL/6J mice (6 weeks old, female, n=6) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with E0771 mouse breast cancer cells (250,000 cells/mouse) (Cosmo Bio, Tokyo Japan) in the left mammary gland. From the 8^{th} day, mice were treated with a bolus dose of 0.3 mg/kg CNP derivative or dCNP (described in Example 1) via SC daily for 5 days in buffer (Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)) and/or 5 mg/Kg anti-PD1 antibody via IP twice (Day 8 and Day 11). At 12^{th} day, animals were sacrificed. Statistical analysis was performed by Student t-tests by using GraphPad Prism **P* < 0.01 vs. Control group (*n* =6).

### Example 29: dCNP suppressed expression of fibrosis-associated and vascular-disrupting genes within the tumor when evaluated in a much larger or more developed mice prostate cancer but not by the cytotoxic chemotherapeutic agent (Docetaxel).

Excessive production of extracellular matrix by cancer-associated fibroblast and fibrosis indicates increase cancer expansion. Fibrosis can cause immunosuppression in the tumor microenvironment through exclusion and inhibition of cytotoxic T cells and activation of myeloid-derived suppressor cells (MDSCs) (Front. Immunol, Aug 2019). In addition, fibrosis also elicited chemoresistance through solid stress and suppressing perfusion (PNAS Feb 2019 116 (6)2210-2219). Angiopoietin-2 (Ang2) inhibit the tumor vascular stabilization through antagonizing Ang1/Tie2 axis. Inhibition of Ang 2 expression stabilizes vasculature, enhances access to tumor, and improves drug delivery (Cancer Cell 2016 Vol 30 953-967).

FIGURES 29A-29C show dCNP, but not Docetaxel or buffer control, inhibited alpha-smooth muscle actin (a-SMA; Top Left graph), TGFβ (Top Right graph), and Ang 2 gene expression within the prostate tumor of mice cancer model. A decrease in expression of these genes indicated a decrease in fibrotic process and such a decrease improves the anti-tumor activity of the immune system. The α-SMA is the marker of tissue fibrogenesis and TGFβ is a well-known mediator of fibrogenesis and upregulated and activated in fibrotic disease (Growth Factors, 2011 29 (5) 196-202). Ang2 inhibit the tumor vascular stabilization through antagonizing Ang1/Tie2 axis. Inhibition of Ang 2 expression stabilizes vasculature, enhances access to tumor, and improves drug delivery (Cancer Cell 2016 Vol 30 953-967). As shown in FIGURE 29C, dCNP stabilized the tumor vasculature and created a better anti-tumor tumor microenvironment for the immune system.

In this study, male C57BL/6J mice (6 weeks old, male, n=10) were purchased from Kyodo (Saga, Japan) and maintained under a 12-hour light/12-hour dark cycle with free access to water and standard mouse diet (MF diet; Oriental Yeast Co., Ltd. Tokyo Japan). Mice were implanted with TRAMPC-1 mouse prostate cancer cells (1,000,000 cells/mouse) (ATCC, Manassas, VA) in the subcutaneous. From the 19^{th} day (averaged tumor size was 70 mm3), mice were treated with a bolus dose of long acting CNP derivative or dCNP (described in Example 1) (0.3 mg/kg s.c. or 1.0 mg/kg s.c.) in buffer [Methionine 100 mM (Tokyo Chemical Industry Co., Ltd., Tokyo, Japan); Sucrose 100 mM (Tokyo Chemical Industry Co., Ltd.); Histidine 50 mM (Tokyo Chemical Industry Co., Ltd.); in H₂O (Otsuka Pharmaceutical, Tokushima, Japan)], Docetaxel (DTX 1 mg/kg i.p.) in Saline, all with or without anti-CD8 (5 mg/Kg i.p.; BioX cell; West Lebanon, NH) or Phosphate buffered saline (PBS) alone via i.p. All Docetaxel groups were used as mechanism of action comparator chemotherapeutic agent. Mice were treated for 2 weeks (5 days of once daily, 2 days of rest, 5 days of once-daily treatment and 2 days rest). Anti-CD8 Ab was administered every 3 days starting day 19^{th}. Tumor was harvested at day 30 and extracted RNA, then Quantitative RT-PCR was performed; error bars are SEM. Statistical analysis was performed by Student t-tests by using GraphPad Prism **P* < 0.05 or **P<0.01 vs. Control group (*n* =3-6).

### Example 30: Pharmacokinetic profile of long acting CNP derivative s1 (dCNP-s1) and CNP derivative s2 (dCNP-s2) from a bolus administration showed sustained presence in the blood over time.

Referring to FIGURE 30, shown is a graph of plasma CNP [mean (SEM); n= 5] in CD-1 mice after subcutaneous bolus administration of 2.0mg/Kg of CNP derivative s1 (dCNP-s1), and CNP derivative s2 (dCNP-s2). For comparison, the inset shows the low plasma level of CNP (diamond) when native CNP was administered. Error bars represent standard error of the mean of n=5 plasma samples. Baseline CNP level prior to administration was 0.391 (0.02) ng/mL [mean (SEM); n=10]. Long acting dCNP-s1 and dCNP-s2 provides 10-fold higher blood level of CNP in a sustain manner (at least 8 hours) than native CNP when given at similar dose weight/Kg dose.

For this pharmacokinetic study, all animals (mice) for this study were maintained under a 12-hour light/12-hour dark cycle with free access to water and standard mouse diet (Lab Pico Rodent #5053; Animal Specialties, Woodburn, OR). Male CD-1 mice (6-8 weeks old; Charles River, Hollister, CA) were treated with 2.0 mg/Kg of CNP derivative s1 (dCNP-s1; PharmaIN Corp, Bothell, WA), and CNP derivative s2 (dCNP-s2; PharmaIN Corp, Bothell, WA) via subcutaneous bolus administration between the shoulder blades. All test articles were formulated or dissolved in 100 mM sucrose, 100 mM methionine, 50 mM histidine, pH 7.4. Blood sampling at various times (0 hour, 0.5 hour, 1 hour, 2 hours, 4 hours, 6 hours, 8 hours, and 24 hours) was performed by retro-orbital bleed, two bleeding per animal at two different timepoints. Blood samples were processed in K2EDTA tubes to obtain plasma. Plasma was analyzed by commercially available CNP ELISA kit from Phoenix Pharmaceuticals (cat# EKE-012-03). CNP is a native human CNP (GLSKGCFGLKLDRIGSMSGLGC [SEQ ID NO. 10] and dCNP-s1 and dCNP-s2 are derivatives of human CNP with the following sequences: HOC(=O)(CH₂)₁₆C(=O)-Aeea-Aeea-GCFGLKLDRIGShomoQSGLGC [SEQ ID NO.21, and HOC(=O)(CH₂)₁₆C(=O)-γE-Aeea-Aeea-GCFGLKLDRIGShomoQSGLGC [SEQ ID NO. 20], each with a disulfide bond between the 2 cysteine residues, and where homoQ: homoglutamine residue; Aeea: 2-[2-(2-aminoethoxy)ethoxy]acetic acid residue, where the amino and carboxylic acid groups are used in forming amide bonds to provide the CNP derivatives; HOC(=O)(CH₂)₁₆C(=O)- was derived from octadecadioic acid; γE: gamma glutamic acid residue.

By example and without limitation, embodiments are disclosed according to the following enumerated paragraphs:
A1. A method of treating a subject having an abnormal vasculature, in any tissue or organ, comprising administering to the subject a therapeutically effective bolus dose of a composition comprising a long acting CNP, a very long acting CNP, a long acting CNP derivative, a very long acting CNP derivative, a long acting NPRB agonist, a very long acting NPRB agonist, or any combination thereof,
   wherein administering the therapeutically effective bolus dose of the composition provides a vasculature normalization or an increase in pericyte coating index by at least 10%,
   wherein the composition does not decrease blood pressure by more than 20% (*e.g.,* by more than 15%, by more than 10%, or by more than 5%) of a baseline blood pressure measurement, where the baseline blood pressure measurement is an average blood pressure prior to administration of the composition, and
   wherein the composition increases plasma cyclic-GMP level at from 1 hour to 12 hours (after administration to above 1.5x of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).
A2. A method of increasing a number of cytotoxic T-cells and/or a number of activated NK cells, comprising administering to a subject in need thereof a therapeutically effective bolus dose of a composition comprising a long acting CNP, a very long acting CNP, along acting CNP derivative, a very long acting CNP derivative, a long acting NPRB agonist, a very long acting NPRB agonist, or any combination thereof,
   wherein the therapeutically effective bolus dose of the composition provides an increase in the number of cytotoxic T cells and/or NK cells of at least 15% above the level prior to administration of the composition or above the level in a healthy subject,
   wherein administering the therapeutically effective bolus dose of the composition does not decrease blood pressure by more than 20% of a baseline blood pressure measurement, where the baseline blood pressure measurement is an average blood pressure prior to administration of the composition, and
   wherein the composition increases plasma cyclic-GMP level at from 1 hour to 12 hours after administration to above 1.5x of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).
A3. A method of Paragraph A1 or Paragraph A2, wherein:
   the subject has a condition (i) to (viii):
      (i) a low number of cytotoxic T cells,
      (ii) a low number of activated NK cells,
      (iii) a high number of Treg cells,
      (iv) a high level of expression of TGFβ,
      (v) a high level or expression of Foxp3,
      (vi) a high number of myeloid-derived suppressor cells or MDSCs,
      (vii) a high level or expression of Bv8; or
      (viii) any combination thereof; or
   the subject is in need of (ix) to (xvi):
      (ix) an increase in a number of cytotoxic T-cells;
      (x) an increase in activated NK cells;
      (xi) a decrease in a number of Treg cells;
      (xii) a decrease in TGF-β expression;
      (xiii) a decrease in Foxp3 expression;
      (xiv) a decrease in a number of myeloid-derived suppressor cells (MDSCs);
      (xv)a decrease in Bv8 expression, or
      (xvi) any combination thereof,
   wherein administering the therapeutically effective bolus dose of the composition provides a reduction in tumor size when present, an increase in the number of cytotoxic T-cells, an increase in the number of activated NK cells, a reduction in the number of Treg cells, a decrease in the level or expression of TGFβ, a decrease in the level or expression of Foxp3, a decrease in the number of myeloid-derived suppressor cells (MDSCs), a decrease in the level or expression of Bv8, an improvement in survival/lifespan, or a combination thereof.
A4. A method of treating a subject of any one of Paragraphs A1 to A3, wherein the subject further has a low number of cytotoxic T cells, a low number of activated NK cells, or both a low number of cytotoxic T cells and a low number of activated NK cells prior to administering the therapeutically effective bolus dose of the composition.
A5. The method of any one of Paragraphs A1 to A4, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
   U is a moiety of Formula (I) or (II), where Formula (I) is

      (aliphatic)ₐ-(X)-; (I)
   wherein
      a is 0 or 1 (preferably a is 1);
      aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1,
   and Formula (II) is

      (polymer)ₐ-(Y)-; (II)
   wherein
      a is 0 or 1 (preferably a is 1);
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ; or
         a peptide linker different from the 1-10 amino acid residue or peptide sequence.
A6. The method of Paragraph A5, wherein Y is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A7. The method of Paragraph A5 or Paragraph A6, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 12], or any combination thereof; and;
   wherein:
   U is a moiety of Formula (I), where Formula (I) is

      (aliphatic)ₐ-(X)-; (I)
   wherein
      a is 0 or 1 (preferably a is 1);
      aliphatic is an optionally substituted C₁₀₋₂₄ chain (*e.g.,* an optionally substituted C₁₂₋₁₈ chain), covalently bound to X via a chemical linkage, such as a carbonyl *(e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1.
A8. The method of any one of Paragraphs A5 to A7, wherein X is a 4-7 amino acid sequence, wherein each amino acid residue is independently selected from lysine (K), arginine (R), and glycine (G), or
   X is a linker (γE)ₘ-(B)ₙ wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A9. The method of any one of Paragraphs A5 to A8, wherein the long acting CNP derivative or the very long acting CNP derivative comprises U-GLSKGCFGLKLDRIGSMSGLGC [SEQ ID NO. 2],
   wherein:
   U is (aliphatic)ₐ-(X)-;
   wherein
      a is 0 or 1 (preferably a is 1);
      aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and
      X is a 1-10 amino acid sequence, wherein each amino acid residue is independently selected from lysine (K), arginine (R), glycine (G), alanine (A), glutamic acid (E), and aspartic acid (D); or
      X is a linker (γE)ₘ-(B)ₙ wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A10. The method of any one of Paragraphs A5 to A9, wherein aliphatic does not comprise a straight or branched optionally substituted C₄₋₉ chain (*e.g.,* an optionally substituted C₃₋₈ alkyl-C(=O)- moiety, and/or an optionally substituted C₄₋₉ alkyl that is covalently bound to the peptide via a linkage such as a thioether, an ether, a thioether, a carbamate moiety, a bond, or the like).
A11. The method of any one of Paragraphs A1 to A10, wherein the long acting CNP derivative is selected from
   CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
A12. The method of any one of Paragraphs A1 to A11, wherein the long acting CNP derivative is CH₃(CH₂)₁₄C(=O)KKKKGGGGLSKGCFGLKLDRIGSMSGLGC [SEQ ID NO. 5].
A13. The method of any one of Paragraphs A1 to A11, wherein the long acting CNP derivative is CH₃(CH₂)₁₆C(=O)KKKKGGGGLSKGCFGLKLDRIGSMSGLGC [SEQ ID NO. 6].
A14. The method of any one of Paragraphs A1 to A11, wherein the long acting CNP derivative is CH₃(CH₂)₁₈C(=O)KKKKGGGGLSKGCFGLKLDRIGSMSGLGC [SEQ ID NO. 7].
A15. The method of any one of Paragraphs A1 to A11, wherein the long acting CNP derivative is CH₃(CH₂)₂₀C(=O)KKKKGGGGLSKGCFGLKLDRIGSMSGLGC [SEQ ID NO. 8].
A16. The method of any one of Paragraphs A1 to A11, wherein the long acting CNP derivative is CH₃(CH₂)₂₂C(=O)KKKKGGGGLSKGCFGLKLDRIGSMSGLGC [SEQ ID NO. 9].
A17. The method of any one of Paragraphs A1 to A11, wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
A18. The method of any one of Paragraphs A1 to A11, wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
A19. The method of any one of Paragraphs A1 to A6, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof;
   wherein:
   U is a moiety of Formula (II), where Formula (II) is

      (polymer)ₐ-(Y)-; (II)
   wherein
      a is 0 or 1 (preferably a is 1);
      polymer is cellulose, poly(ethylene glycol) (PEG), methoxy poly(ethylene glycol) (MPEG), poly(lactic-co-glycolic acid), or poly(N-vinyl pyrrolidone);
      Y is:
         a 4-10 amino acid residue or peptide sequence, wherein each amino acid residue is independently selected from lysine (K), arginine (R), and glycine (G);
         a non-amino acid linker comprising an ester, an amide, a thioether, an ether, a thioether, a carbamate moiety, or a combination thereof; or
         a linker (γE)ₘ-(B)ₙ, wherein B is 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A20. The method of any one of Paragraphs A1 to A6 and A19, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof;
   wherein:
   U is a moiety of Formula (II), where Formula (II) is

      (polymer)ₐ-(Y)-; (II)
   wherein
      a is 0 or 1 (preferably a is 1);
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ;
         a peptide linker different from the 1-10 amino acid residue or peptide sequence; or
         a linker (γE)ₘ-(B)ₙ, wherein B is 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A21. The method of any one of Paragraphs A1 to A6, A19, and A20, wherein the polymer does not include poly(ethylene glycol), MPEG, or both poly(ethylene glycol) and MPEG.
A22. The method of any one of Paragraphs A1 to A6 and A19 to A21, wherein Y is:
   a 4-10 amino acid residue or peptide sequence, wherein each amino acid residue is independently selected from lysine (K), arginine (R), and glycine (G); or
   a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A23. The method of any one of Paragraphs A1 to A22, wherein the bolus dose administration occurs at most twice a day and the route of administration comprises subcutaneous, intravenous, by inhalation, intramuscular, nasal, enteral, or any combination thereof, or
   wherein the route of administration is subcutaneous; or
   wherein the route of administration is intravenous; or
   wherein the route of administration is intramuscular; or
   wherein the route of administration is by inhalation; or
   wherein the route of administration is nasal; or
   wherein the enteral route of administration is oral.
A24. The method of any one of Paragraphs A1 to A23, wherein the subject has cancer.
A25. The method of Paragraph A24, wherein the cancer is selected from breast cancer; bone cancer (*e.g.,* osteosarcoma); prostate cancer (*e.g.,* early stage prostate cancer, late stage prostate cancer); colon cancer, head cancer, neck cancer, liver cancer, kidney cancer, cervical cancer, lung cancer, stomach cancer, urethra cancer, bladder cancer, cancer of the ureter, renal pelvic cancer, cancer of the rectum, esophageal cancer, cancer of the lymph node, pancreatic cancer, stomach cancer, ovarian cancer; cancer of the central nervous system, soft tissue cancer, endocrine gland cancer; or any combination thereof, or
   wherein the subject has skin cancer; or
   wherein the subject has colon cancer; or
   wherein the subject has breast cancer; or
   wherein the subject has bone cancer (*e.g.,* osteosarcoma); or
   wherein the subject has prostate cancer.
A26. The method of Paragraph A24 or Paragraph A25, wherein the cancer is responsive to a cytotoxic cell immunostimulant.
A27. The method of any one of Paragraphs A24 to A26, wherein the cancer is selected from head and neck cancer, skin cancer, liver cancer, kidney cancer, cervical cancer, lung cancer, breast cancer, stomach cancer, colon cancer, lymph node cancer, pancreatic cancer, ovary such as dMMR, cancer of the urethra, bladder, ureters, renal pelvis, and surrounding organs.
A28. The method of any one of Paragraphs A25 to A27, wherein
   the skin cancer comprises Merkel cell carcinoma, squamous cell carcinoma, melanoma, or any combination thereof;
   the liver cancer comprises hepatocellular carcinoma;
   the kidney cancer comprises renal cell carcinoma;
   the lung cancer comprises small cell or non-small cell lung carcinoma;
   the breast cancer comprises triple negative breast cancer;
   the stomach cancer comprises gastric cancer, adenocarcinoma of esophageal junction, or dMMR);
   the lymph node cancer comprises Hodgkin or non-Hodgkin PMBCL;
   the pancreatic cancer and ovarian cancer each independent comprises dMMR; and
   the cancer of the surrounding organs to the renal pelvic area comprises urothelial cancer.
A29. The method of any one of Paragraphs A1 to A28, further comprising
   administering to the subject an immune adjuvant, wherein the immune adjuvant modulates a toll-like receptor, or a cytotoxic cell immunostimulant comprising therapeutic agents (*e.g.,* protein and/or small molecule compounds) or antibodies targeting an immune checkpoint protein selected from CTLA-4 (cytotoxic T-lymphocyte-associated protein 4) , PD1 (programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, an immune checkpoint member of the B7 and CD28 families), B7H4 (a molecule of the B7 family, negatively regulates T cell immunity), and TIM-3 (a co-inhibitory receptor that is expressed on IFN-γ-producing T cells); wherein the cytotoxic cell immunostimulant inhibits an immune checkpoint protein; or
   wherein cytotoxic cell immunostimulant comprises an antibody or portion of an antibody against an immune checkpoint protein, a soluble ligand of an immune checkpoint protein, pembrolizumab, Nivolumab, Ipilimumab, Atezolizumab, Avelumab, Durvalumab, Cemiplimab, tremelimumab, lambrolizumab, and/or pidilizumab; or
   wherein the immune adjuvant comprises a toll-like receptor 9 agonist; or
   wherein the immune adjuvant comprises a CpG oligodeoxynucleotide.
A30. The method of any one of Paragraphs A1 to A29, further comprising administering to the subject CAR T-cells.
A31. The method of any one of Paragraphs A1 to A28, wherein the composition comprises a long acting CNP composition or a very long acting CNP derivative composition comprising a CNP, a CNP derivative, or a long acting CNP derivative and a polymer excipient, the polymer excipient comprising a poly(amino acid) grafted with polyethylene glycol, fatty acid, anionic moieties, or any combination thereof, wherein the polymer excipient is adapted to sequester or non-covalently bind to any of the CNP or CNP derivatives..
A32. The method of any one of Paragraphs A1 to A31, wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; and wherein the polymer excipient is adapted to sequester or non-covalently bind to the long acting CNP derivative.
A33. The method of any one of Paragraphs A1 to A4 and A23 to A30, wherein the long acting NPRB agonist or the very long acting NPRB agonist comprises a polypeptide.
A34. The method of Paragraph A33, wherein the polypeptide comprises an antibody.
A35. The method of any one of Paragraphs A1 to A4 and A23 to A33, wherein the long acting NPRB agonist or the very long acting NPRB agonist comprises a molecule of a molecular weight of less than 2kDa.
A36. The method of any one of Paragraphs A1 to A35, wherein the composition does not decrease blood pressure by more than 15% of a baseline blood pressure measurement.
A37. The method of any one of Paragraphs A1 to A35, wherein the composition does not decrease blood pressure by more than 10% of a baseline blood pressure measurement.
A38. A method of treating a subject having cancer, comprising
   administering to the subject in need thereof a therapeutically effective bolus dose of a composition comprising a long acting CNP derivative or a very long acting CNP derivative comprising U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
      U is a moiety of Formula (I) or (11), where Formula (1) is

         (aliphatic)ₐ-(X)-; (I)
      wherein
         a is 0 or 1 (preferably a is 1);
         aliphatic is an optionally substituted C₄₋₂₄ chain (*e.g.,* an optionally substituted C₁₀₋₂₄ chain, an optionally substituted C ₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
         X is a 1-10 amino acid residue or peptide sequence, wherein each amino acid residue is independently selected from lysine (K), arginine (R), glycine (G), alanine (A), glutamic acid (E), and aspartic acid (D); or
         X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1;
      and Formula (II) is

         (polymer)ₐ-(Y)-; (II)
      wherein
         a is 0 or 1 (preferably a is 1);
         polymer is cellulose, poly(ethylene glycol) (PEG), methoxy poly(ethylene glycol) (MPEG), poly(lactic-co-glycolic acid), poly(N-vinyl pyrrolidone), or a derivative thereof;
         Y is:
            a 1-10 amino acid residue or peptide sequence, wherein each amino acid residue is independently selected from lysine (K), arginine (R), glycine (G), alanine (A), glutamic acid (E), and aspartic acid (D);
            a non-amino acid linker comprising an ester, an amide, a thioether, an ether, a thioether, a carbamate moiety, or a combination thereof,
            an amino acid residue-containing linker, wherein the amino acid residue is covalently attached to (polymer)ₐ; or
            a peptide linker different from the 1-10 amino acid residue or peptide sequence;
   wherein the composition does not decrease blood pressure by more than 15% (*e.g.,* by more than 15%, or by more than 10%) of a baseline blood pressure measurement, where the baseline blood pressure measurement is an average blood pressure prior to administration of the composition; and
   wherein the composition increases plasma cyclic-GMP level at from 1 hour to 12 hours (*e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) to above 1.5x (*e.g.,* above 2x, above 3x, above 4x, or above 5x) of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).
A39. The method of Paragraph A38, wherein Y is a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A40. The method of Paragraph A38 or Paragraph A39, wherein the long acting CNP derivative is selected from:
   CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
A41. The method of any one of Paragraphs A38 to A40, wherein the long acting CNP derivative is CH₃(CH₂)₁₄C(=O)KKKKGGGGLSKGCFGLKLDRIGSMSGLGC [SEQ ID NO. 5].
A42. The method of any one of Paragraph A38 to A40, wherein the long acting CNP derivative is CH₃(CH₂)₁₆C(=O)KKKKGGGGLSKGCFGLKLDRIGSMSGLGC [SEQ ID NO. 6].
A43. The method of any one of Paragraph A38 to A40, wherein the long acting CNP derivative is CH₃(CH₂)₁₈C(=O)KKKKGGGGLSKGCFGLKLDRIGSMSGLGC [SEQ ID NO. 7].
A44. The method of any one of Paragraphs A38 to A40, wherein the long acting CNP derivative is CH₃(CH₂)₂₀C(=O)KKKKGGGGLSKGCFGLKLDRIGSMSGLGC [SEQ ID NO. 8].
A45. The method of any one of Paragraphs A38 to A40, wherein the long acting CNP derivative is CH₃(CH₂)₂₂C(=O)KKKKGGGGLSKGCFGLKLDRIGSMSGLGC [SEQ ID NO. 9].
A46. The method of any one of Paragraphs A38 to A40, wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
A47. The method of any one of Paragraphs A38 to A40, wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
A48. The method of any one of Paragraphs A38 to A47, wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; wherein the polymer excipient is adapted to sequester or non-covalently bind to the long acting CNP derivative.
A49. The method of any one of Paragraphs A1 to A48, wherein the subject has a tumor and administering to the subject the therapeutically effective bolus dose of the composition increases vasculature normalization or an increase in pericyte coating index by at least 20% within a tumor tissue.
A50. The method of any one of Paragraphs A1 to A49, wherein the subject has a tumor and administering to the subject the therapeutically effective bolus dose of the composition decreases the tumor size.
A51. The method of any one of Paragraphs A1 to A50, wherein the subject has a tumor and administering to the subject the therapeutically effective bolus dose of the composition improves survival of the subject.
A52. The method of any one of Paragraphs A1 to A51, wherein the subject has a tumor and administering to the subject the therapeutically effective bolus dose of the composition increases the number of cytotoxic T-cells within the tumor.
A53. The method of any one of Paragraphs A1 to A52, wherein the subject has a tumor and administering to the subject the therapeutically effective bolus dose of the composition increases the number of activated NK cells within the tumor.
A54. The method of any one of Paragraphs A1 to A53, wherein the subject has a tumor and administering to the subject the therapeutically effective bolus dose of the composition increases vascular normalization within the tumor, decreases the tumor size, improves survival, increases the number of cytotoxic T-cells within the tumor and/or increases number of activated NK cells within the tumor.
A55. A composition comprising a long acting CNP derivative of comprising a formula U-CFGLKLDRIGSxSGLGC [SEQ ID NO. 30], wherein
   x is a natural or unnatural amino acid residue, provided that x is not a methionine residue; and
   U has is a moiety of Formula (I):

      (aliphatic)ₐ-(X)-; (I)
   wherein a is 0 or 1 (preferably a is 1);
   aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A56. The composition of Paragraph A55, wherein x is homoglutamine, aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); a is 1; B is Gly; m is 0, 1, or 2; and n is 1.
A57. The composition of Paragraph A55, wherein x is homoglutamine, aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); a is 1; B is Gly; m is 1; and n is 1.
A58. The composition of Paragraph A55, wherein x is homoglutamine, aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); a is 1; m is 1; and n is 0.
A59. The composition of Paragraph A55, wherein x is homoglutamine (homoQ) [SEQ ID NO. 16], U is (aliphatic)ₐ-(X)-; wherein a is 0 or 1 (preferably a is 1); aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 0, and n is 2.
A60. The composition of Paragraph A55, wherein x is homoglutamine (homoQ) [SEQ ID NO. 17, U is (aliphatic)ₐ-(X)-; wherein a is 0 or 1 (preferably a is 1); aliphatic is a branched or straight optionally substituted C₁₈ chain covalently bound to X via a carbonyl (*e*.*g*., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(-O)(CH₂)₁₆C(=O)); X is a linker (γE)ₘ-(B)ₙ; B is 2-[2-(2-aminoethoxy)ethoxy] acetic acid residue, m is 1, and n is 2.
A61. The composition of Paragraph A55, wherein x is homoglutamine, aliphatic is CH₃(CH₂)₁₆C(=O) or HOC(=O)(CH₂)₁₆C(=O); B is a 2-[2-(2-aminoethoxy)ethoxy]acetic acid residue, m is 0, and n is 2.
A62. The composition of Paragraph A55, wherein x is homoglutamine, aliphatic is CH₃(CH₂)₁₆C(=O) or HOC(=O)(CH₂)₁₆C(=O); B is a 2-[2-(2-aminoethoxy)ethoxy]acetic acid residue, m is 1, and n is 2.
A63. The composition of Paragraph A55, wherein x is homoglutamine, aliphatic is CH₃(CH₂)₁₆C(=O) or HOC(=O)(CH₂)₁₆C(=O); B is (2-[2-(2-aminoethoxy)ethoxy]acetic acid)-(2-[2-(2-aminoethoxy)ethoxy]acetic acid)-(Gly), m is 1, and n is 1.
A64. The composition of Paragraph A55, wherein the long acting CNP derivative is HOC(=O)(CH₂)₁₆C(=O)-γE-Aeea-Aeea-GCFGLKLDRIGShomoQSGLGC with a disulfide bond between the cysteine residues [SEQ ID NO. 20].
A65. The composition of Paragraph A55, wherein the long acting CNP derivative is HOC(=O)(CH₂)₁₆C(=O)-Aeea-Aeea-GCFGLKLDRIGShomoQSGLGC with a disulfide bond between the cysteine residues [SEQ ID NO. 21].
A66. The method of any one of Paragraphs A5 to A54, wherein a is 1.
A67. The composition of any one of Paragraphs A55 to A65, wherein a is 1.

While illustrative embodiments have been illustrated and described, it will be appreciated that various changes can be made therein without departing from the spirit and scope of the disclosure.

### EMBODIMENTS OF THE INVENTION

Also provided are the following embodiments:
1. A method of treating a subject having an abnormal vasculature, in any tissue or organ, comprising administering to the subject a therapeutically effective bolus dose of a composition comprising a long acting CNP, a very long acting CNP, a long acting CNP derivative, a very long acting CNP derivative, a long acting NPRB agonist, a very long acting NPRB agonist, or any combination thereof,
   wherein administering the therapeutically effective bolus dose of the composition provides a vasculature normalization or an increase in pericyte coating index by at least 10%,
   wherein the composition does not decrease blood pressure by more than 20% of a baseline blood pressure measurement, and
   wherein the composition increases plasma cyclic-GMP level at from 1 hour to 12 hours after administration to above 1.5x of a baseline plasma cyclic-GMP level, and the baseline plasma cyclic-GMP level is an average plasma cyclic-GMP level prior to administration of the composition or the average plasma cyclic-GMP level of a healthy subject.
2. A method of increasing a number of cytotoxic T-cells and/or a number of activated NK cells, comprising administering to a subject in need thereof a therapeutically effective bolus dose of a composition comprising a long acting CNP, a very long acting CNP, a long acting CNP derivative, a very long acting CNP derivative, a long acting NPRB agonist, a very long acting NPRB agonist, or any combination thereof,
   wherein the therapeutically effective bolus dose of the composition provides an increase in the number of cytotoxic T cells and/or NK cells of at least 15% above the level prior to administration of the composition or above the level in a healthy subject,
   wherein administering the therapeutically effective bolus dose of the composition does not decrease blood pressure by more than 20% of a baseline blood pressure measurement, and
   wherein the composition increases plasma cyclic-GMP level at from 1 hour to 12 hours after administration to above 1.5x of a baseline plasma cyclic-GMP level, and the baseline plasma cyclic-GMP level is an average plasma cyclic-GMP level prior to administration of the composition or the average plasma cyclic-GMP level of a healthy subject.
3. A method of embodiment [1] or embodiment [2], wherein:
   the subject has a condition (i) to (viii):
      (i) a low number of cytotoxic T cells,
      (ii) a low number of activated NK cells,
      (iii) a high number of Treg cells,
      (iv) a high level of expression of TGFβ,
      (v) a high level or expression of Foxp3,
      (vi) a high number of myeloid-derived suppressor cells or MDSCs,
      (vii) a high level or expression of Bv8; or
      (viii) any combination thereof; or
   the subject is in need of (ix) to (xvi):
      (ix) an increase in a number of cytotoxic T-cells;
      (x) an increase in activated NK cells;
      (xi) a decrease in a number of Treg cells;
      (xii) a decrease in TGF-β expression;
      (xiii) a decrease in Foxp3 expression;
      (xiv) a decrease in a number of myeloid-derived suppressor cells (MDSCs);
      (xv)a decrease in Bv8 expression, or
      (xvi) any combination thereof,
   wherein administering the therapeutically effective bolus dose of the composition provides a reduction in tumor size when present, an increase in the number of cytotoxic T-cells, an increase in the number of activated NK cells, a reduction in the number of Treg cells, a decrease in the level or expression of TGFβ, a decrease in the level or expression of Foxp3, a decrease in the number of myeloid-derived suppressor cells (MDSCs), a decrease in the level or expression of Bv8, an improvement in survival/lifespan, or a combination thereof.
4. A method of treating a subject of any one of embodiments [1] to [3], wherein the subject further has a low number of cytotoxic T cells, a low number of activated NK cells, or both a low number of cytotoxic T cells and a low number of activated NK cells prior to administering the therapeutically effective bolus dose of the composition.
5. The method of any one of embodiments [1] to [3], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
   U is a moiety of Formula (I) or (II), where Formula (I) is

      (aliphatic)ₐ-(X)-; (I)
   wherein
      a is 1;
      aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1,
   and Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ; or
         a peptide linker different from the 1-10 amino acid residue or peptide sequence.
6. The method of embodiment [5], wherein Y is a linker (γE)ₘ-(B)ₙ, wherein B is a 1-8 amino acid residue or peptide sequence wherein each amino acid residue is independently selected from a 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
7. The method of embodiment [5], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 12], or any combination thereof; and;
   wherein:
   U is a moiety of Formula (I), where Formula (I) is

      (aliphatic)ₐ-(X)-; (I)
   wherein
      a is 1;
      aliphatic is an optionally substituted C₁₀₋₂₄ chain (*e.g.,* an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1.
8. The method of embodiment [5], wherein X is a 4-7 amino acid sequence, wherein each amino acid residue is independently selected from lysine (K), arginine (R), and glycine (G), or
   X is a linker (γE)ₘ-(B)ₙ wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
9. The method of embodiment [5], wherein the long acting CNP derivative or the very long acting CNP derivative comprises U-GLSKGCFGLKLDRIGSMSGLGC [SEQ ID NO. 2],
   wherein:
   U is (aliphatic)ₐ-(X)-;
   wherein
      a is 1;
      aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
10. The method of embodiment [5], wherein aliphatic does not comprise a straight or branched optionally substituted C₄₋₉ chain (*e.g.,* an optionally substituted C₃₋₈ alkyl-C(=O)- moiety, and/or an optionally substituted C₄₋₉ chain that is covalently bound to the peptide via a linkage such as a thioether, an ether, a thioether, a carbamate moiety, a bond, or the like).
11. The method of any one of embodiments [1] to [3], wherein the long acting CNP derivative is selected from
   CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
12. The method of any one of embodiments [1] to [3], wherein the long acting CNP derivative is CH₃(CH₂)₁₄C(=O)KKKKGGGGLSKGCFGLKLDRIGSMSGLGC [SEQ ID NO. 5].
13. The method of any one of embodiments [1] to [3], wherein the long acting CNP derivative is CH₃(CH₂)₁₆C(=O)KKKKGGGGLSKGCFGLKLDRIGSMSGLGC [SEQ ID NO. 6].
14. The method of any one of embodiments [1] to [3], wherein the long acting CNP derivative is CH₃(CH₂)₁₈C(=O)KKKKGGGGLSKGCFGLKLDRIGSMSGLGC [SEQ ID NO. 7].
15. The method of any one of embodiments [1] to [3], wherein the long acting CNP derivative is CH₃(CH₂)₂₀C(=O)KKKKGGGGLSKGCFGLKLDRIGSMSGLGC [SEQ ID NO. 8].
16. The method of any one of embodiments [1] to [3], wherein the long acting CNP derivative is CH₃(CH₂)₂₂C(=O)KKKKGGGGLSKGCFGLKLDRIGSMSGLGC [SEQ ID NO. 9].
17. The method of any one of embodiments [1] to [3], wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20]
18. The method of any one of embodiments [1] to [3], wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21]
19. The method of embodiment [5], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof;
   wherein:
   U is a moiety of Formula (II), where Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), or poly(N-vinyl pyrrolidone);
      Y is:
         a 4-10 amino acid residue or peptide sequence, wherein each amino acid residue is independently selected from lysine (K), arginine (R), and glycine (G);
         a non-amino acid linker comprising an ester, an amide, a thioether, an ether, a thioether, a carbamate moiety, or a combination thereof; or
         a linker (γE)ₘ-(B)ₙ, wherein B is 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
20. The method of embodiment [19], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], or any combination thereof;
   wherein:
   U is a moiety of Formula (II), where Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ;
         a peptide linker different from the 1-10 amino acid residue or peptide sequence; or
         a linker (γE)ₘ-(B)ₙ, wherein B is 1-8 amino acid residue sequence wherein each amino acid residue is independently selected from a 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
21. The method of embodiment [19], wherein the polymer does not include poly(ethylene glycol), MPEG, or both poly(ethylene glycol) and MPEG.
22. The method of embodiment [5], wherein Y is:
   a 4-10 amino acid residue or peptide sequence, wherein each amino acid residue is independently selected from lysine (K), arginine (R), and glycine (G); or
   a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
23. The method of any one of embodiments [1] to [3], wherein the bolus dose administration occurs at most twice a day and the route of administration comprises subcutaneous, intravenous, by inhalation, intramuscular, nasal, enteral, or any combination thereof, or
   wherein the route of administration is subcutaneous; or
   wherein the route of administration is intravenous; or
   wherein the route of administration is intramuscular; or
   wherein the route of administration is by inhalation; or
   wherein the route of administration is nasal; or
   wherein the enteral route of administration is oral.
24. The method of any one of embodiments [1] to [3], wherein the subject has cancer.
25. The method of embodiment [24], wherein the cancer is selected from breast cancer; bone cancer; prostate cancer; colon cancer, head cancer, neck cancer, liver cancer, kidney cancer, cervical cancer, lung cancer, stomach cancer, urethra cancer, bladder cancer, cancer of the ureter, renal pelvic cancer, cancer of the rectum, esophageal cancer, cancer of the lymph node, pancreatic cancer, stomach cancer, ovarian cancer; cancer of the central nervous system, soft tissue cancer, endocrine gland cancer; or any combination thereof, or
   wherein the subject has skin cancer; or
   wherein the subject has colon cancer; or
   wherein the subject has breast cancer; or
   wherein the subject has bone cancer; or
   wherein the subject has prostate cancer.
26. The method of embodiment [24], wherein the cancer is responsive to a cytotoxic cell immunostimulant.
27. The method of embodiment [24], wherein the cancer is selected from head and neck cancer, skin cancer, liver cancer, kidney cancer, cervical cancer, lung cancer, breast cancer, stomach cancer, colon cancer, lymph node cancer, pancreatic cancer, ovary such as dMMR, cancer of the urethra, bladder, ureters, renal pelvis, and surrounding organs.
28. The method of embodiment [25], wherein
   the skin cancer comprises Merkel cell carcinoma, squamous cell carcinoma, melanoma, or any combination thereof;
   the liver cancer comprises hepatocellular carcinoma;
   the kidney cancer comprises renal cell carcinoma;
   the lung cancer comprises small cell or non-small cell lung carcinoma;
   the breast cancer comprises triple negative breast cancer;
   the stomach cancer comprises gastric cancer, adenocarcinoma of esophageal junction, or dMMR);
   the lymph node cancer comprises Hodgkin or non-Hodgkin PMBCL;
   the pancreatic cancer and ovarian cancer each independent comprises dMMR; and
   the cancer of the surrounding organs to the renal pelvic area comprises urothelial cancer.
29. The method of embodiment [24], further comprising
   administering to the subject an immune adjuvant, wherein the immune adjuvant modulates a toll-like receptor, or a cytotoxic cell immunostimulant comprising therapeutic agents or antibodies targeting an immune checkpoint protein selected from CTLA-4 (cytotoxic T-lymphocyte-associated protein 4), PD1 (programmed cell death protein 1 or CD279), PD-L1 (programmed death-ligand 1), PD-L2 (programmed death-ligand 2), LAG-3 (lymphocyte-activation gene 3 protein), BTLA (B- and T-lymphocyte attenuator), B7H3 ((CD276, an immune checkpoint member of the B7 and CD28 families), B7H4 (a molecule of the B7 family, negatively regulates T cell immunity), and TIM-3 (a co-inhibitory receptor that is expressed on IFN-γ-producing T cells); wherein the cytotoxic cell immunostimulant inhibits an immune checkpoint protein; or
   wherein cytotoxic cell immunostimulant comprises an antibody or portion of an antibody against an immune checkpoint protein, a soluble ligand of an immune checkpoint protein, pembrolizumab, Nivolumab, Ipilimumab, Atezolizumab, Avelumab, Durvalumab, Cemiplimab, tremelimumab, lambrolizumab, and/or pidilizumab; or
   wherein the immune adjuvant comprises a toll-like receptor 9 agonist; or
   wherein the immune adjuvant comprises a CpG oligodeoxynucleotide.
30. The method of embodiment [24], further comprising administering to the subject CAR T-cells.
31. The method of any one of embodiments [1] to [3], wherein the composition comprises a long acting CNP composition or a very long acting CNP derivative composition comprising a CNP, a CNP derivative, or a long acting CNP derivative and a polymer excipient, the polymer excipient comprising a poly(amino acid) grafted with polyethylene glycol, fatty acid, anionic moieties, or any combination thereof; wherein the polymer excipient is adapted to sequester or non-covalently bind to any of the CNP or CNP derivatives..
32. The method of any one of embodiments [1] to [3], wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; and wherein the polymer excipient is adapted to sequester or non-covalently bind to the long acting CNP derivative.
33. The method of any one of embodiments [1] to [3], wherein the long acting NPRB agonist or the very long acting NPRB agonist comprises a polypeptide.
34. The method of embodiment [33], wherein the polypeptide comprises an antibody.
35. The method of embodiment [33], wherein the long acting NPRB agonist or the very long acting NPRB agonist comprises a molecule of a molecular weight of less than 2kDa.
36. The method of any one of embodiments [1] to [3], wherein the composition does not decrease blood pressure by more than 15% of a baseline blood pressure measurement.
37. The method of any one of embodiments [1] to [3], wherein the composition does not decrease blood pressure by more than 10% of a baseline blood pressure measurement.
38. A method of treating a subject having cancer, comprising
   administering to the subject in need thereof a therapeutically effective bolus dose of a composition comprising a long acting CNP derivative or a very long acting CNP derivative comprising U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
      U is a moiety of Formula (I) or (II), where Formula (I) is

         (aliphatic)ₐ-(X)-; (I)
      wherein
         a is 1;
         aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
         X is a 1-10 amino acid residue or peptide sequence, wherein each amino acid residue is independently selected from lysine (K), arginine (R), glycine (G), alanine (A), glutamic acid (E), and aspartic acid (D); or
         X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1;
      and Formula (II) is

         (polymer)ₐ-(Y)-; (II)
      wherein
         a is 1;
         polymer is cellulose, poly(ethylene glycol) (PEG), methoxy poly(ethylene glycol) (MPEG), poly(lactic-co-glycolic acid), poly(N-vinyl pyrrolidone), or a derivative thereof;
         Y is:
            a 1-10 amino acid residue or peptide sequence, wherein each amino acid residue is independently selected from lysine (K), arginine (R), glycine (G), alanine (A), glutamic acid (E), and aspartic acid (D);
            a non-amino acid linker comprising an ester, an amide, a thioether, an ether, a thioether, a carbamate moiety, or a combination thereof;
            an amino acid residue-containing linker, wherein the amino acid residue is covalently attached to (polymer)ₐ; or
            a peptide linker different from the 1-10 amino acid residue or peptide sequence;
   wherein the composition does not decrease blood pressure by more than 15% of a baseline blood pressure measurement; and
   wherein the composition increases plasma cyclic-GMP level at from 1 hour to 12 hours to above 1.5x of a baseline plasma cyclic-GMP level, and the baseline plasma cyclic-GMP level is an average plasma cyclic-GMP level prior to administration of the composition or the average plasma cyclic-GMP level of a healthy subject.
39. The method of embodiment [38], wherein Y is a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
40. The method of embodiment [38] or embodiment [39], wherein the long acting CNP derivative is selected from CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
41. The method of any one of embodiments [38] to [40], wherein the long acting CNP derivative is CH₃(CH₂)₁₄C(=O)KKKKGGGGLSKGCFGLKLDRIGSMSGLGC [SEQ ID NO. 5].
42. The method of any one of embodiments [38] to [40], wherein the long acting CNP derivative is CH₃(CH₂)₁₆C(=O)KKKKGGGGLSKGCFGLKLDRIGSMSGLGC [SEQ ID NO. 6].
43. The method of any one of embodiments [38] to [40], wherein the long acting CNP derivative is CH₃(CH₂)₁₈C(=O)KKKKGGGGLSKGCFGLKLDRIGSMSGLGC [SEQ ID NO. 7].
44. The method of any one of embodiments [38] to [40], wherein the long acting CNP derivative is CH₃(CH₂)₂₀C(=O)KKKKGGGGLSKGCFGLKLDRIGSMSGLGC [SEQ ID NO. 8].
45. The method of any one of embodiments [38] to [40], wherein the long acting CNP derivative is CH₃(CH₂)₂₂C(=O)KKKKGGGGLSKGCFGLKLDRIGSMSGLGC [SEQ ID NO. 9].
46. The method of any one of embodiments [38] to [40], wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
47. The method of any one of embodiments [38] to [40], wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
48. The method of any one of embodiments [34] to [47], wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; wherein the polymer excipient is adapted to sequester or non-covalently bind to the long acting CNP derivative.
49. The method of any one of embodiments [1] to [48], wherein the subject has a tumor and administering to the subject the therapeutically effective bolus dose of the composition increases vasculature normalization or an increase in pericyte coating index by at least 20% within a tumor tissue.
50. The method of any one of embodiments [1] to [49], wherein the subject has a tumor and administering to the subject the therapeutically effective bolus dose of the composition decreases the tumor size.
51. The method of any one of embodiments [1] to [50], wherein the subject has a tumor and administering to the subject the therapeutically effective bolus dose of the composition improves survival of the subject.
52. The method of any one of embodiments [1] to [51], wherein the subject has a tumor and administering to the subject the therapeutically effective bolus dose of the composition increases the number of cytotoxic T-cells within the tumor.
53. The method of any one of embodiments [1] to [52], wherein the subject has a tumor and administering to the subject the therapeutically effective bolus dose of the composition increases the number of activated NK cells within the tumor.
54. The method of any one of embodiments [1] to [53], wherein the subject has a tumor and administering to the subject the therapeutically effective bolus dose of the composition increases vascular normalization within the tumor, decreases the tumor size, improves survival, increases the number of cytotoxic T-cells within the tumor and/or increases number of activated NK cells within the tumor.
55. A composition comprising a long acting CNP derivative of comprising a formula U-CFGLKLDRIGSxSGLGC [SEQ ID NO. 30], wherein
   x is a natural or unnatural amino acid residue, provided that x is not a methionine residue; and
   U has is a moiety of Formula (I):

      (aliphatic)ₐ-(X)-; (I)
   wherein a is 1;
   aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
56. The composition of embodiment [55], wherein x is homoglutamine, aliphatic is a branched or straight optionally substituted C₁₈ chain covalently bound to X via a carbonyl (*e.g*., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); a is 1; B is Gly; m is 0, 1, or 2; and n is 1.
57. The composition of embodiment [55], wherein x is homoglutamine, aliphatic is a branched or straight optionally substituted C₁₈ chain covalently bound to X via a carbonyl (*e.g*., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); a is 1; B is Gly; m is 1; and n is 1.
58. The composition of embodiment [55], wherein x is homoglutamine, aliphatic is a branched or straight optionally substituted C₁₈ chain covalently bound to X via a carbonyl (e.g., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (e.g., CH₃(CH₂)₁₆C(=O)) as part of an amide or ester linkage with X; or more preferably aliphatic is a branched or straight optionally substituted C₁₈ chain covalently bound to X via a carbonyl (e.g., CH₃(CH₂)₁₆C(=O)) as part of an amide linkage with X, or aliphatic is HOC(=O)(CH₂)₁₆C(=O)); a is 1; m is 1; and n is 0.
59. The composition of embodiment [55], wherein x is homoglutamine (homoQ) [SEQ ID NO. 16], U is (aliphatic)ₐ-(X)-; wherein a is 0 or 1 (preferably a is 1); aliphatic is a branched or straight optionally substituted C₁₈ chain covalently bound to X via a carbonyl (*e.g*., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 0, and n is 2.
60. The composition of embodiment [55], wherein x is homoglutamine (homoQ) [SEQ ID NO. 17], U is (aliphatic)ₐ-(X)-; wherein a is 0 or 1 (preferably a is 1); aliphatic is a branched or straight optionally substituted C₁₈ chain covalently bound to X via a carbonyl (*e.g*., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 1, and n is 2.
61. The composition of embodiment [55], wherein x is homoglutamine, aliphatic is a CH₃(CH₂)₁₆C(=O) or HOC(=O)(CH₂)₁₆C(=O); B is a 2-[2-(2-aminoethoxy)ethoxy]acetic acid residue, m is 0, and n is 2.
62. The composition of embodiment [55], wherein x is homoglutamine, aliphatic is a CH₃(CH₂)₁₆C(=O) or HOC(=O)(CH₂)₁₆C(=O); B is a 2-[2-(2-aminoethoxy)ethoxy]acetic acid residue, m is 1, and n is 2.
63. The composition of embodiment [55], wherein x is homoglutamine, aliphatic is a CH₃(CH₂)₁₆C(=O) or HOC(=O)(CH₂)₁₆C(=O); B is (2-[2-(2-aminoethoxy)ethoxy]acetic acid)-(2-[2-(2-aminoethoxy)ethoxy]acetic acid)-(Gly), m is 1, and n is 1.
64. The composition of embodiment [55], wherein the long acting CNP derivative is HOC(=O)(CH₂)₁₆C(-O)-γE-Aeea-Aeea-GCFGLKLDRIGShomoQSGLGC with a disulfide bond between the cysteine residues [SEQ ID NO. 20] .
65. The composition of embodiment [55], wherein the long acting CNP derivative is HOC(=O)(CH₂)₁₆C(=O)-Aeea-Aeea-GCFGLKLDRIGShomoQSGLGC with a disulfide bond between the cysteine residues [SEQ ID NO. 21].

## Claims

1. A composition for use in a method of treating a subject having an abnormal vasculature, in any tissue or organ, the method comprising administering to the subject a therapeutically effective bolus dose of the composition, the composition comprising CH₃(CH₂)₁₆C(=O)KKKKGGGGLSKGCFGLKLDRIGSMSGLGC [SEQ ID NO.6],
wherein administering the therapeutically effective bolus dose of the composition provides a vasculature normalization or an increase in pericyte coating index by at least 10%,
wherein the composition does not decrease blood pressure by more than 20% of a baseline blood pressure measurement, and
wherein the composition increases plasma cyclic-GMP level at from 1 hour to 12 hours after administration to above 1.5x of a baseline plasma cyclic-GMP level, and the baseline plasma cyclic-GMP level is an average plasma cyclic-GMP level prior to administration of the composition or the average plasma cyclic-GMP level of a healthy subject.

2. A composition for use in a method of treating a subject by increasing a number of cytotoxic T-cells and/or a number of activated NK cells, the method comprising administering to a subject in need thereof a therapeutically effective bolus dose of the composition, the composition comprising CH₃(CH₂)₁₆C(=O)KKKKGGGGLSKGCFGLKLDRIGSMSGLGC [SEQ ID NO. 6],
wherein the therapeutically effective bolus dose of the composition provides an increase in the number of cytotoxic T cells and/or NK cells of at least 15% above the level prior to administration of the composition or above the level in a healthy subject,
wherein administering the therapeutically effective bolus dose of the composition does not decrease blood pressure by more than 20% of a baseline blood pressure measurement, and
wherein the composition increases plasma cyclic-GMP level at from 1 hour to 12 hours after administration to above 1.5x of a baseline plasma cyclic-GMP level, and the baseline plasma cyclic-GMP level is an average plasma cyclic-GMP level prior to administration of the composition or the average plasma cyclic-GMP level of a healthy subject.

3. The composition for use according to claim 1 or 2, wherein the subject has cancer.

4. The composition for use according to claim 1 or 2, wherein the subject has fibrosis associated with tumors.

5. The composition for use according to claim 3, wherein the cancer is skin cancer, breast cancer; bone cancer; prostate cancer; colon cancer, head cancer, neck cancer, liver cancer, kidney cancer, cervical cancer, lung cancer, stomach cancer, urethra cancer, bladder cancer, cancer of the ureter, renal pelvic cancer, cancer of the rectum, esophageal cancer, cancer of the lymph node, pancreatic cancer, stomach cancer, ovarian cancer; cancer of the central nervous system, soft tissue cancer, endocrine gland cancer; or any combination thereof.

6. The composition for use according to claim 5, wherein the cancer is skin cancer, colon cancer, breast cancer; bone cancer; prostate cancer; or any combination thereof.

7. The composition for use according to claim 6, wherein the cancer is skin cancer.

8. The composition for use according to claim 7, wherein the skin cancer comprises Merkel cell carcinoma, squamous cell carcinoma, melanoma, or any combination thereof.

9. The composition for use according to claim 6, wherein the cancer is colon cancer.

10. The composition for use according to claim 6, wherein the cancer is breast cancer.

11. The composition for use according to claim 10, wherein the breast cancer comprises triple negative breast cancer

12. The composition for use according to claim 6, wherein the cancer is bone cancer.

13. The composition for use according to claim 6, wherein the cancer is prostate cancer.

14. The composition for use of any one of any one of the preceding claims, wherein the composition further comprises a polymer excipient, the polymer excipient comprising a poly(amino acid) grafted with polyethylene glycol, fatty acid, anionic moieties, or any combination thereof, wherein the polymer excipient is adapted to sequester or non-covalently bind to the CH₃(CH₂)₁₆C(=O)KKKKGGGGLSKGCFGLKLDRIGSMSGLGC [SEQ ID NO. 6].

15. The composition for use according to claim 14, wherein the polymer excipient comprises polylysine grafted with polyethylene glycol.
